(19) Europäisches Patentamt
European Patent Office
Office européen des brevets

(11) Veröffentlichungsnummer : **0 337 944 B1**

(12) # EUROPÄISCHE PATENTSCHRIFT

(45) Veröffentlichungstag der Patentschrift : **28.07.93 Patentblatt 93/30**

(51) Int. Cl.$^5$ : **C07D 239/42,** C07D 239/70, C07D 239/84, A01N 43/54

(21) Anmeldenummer : **89810252.0**

(22) Anmeldetag : **04.04.89**

(54) **Harnstoffe.**

(30) Priorität : **12.04.88 CH 1355/88**

(43) Veröffentlichungstag der Anmeldung : **18.10.89 Patentblatt 89/42**

(45) Bekanntmachung des Hinweises auf die Patenterteilung : **28.07.93 Patentblatt 93/30**

(84) Benannte Vertragsstaaten : **AT BE CH DE ES FR GB GR IT LI LU NL SE**

(56) Entgegenhaltungen :
**EP-A- 0 013 143**
**EP-A- 0 065 480**

(56) Entgegenhaltungen :
**EP-A- 0 126 296**
**EP-A- 0 172 786**
**EP-A- 0 243 136**
**EP-A- 0 264 348**
**DD-A- 151 404**

(73) Patentinhaber : **CIBA-GEIGY AG**
**Klybeckstrasse 141**
**CH-4002 Basel (CH)**

(72) Erfinder : **Rempfler, Hermann, Dr.**
**Brücklismattstrasse 16**
**CH-4107 Ettingen (CH)**
Erfinder : **Dürr, Dieter, Dr.**
**Brändelistalweg 16**
**CH-4103 Bottmingen (CH)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 337 944 B1

Jouve, 18, rue Saint-Denis, 75001 PARIS

## Beschreibung

Die vorliegende Erfindung betrifft neue N-Phenyl-N-pyrimidin-2-yl-harnstoffe mit herbizider und pflanzenwuchsregulierender Wirkung, agrochemische Mittel, welche diese Substanzen als Wirkstoffe enthalten, die Verwendung der neuen Harnstoffe zur Bekämpfung von Unkräutern oder zur Regulierung des Pflanzenwuchses so wie Verfahren zur Herstellung der neuen Verbindungen. Ferner betrifft die Erfindung auch neue Zwischenprodukte und Verfahren zu deren Herstellung.

Aus der Patentschrift DD-151 404 und der europäischen Patentanmeldung EP-A-0 172 786 sind (Pyrimidin-2-yl) -2-nitroaniline bekannt geworden. Diese Verbindungen sind fungizid wirksam. Demgegenüber wurde überraschenderweise gefunden, dass N-Phenyl-N-pyrimidin-2-yl-harnstoffe herbizide. bzw. pflanzenwachstumsregulatorische Wirkung haben. Aus der nicht vorpublizierten EP-A-0 264 348 sind herbizid wirksame N-(2-Nitrophenyl)-N-pyrimidin-2-yl-harnstoffe bekannt geworden, die sich in charakteristischer Weise in der 5-Stellung des Pyrimidinrestes von den erfindungsgemässen Harnstoffen unterscheiden.

Die Erfindung betrifft Harnstoffe der Formel I

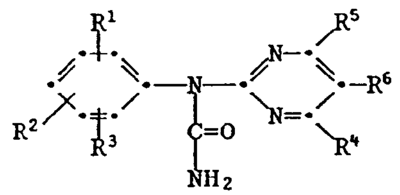

(I),

worin

| | |
|---|---|
| $R^1$, $R^2$ und $R^3$ | unabhängig voneinander Wasserstoff; Nitro; Cyano; Halogen; $C_1$-$C_4$-Alkyl; $C_1$-$C_4$-Alkyl-$S(O)_n$; $C_1$-$C_4$-Alkoxy; $C_1$-$C_4$-halogenalkyl; $C_1$-$C_4$-Halogenalkoxy; $C_1$-$C_4$-halogenalkyl-$S(O)_n$ ; $C_1$-$C_4$-Alkoxycarbonyl; $C_1$-$C_4$-Alkylcarbonyl; Aminocarbonyl; Mono-$C_1$-$C_4$-alkylaminocarbonyl; oder Di-$C_1$-$C_4$-Alkylaminocarbonyl; |
| $R^4$ und $R^5$ | unabhängig voneinander Wasserstoff; $C_1$-$C_4$-Alkyl; $C_1$-$C_4$-Alkoxy; $C_1$-$C_4$-Alkyl-$S(O)_n$; $C_1$-$C_4$-Halogenalkyl; $C_1$-$C_4$-Halogenalkoxy; $C_1$-$C_4$-Halogenalkyl-$S(O)_n$; unsubstituiertes oder bis zu dreifach gleich oder verschieden durch $C_1$-$C_4$-Alkyl, Halogen, $C_1$-$C_4$-Alkoxy, $C_1$-$C_4$-Halogenalkyl, Nitro oder Cyano substituiertes Phenyl; Furanyl; Thiophenyl; $C_3$-$C_6$-Cycloalkyl; $C_1$-$C_4$-Alkoxycarbonyl; $C_1$-$C_4$-Alkoxy-$C_1$-$C_4$-alkyl; $C_1$-$C_4$-Alkoxycarbonyl-$C_1$-$C_4$-alkyl; $C_1$-$C_4$-Alkylcarbonyl-$C_1$-$C_4$-alkyl; $C_3$-$C_4$-Alkenyloxycarbonyl-$C_1$-$C_4$-alkyl; $C_3$-$C_4$-Alkinyloxycarbonyl-$C_1$-$C_4$-alkyl; Halogen; oder Cyano; |
| $R^6$ | $C_1$-$C_4$-Alkyl; Halogen; Cyano; $C_3$-$C_6$-Cycloalkyl; $C_1$-$C_4$-Halogenalkyl; $C_1$-$C_4$-Alkoxy; Nitro; $C_1$-$C_4$-Alkyl-$S(O)_n$; $C_1$-$C_4$-Halogenalkoxy; $C_1$-$C_4$-Halogenalkyl-$S(O)_n$; oder unsubstituiertes oder bis zu dreifach gleich oder verschieden durch Halogen, $C_1$-$C_4$-Alkyl, $C_1$-$C_4$-Halogenalkyl, $C_1$-$C_4$-Alkyl-$S(O)_n$; $C_1$-$C_4$-Alkoxy, $C_1$-$C_4$-Halogenalkoxy, Nitro oder Cyano substituiertes Phenyl; oder $C_1$-$C_4$-Alkylthio-$C_1$-$C_4$-alkyl; |
| n | 0, 1 oder 2; oder |
| $R^5$ und $R^6$ | gemeinsam mit den beiden Kohlenstoffatomen an welche sie gebunden sind einen anellierten, teilweise ungesättigten 4- bis 8-gliedrigen Ring, der bis zu dreifach gleich oder verschieden durch $C_1$-$C_4$-Alkyl, Halogen, $C_1$-$C_4$-Alkoxycarbonyl substituiert sein kann und/oder durch O, S oder N-($C_1$-$C_4$)-Alkyl unterbrochen ist und/oder eine Doppelbindung und/oder eine Carbonylgruppe enthalten kann; |

bedeutet, unter Einschluss ihrer Salze mit Säuren, Basen und Komplexbildnern.

Im Rahmen der hier offenbarten Erfindung umfassen die angegebenen generischen Begriffe beispielsweise die folgenden spezifischen Einzelsubstituenten:

Alkyl umfasst die geradkettigen oder verzweigten $C_1$-$C_4$-Alkyle, wie Methyl, Ethyl, n-Propyl, iso-Propyl, n-Butyl, sek-Butyl, tert-Butyl und iso-Butyl. Bevorzugt sind die $C_1$-$C_3$-Alkylreste.

Halogen ist Fluor, Chlor, Brom und Jod. Bevorzugt ist im Falle der Substituenten $R^4$ bis $R^6$ Fluor, Chlor und Brom und im Fall der Substituenten $R^1$ bis $R^3$ Fluor, Chlor, Brom und Iod.

Mit Halogenalkyl sind die ganz oder teilweise gleich oder unterschiedlich halogensubstituierten Alkyle, gemäss der jeweiligen Definitionsbreite gemeint, wie etwa Trifluormethyl, Difluormethyl, 1,1,2,2-Tetrafluorethyl, 2-Chlorethyl, Pentafluorethyl, Chlordifluormethyl, Dichlormethyl, Chlorfluormethyl, 1,1-Dichlor-2,2,2-trifluorethyl, 1,1-Dichlorethyl oder Heptafluorpropyl.

Als $C_1$-$C_4$-Alkoxycarbonylreste sind unter anderem Methoxycarbonyl, Ethoxycarbonyl sowie die isomeren

Propyloxycarbonyle und Butyloxycarbonyle zu nennen.

Alkoxy sind im Rahmen der jeweiligen Definitionsbreite der isomeren Alkyloxyradikale, in erster Linie Methoxy, Ethoxy, (i)-Propyloxy, (n)-Propyloxy, (i)-Butyloxy, (t)-Butyloxy, (n)-Butyloxy und (sec)-Butyloxy.

Halogenalkoxy sind im Rahmen der jeweiligen Definitionsbreite die isomeren ein- oder mehrfach gleich oder verschieden halogensubstituierten Alkylreste, wie etwa Trifluormethoxy, 2,2,2-Trifluorethoxy, 2,2,3,3,3-Pentafluorpropyloxy oder 1,1,2,2-Tetrafluorethoxy, Difluormethoxy oder 2-Chlorethoxy.

Als Alkoxyalkylreste sind unter anderem zu nennen: 2-Ethoxyethyl, 2-Methoxyethyl, 3-Methoxypropyl, 2-Methoxy-1-methylethyl und Methoxymethyl.

Die Gruppe $C_1$-$C_4$-Alkyl-S(O)$_n$- steht für die jeweiligen Alkylthio-, Alkylsulfinyl- und Alkylsulfonylradikale. Als besonders bevorzugt sind Methylthio, Methylsulfinyl, Methylsulfonyl, Ethylthio, Ethylsulfinyl oder Ethylsulfonyl zu nennen.

Die Gruppe $C_1$-$C_4$-Halogenalkyl-S(O)$_n$- steht für die jeweiligen Halogenalkylthio-, Halogenalkylsulfinyl- und Halogenalkylsulfonylradikale. Besonders bevorzugt sind Difluormethylthio, Difluormethylsulfinyl, Difluormethylsulfonyl, Trifluormethylthio oder Chlorfluormethylthio.

Bei der Gruppe $C_1$-$C_4$-Alkylthio-$C_1$-$C_4$-alkyl ist insbesondere 2-Methylthioethyl und 2-Ethylthioethyl zu nennen.

$C_3$-$C_6$-Cycloalkyl sind Cyclopropyl, Cyclobutyl, Cyclopentyl und Cyclohexyl, vorzugsweise Cyclopropyl, Cyclopentyl und Cyclohexyl. Bei den Resten $R^4$ bis $R^6$ ist Cyclopropyl hervorzuheben.

Das als Substituent genannte Phenylradikal kann innerhalb der angegebenen Definitionsbreite substituiert sein. Als Einzelsubstituenten zu nennen sind unter anderem unsubstituiertes Phenyl, 2-Chlorphenyl, 4-Chlorphenyl, 2,4-Dichlorphenyl, o-Tolyl, p-Tolyl, 2-Trifluormethylphenyl und 4-Trifluormethylphenyl.

In den Fällen, in denen die Reste $R^5$ und $R^6$ gemeinsam mit den beiden Kohlenstoffatomen, an welche sie gebunden sind 4 bis 8 gliedrige Ringe bilden, sind die nachstehend genannten Ringsysteme bevorzugt:

a)

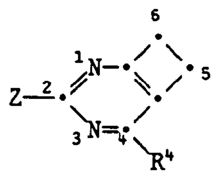

5,6-Dihydro-cyclobuta[d]pyrimidine

b)

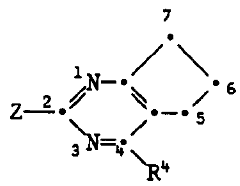

5H-6,7-Dihydro-cyclopenta[d]pyrimidine

c)

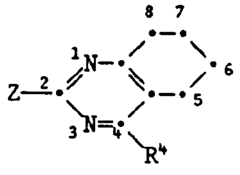

5,6,7,8-Tetrahydro-chinazoline

d)

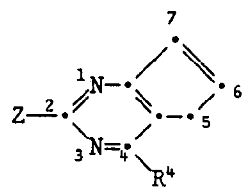

5H-Cyclopenta[d]pyrimidine
e)

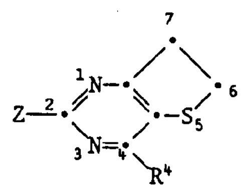

6,7-Dihydro-thieno[2,3-d]pyrimidine
f)

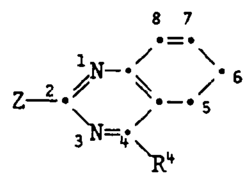

5,6,-Dihydro-chinazoline
g)

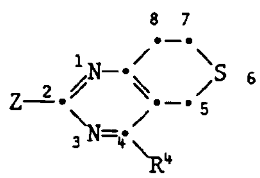

5H-7,8-Dihydro-thiopyrano[4,3-d]pyrimidine
h)

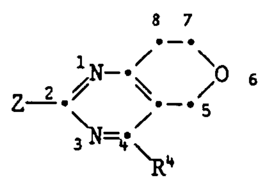

5H-7,8-Dihydro-pyrano[4,3-d]pyrimidine
i)

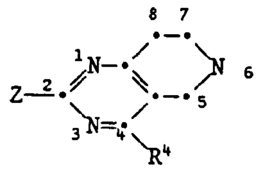

5,6,7,8-Tetrahydro-pyrido[4,3-d]pyrimidine

j)

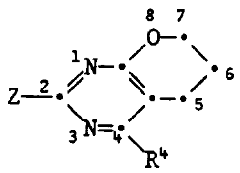

7H-5,6-Dihydro-pyrano[2,3-d]pyrimidine

k)

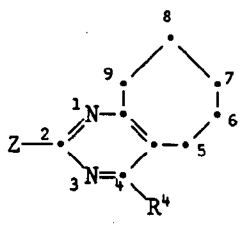

9H-5,6,7,8-Tetrahydro-cyclohepta[d]pyrimidine

l)

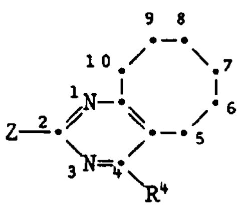

5,6,7,8,9,10-Hexahydro-cycloocta[d]pyrimidine

m)

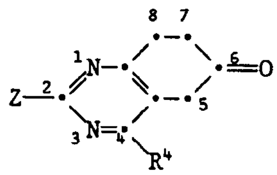

5H-7,8-Dihydro-chinoxaline

n)

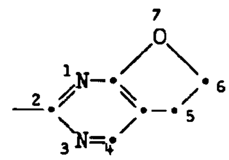

5,6-Dihydro-furano[3,2-e]pyrimidine

o)

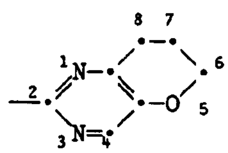

6H-7,8-Dihydro-pyrano[2,3-e]pyrimidine

p)

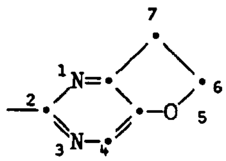

6,7-Dihydro-furano[2,3-e]pyrimidine

In der vorstehend angegebenen Auflistung von Heterocyclen steht Z für

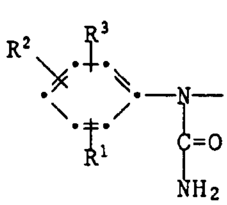

Die die Heterocyclen a bis m enthaltenden Verbindungen I können, wie bei der Definition von $R^5$ und $R^6$ angegeben, weiter substituiert sein.

Als bevorzugte Reste sind neben den unsubstituierten Radikalen a bis p die folgenden Radikale zu nennen:
6-Methyl-5,6-dihydrocyclobuta[d]pyrimidinyl,
5H-7-Methyl-6,7-dihydrocyclopenta[d]pyrimidinyl,
7,7-Dimethyl-6,7-dihydrocyclopenta[d]pyrimidinyl,
5H-6-Methyl-cyclopenta[d]pyrimidinyl,
5H-6-Methyl-6,7-dihydrocyclopenta[d]pyrimidinyl,
5H-5-Methyl-6,7-dihydrocyclopenta[d]pyrimidinyl,
5H-7-($C_1$-$C_4$-Alkoxycarbonyl)-6,7-dihydrocyclopenta[d]pyrimidinyl,
5H-6,6-Dimethyl-6,7-dihydro-cyclopenta[d]pyrimidinyl,
5H-7,7-Dimethyl-6,7-dihydro-cyclopenta[d]pyrimidinyl,
5H-6,7-Dimethyl-6,7-dihydro-cyclopenta[d]pyrimidinyl,
5H-7-Ethyl-6,7-dihydrocyclopenta[d]pyrimidinyl,
5H-7-Ethyl-7-methyl-6,7-dihydrocyclopenta[d]pyrimidinyl,
8-Methyl-5,6,7,8-tetrahydrochinazolin,
8,8-Dimethyl-5,6,7,8-tetrahydrochinazolin,
7-Methyl-5,6,7,8-tetrahydrochinazolin,
5-Methyl-5,6,7,8-tetrahydrochinazolin,
6-Methyl-5,6,7,8-tetrahydrochinazolin,
6,6-Dimethyl-5,6,7,8-tetrahydrochinazolin,
5,7-Dimethyl-5,6-dihydrochinazolin,
6-Methyl-5,6,7,8-tetrahydropyrido[4,3-d]pyrimidin,
8-($C_1$-$C_4$-Alkoxycarbonyl)-5,6,7,8-tetrahydrochinazolin,
6,8,8-Trimethyl-5,6,7,8-tetrahydrochinazolin,
9H-9-Methyl-5,6,7,8-tetrahydrocyclohepta[d]pyrimidin,
9H-9,9-Dimethyl-5,6,7,8-tetrahydrocyclohepta[d]pyrimidin,
6-Chlor-5,6,7,8-tetrahydro-chinazolin.

In den weiteren Substituenten, welche aus mehreren Grundelementen zusammengesetzt sind, können die Teilelemente innerhalb der Definitionsbreite frei gewählt werden und obige Bedeutung haben.

Hervorzuheben sind in der 4-Position des Phenylringes unsubstituierte Verbindungen der Formel Ih

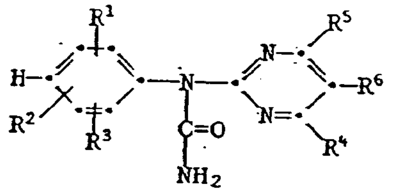

(Ih),

worin
die Reste $R^1$ bis $R^6$ wie zuvor definiert sind.

Insbesondere betrifft die Erfindung Verbindungen der Formel Ih

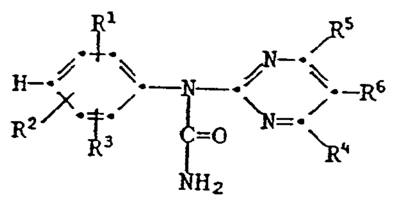

(Ih),

worin

- $R^1$ Wasserstoff; Nitro; Cyano; Halogen; $C_1$-$C_4$-Alkyl; $C_1$-$C_4$-Alkyl-$S(O)_n$; $C_1$-$C_4$-Alkoxy; $C_1$-$C_4$-Halogenalkyl; $C_1$-$C_4$-Halogenalkoxy; $C_1$-$C_4$-Halogenalkyl-$S(O)_n$; $C_1$-$C_4$-Alkoxycarbonyl; $C_1$-$C_4$-Alkylcarbonyl; Aminocarbonyl; Mono-$C_1$-$C_4$-alkylaminocarbonyl; oder Di-$C_1$-$C_4$-Alkylaminocarbonyl;
- $R^2$ Wasserstoff; Nitro; Halogen; $C_1$-$C_4$-Alkyl; oder $C_1$-$C_4$-Halogenalkyl; und
- $R^3$ Wasserstoff; Halogen; oder $C_1$-$C_4$-Alkyl bedeutet und
- $R^4$ bis $R^6$ und n wie zuvor definiert sind.

Bevorzugt sind Verbindungen der Formel Ih

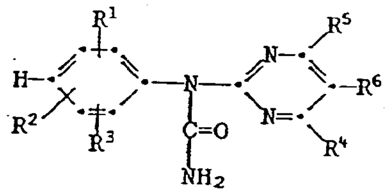

(Ih),

worin

| | |
|---|---|
| $R^1$ | Wasserstoff; Nitro; Cyano; Halogen; $C_1$-$C_3$-Alkyl; $C_1$-$C_2$-Alkyl-$S(O)_n$; $C_1$-$C_2$-halogenalkyl-$S(O)_n$; $C_1$-$C_3$-Alkoxy; $C_1$-$C_3$-Halogenalkyl; $C_1$-$C_2$-Halogenalkoxy; $C_1$-$C_3$-Alkoxycarbonyl; oder Dimethylaminocarbonyl; |
| $R^2$ | Wasserstoff; Nitro; Fluor; Chlor; Brom; oder $C_1$-$C_3$-Alkyl; |
| $R^3$ | Wasserstoff; Chlor; oder $C_1$-$C_3$-Alkyl; |
| $R^4$ | Wasserstoff; $C_1$-$C_4$-Alkyl; $C_1$-$C_3$-Alkoxy; $C_1$-$C_4$-Alkyl-$S(O)_n$-; $C_1$-$C_3$-Halogenalkyl; $C_1$-$C_2$-halogenalkoxy; Phenyl; Chlorphenyl; Furanyl; $C_3$-$C_6$-Cycloalkyl; $C_1$-$C_4$-Alkoxycarbonyl-$C_1$-$C_2$-alkyl; $C_1$-$C_2$-Alkoxy-$C_1$-$C_2$-alkyl; $C_1$-$C_2$-Halogenalkylthio; Chlor; Cyano; oder Acetonyl; |
| $R^5$ | Wasserstoff; $C_1$-$C_3$-Alkyl; $C_1$-$C_3$-Alkoxy; Halogen; $C_1$-$C_3$-Halogenalkyl; oder $C_1$-$C_3$-Halogenalkoxy; |
| $R^6$ | $C_1$-$C_3$-Alkyl; $C_1$-$C_3$-Alkoxy; $C_1$-$C_3$-Halogenalkyl; $C_1$-$C_3$-Halogenakoxy; Chlor; Brom; $C_1$-$C_3$-Alkylthio; Cyano; Nitro; Phenyl; Chlorphenyl: $C_1$-$C_2$-Alkylthio-$C_1$-$C_2$-alkyl; oder $C_3$-$C_6$-Cycloalkyl; |
| n | 0, 1 oder 2; |
| $R^5$ und $R^6$ | gemeinsam eine gegebenenfalls bis zu dreifach durch $C_1$-$C_3$-Alkyl oder gegebenenfalls einfach durch $C_1$-$C_3$-Alkoxycarbonyl oder Chlor substituierte $-CH_2CH_2-$, $-CH_2-CH_2-CH_2-$, $-CH_2-CH_2-CH_2-CH_2-$, $-CH_2-CH_2-CO-CH_2-$, $-CH=CH-CH_2-$, $-CH=CH-CH_2-CH_2-$, $-O-CH_2-CH_2-CH_2-$, $-CH_2-CH_2-S-$, $-CH_2-CH_2-S-CH_2-$, $-CH_2-CH_2-O-CH_2-$ oder $-CH_2-CH_2-N(C_1-C_3\text{-Alkyl})-CH_2$-Brücke |

bedeutet.

Besonders hervorzuheben sind Verbindungen der Formel Ih

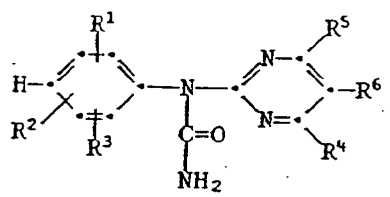

(Ih),

worin

| | |
|---|---|
| $R^1$ | Nitro; Cyano; Halogen; Methyl; Ethyl; $C_1$-$C_3$-Halogenalkyl; $C_1$-$C_2$-Halogenalkyl-$S(O)_n$; Methoxy; Ethoxy; $C_1$-$C_3$-Halogenalkyl; Halogenmethoxy; $C_1$-$C_3$-Alkoxycarbonyl; oder Dimethylaminocarbonyl; |
| n | 0, 1 oder 2; |
| $R^2$ | Wasserstoff; Fluor; Chlor; Brom; Nitro; oder Methyl; |
| $R^3$ | Wasserstoff; Chlor; oder Methyl; |
| $R^4$ | $C_1$-$C_4$-Alkyl; $C_1$-$C_3$-Alkoxy; $C_1$-$C_4$-Alkylthio; Methylsulfinyl; Ethylsulfinyl; Methylsulfonyl; Ethylsulfonyl; $C_3$-$C_6$-Cycloalkyl; Chlor; Cyano; $C_1$-$C_3$-Halogenalkyl; Methoxymethyl; Methoxycarbonylmethyl; Acetonyl; Phenyl; 4-Chlorphenyl; oder 2-Furanyl; |
| $R^5$ | $C_1$-$C_2$-Alkyl; Chlor; $C_1$-$C_3$-Haloalkyl; oder Halogenmethoxy; |
| $R^6$ | $C_1$-$C_3$-Alkyl; Methoxy; Ethoxy; $C_1$-$C_2$-Haloalkyl; Chlor; Brom; $C_1$-$C_3$-Alkylthio; Cyano; Nitro; Phenyl; 4-Chlorphenyl; 2-Methylthioethyl; oder $C_3$-$C_6$-Cycloalkyl; oder |
| $R^5$ und $R^6$ | gemeinsam $-(CH_2)_3-$, $CHCH_3-CH_2-CH_2-$, $-C(CH_3)_2-CH_2-CH_2-$, $-CH_2-CH(CH_3)-CH_2-$, $-CH_2-CH_2-CH(CH_3)-$, $-CH_2-C(CH_3)_2-CH_2-$, $-CH_2-CH_2-$, $-(CH_2)_4-$, $-CH(CH_3)-(CH_2)_3-$, $-CH_2-CH(CH_3)-(CH_2)_2-$, $-(CH_2)_3-CH(CH_3)-$, $-(CH_2)_2-CH(CH_3)-CH_2-$, $-(CH_2)_5-$, $-(CH_2)_6-$ oder $-(CH_2)_3-O-$ |

bedeutet.

Im Hinblick auf ihre biologische Wirkung sind ausserdem die nachstehend genannten Verbindungen der Formeln Ia bis Ig herauszustellen, welche folgendes Substitutionsmuster im Phenylring aufweisen:

Verbindungen der Formel Ia bis Ig

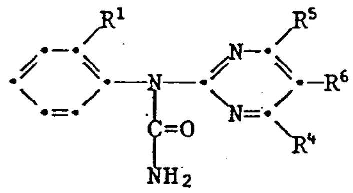

Ia

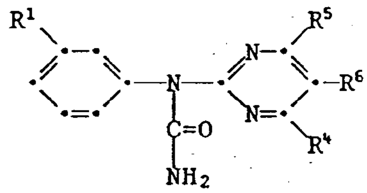

Ib

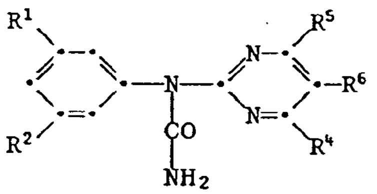

Ic

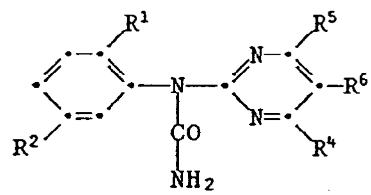

Id

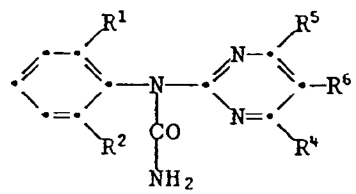

Ie

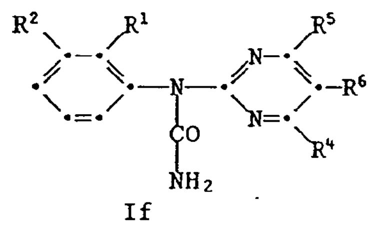

If

und

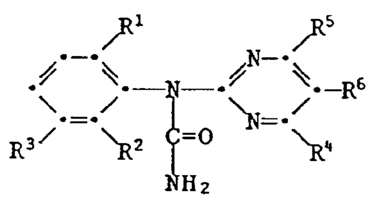

Ig

In den Verbindungen der Formeln Ia bis Ig können die Substituenten $R^1$ bis $R^6$ die vorstehend unter Formel I genannten Definitionsbreiten haben.

Als besonders bevorzugt ist insbesondere diejenige Untergruppe der Verbindungen der Formel I zu nennen, in denen die Reste $R^1$ bis $R^4$ den vorstehend genannten Definitionsbreiten entsprechen und $R^5$ und $R^6$ gemeinsam für ein anelliertes Ringsystem gemäss einer der vorstehenden Definitionen stehen.

Ganz besonders hervorzuheben sind auch die Verbindungen der Formel Ie, in denen

| | |
|---|---|
| $R^1$ | Nitro, Fluor oder Chlor, |
| $R^2$ | Wasserstoff, Fluor, Chlor oder Methyl, |
| $R^4$ | Trifluormethyl und |
| $R^5$ und $R^6$ | gemeinsam $-(CH_2)_3-$ oder $-(CH_2)_4-$ |

bedeutet.

Namentlich zu nennen sind die folgenden Verbindungen der Formel I:
2-[N-Carbamyl-N-(2-methyl-6-nitrophenyl)-amino]-5,6,7,8-tetrahydro-4-trifluormethyl-chinazolin,
2-[N-Carbamyl-N-(2-nitrophenyl)-amino]-4-trifluormethyl-5H-6,7-dihydro-cyclopenta[d]pyrimidin,
2-[N-Carbamyl-N-(2-nitrophenyl)-amino]-4-chlor-5-(2-chlorethyl)-6-methyl-pyrimidin,
2-[N-Carbamyl-N-(2-nitrophenyl)-amino]-4-chlor-5-methyl-6-trifluormethyl-pyrimidin,
2-[N-Carbamyl-N-(6-methyl-2-nitrophenyl)-amino]-4-ethyl-5-methyl-6-trifluormethyl-pyrimidin,
2-[N-Carbamyl-N-(2-nitrophenyl)-amino]-4-trifluormethyl-5-methyl-5,6,7,8-tetrahydro-chinazolin,
2-[N-Carbamyl-N-(2-nitrophenyl)-amino]-4-chlor-5,6,7,8-tetrahydro-chinazolin,
2-[N-Carbamyl-N-(2-nitrophenyl)-amino]-4-trifluormethyl-5,6,7,8-tetrahydro-chinazolin,

2-[N-Carbamyl-N-(2-nitrophenyl)-amino]-4-trifluormethyl-9H-5,6,7,8-tetrahydro-cyclopenta[d]pyrimidin,
2-[N-Carbamyl-N-(2-nitrophenyl)-amino]-4-trifluormethyl-5,6,7,8,9,10-hexahydro-cyclopenta[d]pyrimidin,
2-[N-Carbamyl-N-(6-methyl-2-nitrophenyl)-amino]-4-trifluormethyl-5H-6,7-dihydro-cyclopenta[d]pyrimidin,
2-[N-Carbamyl-N-(6-methyl-2-nitrophenyl)-amino]-4-trifluormethyl-5,6,7,8-tetrahydro-chinazolin,
2-[N-Carbamyl-N-(6-methyl-2-nitrophenyl)-amino]-4-trifluormethyl-9H-5,6,7,8-tetrahydro-cyclopenta[d]pyrimidin,
2-[N-Carbamyl-N-(2,5-dichlorphenyl)-amino]-4-trifluormethyl-5H-6,7-dihydro-cyclopenta[d]pyrimidin,
2-[N-Carbamyl-N-(2,5-dichlorphenyl)-amino]-4-chlor-5,6,7,8-tetrahydro-chinazolin,
2-[N-Carbamyl-N-(2,5-dichlorphenyl)-amino]-4-trifluormethyl-5,6,7,8-tetrahydro-chinazolin,
2-[N-Carbamyl-N-(2,5-dichlorphenyl)-amino]-4-trifluormethyl-9H-5,6,7,8-tetrahydro-cyclohepta[d]pyrimidin,
2-[N-Carbamyl-N-(2,5-dichlorphenyl)-amino]-4-trifluormethyl-5,6,7,8,9,10 hexahydro-cycloocta[d]pyrimidin,
2-[N-Carbamyl-N-(6-chlor-2-fluorphenyl)-amino]-4-trifluormethyl-5,6,7,8-tetrahydro-chinazolin,
2-[N-Carbamyl-N-(6-chlor-2-fluorphenyl)-amino]-4-trifluormethyl-5H-6,7-dihydro-cyclopenta[d]pyrimidin,
2-[N-Carbamyl-N-(2,5-dichlorphenyl)-amino]-4-trifluormethyl-5H-6,7-dihydro-cyclopenta[d]pyrimidin,
2-[N-Carbamyl-N-(2-nitro-6-methylphenyl)-amino]-4-trifluormethyl-5H-6,7-dihydro-cyclopenta[d]pyrimidin,

Die Verbindungen der Formel I können hergestellt werden, dadurch dass man

a) ein Anilin der Formel II mit Phosgen zu einem Carbaminchlorid der Formel III umsetzt und dieses in einer zweiten Stufe mit $NH_3$ zu

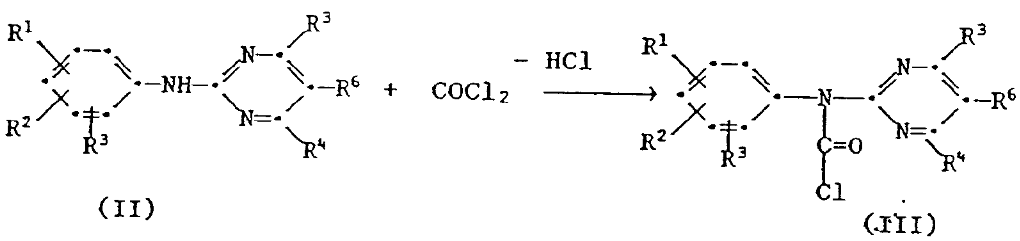


(II) (III)

$$III + NH_3 \xrightarrow{-\ HCl} I$$

einem harnstoff der Formel I umsetzt oder

b) ein Anilin der Formel II mit Halogensulfonylisocyanat XVIII zu einem Halogensulfonylharnstoff der Formel IV umsetzt

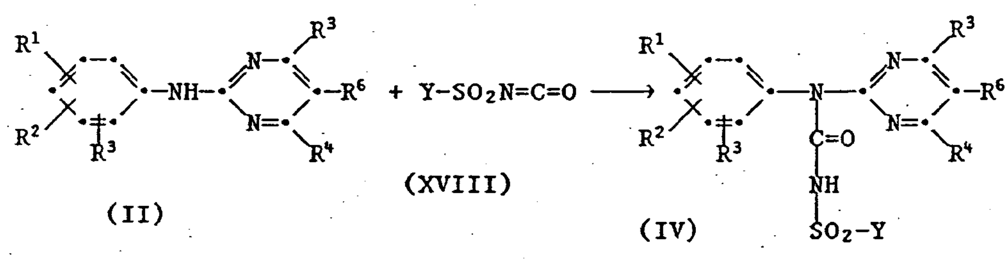

(II) (XVIII) (IV)

$$IV + 2H_2O \xrightarrow[-\ SO_4H_2]{-\ HY} I$$

und diesen in einer zweiten Stufe oder direkt zu einer Verbindung der Formel I hydrolisiert, wobei Y für eine unter den Reaktionsbedingungen abspaltbare Gruppe wie Halogen, vorzugsweise Chlor, steht.

Weiterhin können Harnstoffe der Formel I'

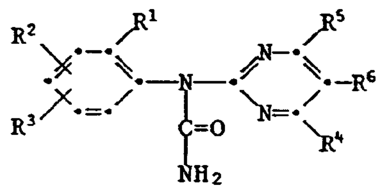

(I'),

worin $R^1$ in der ortho-Stellung des Phenylringes gebunden ist und Nitro bedeutet, hergestellt werden, dadurch dass man

c) einen Sulfonylharnstoff der Formel V unter Einwirkung einer wässrigen Base zu einem Harnstoff der Formel I' umlagert

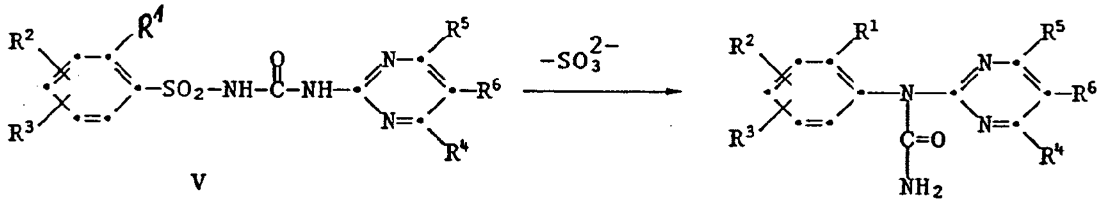


V

I'

wobei als wässrige Basen vorzugsweise NaOh/Wasser oder KOH/Wasser verwendet werden.

Die unter Halogenwasserstoffabspaltung bzw. HY-Eliminierung verlaufenden Umsetzungen II → III, III → I, V → I' und IV → I werden vorzugsweise unter Verwendung säurebindender Mittel (Basen) durchgeführt.

Als solche kommen organische oder anorganische Basen in Betracht, z.B. tertiäre Amine wie Trialkylamine (Trimethylamin, Triethylamin, Tripropylamin usw.), Pyridine (Pyridin, 4-Dimethylaminopyridin, 4-Pyrrolidylaminopyridin usw.), Alkoholate wie z.B. Kalium-tert.-butylat, Natriummethylat oder -ethylat. Die zuvor genannten Reaktionen wie auch die Umsetzung V → I', können auch unter Phasentransferbedingungen mit Basen nach an sich bekannten Verfahren durchgeführt werden (Lit. Dehmlow & Dehmlow, Phase Transfer Catalysis Verlag Chemie, Weinheim, 1983).

Bei den Verfahrensvarianten a), b) und c) können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Die Aniline der Formel II sind ebenso wie die Carbamoylchloride der Formel III und die neuen Harnstoffe der Formel IV wertvolle Zwischenverbindungen.

Einige Aniline der Formel II sind vorbekannt (Tagungsber. Akad. Landwirtschaftswiss. DDR 1984 222 / Chem. Abstr. 102 162 065b; Biomed. Mass. Spectrum 11 (1984), 435-40; DD 151 404; Tetrahedron Lett. 1969 2595-8; DE-OS 18 00 708; Z. Chem. 8 (1968) 103-4).

Neu sind die Aniline der Formel II,

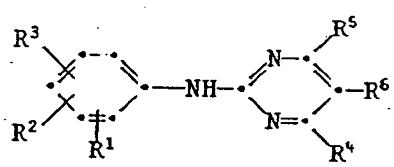

(II),

worin die Reste $R^1$ bis $R^6$ wie zuvor definiert sind, mit der Massgabe, dass wenn $R_1$,$R_2$ oder $R_3$ ortho-Nitro bedeuten, wobei höchstens 2 Substituenten Nitro sein dürfen, die andern Reste nicht Wasserstoff oder $CF_3$ bedeuten, wenn diese in para- und ortho-Position sind.

Die Erfindung betrifft ausser dem die neuen Carbamoylchloride der Formel III

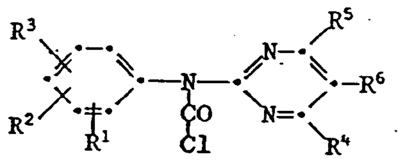

(III),

worin die Reste $R^1$ bis $R^6$ wie zuvor definiert sind und die neuen Harnstoffe der Formel IV

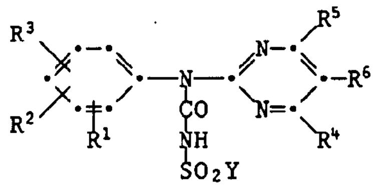

worin die Reste $R^1$ bis $R^6$ wie zuvor definiert sind und Y Halogen bedeutet.

Die neuen Aniline der Formel II können analog zu literaturbekannten Verfahren hergestellt werden, dadurch dass man

d) Guanidine der Formel VI worin die Reste $R^1$ bis $R^3$ wie zuvor definiert sind, mit 1,3-Dicarbonylverbindungen der Formel VII worin die Reste $R^4$ bis $R^6$ wie zuvor definiert sind, umsetzt

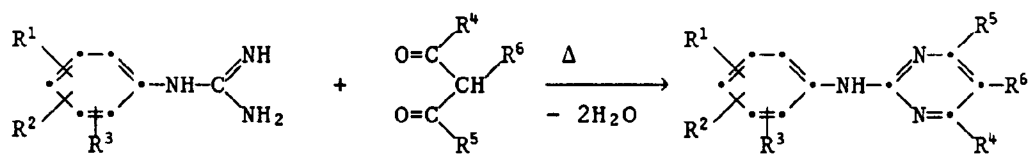


(VI) (VII) (II)

wobei die Kondensationsreaktion gewünschtenfalls in Gegenwart wasserbindender Mittel durchgeführt wird (Lit: D.J. Brown in "The Chemistry of Heterocyclic Compounds" Bd. VI 1962, Interscience Publ. New York; J. Am. Chem. Soc. 69 1819 (1947); J. Am. Chem. Soc. 72 2948 (1950); J. Org. Chem. 29 1439 (1964) oder J. Org. Chem. 29 1883 (1964), Houben-Weyl "Methoden d. org. Chemie Bd. VIII S. 180ff) oder

e) ein Anilin der Formel VIII mit einem Pyrimidin der Formel IX unter Baseneinwirkung umsetzt,

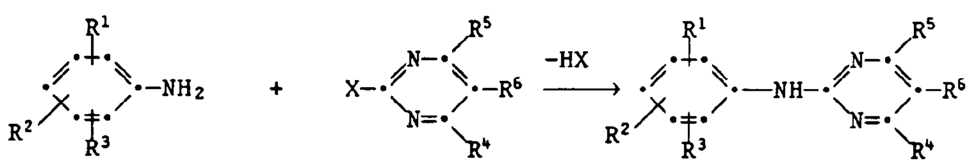


VIII IX II

wobei die Reste $R^1$ bis $R^6$ wie zuvor definiert sind und X für eine nucleofuge Gruppe, wie Halogen, oder $C_1$-$C_4$-Alkylsulfonyl steht.

Geeignete Basen zur Durchführung dieses Verfahrens sind u.a. Kalium-, tert.-Butylat, $Na_2CO_3$; $K_2CO_3$ oder NaH.

Die Verfahren stehen generell zur Synthese von Verbindungen der Formel II zur Verfügung und sind auf alle Edukte der Formel V, VI, VII und VIII (in denen die Reste $R^1$ bis $R^6$ im Formelumfang von Verbindungen I definiert ist) anwendbar.

Die Sulfonylharnstoffe V sind analog zu literaturbekannten Verfahren erhältlich in denen man

f) ein Sulfonamid X, worin $R^2$ und $R^3$ wie zuvor definiert sind, mit Phosgen zu einem Isocyanat XI umsetzt und dieses anschliessend mit einem 2-Aminopyrimidin XII zu V reagieren lässt:

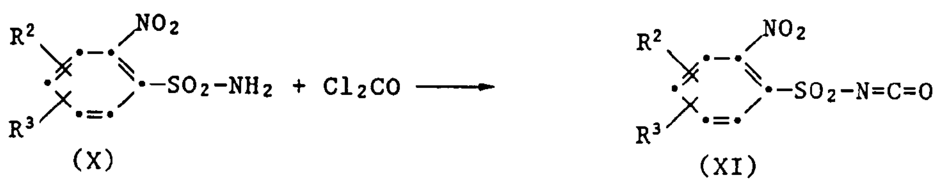

(X) (XI)

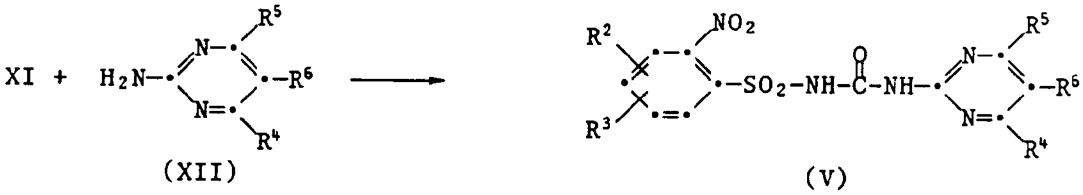

(XII) (V)

oder

g) ein 2-Aminopyrimidin XII, worin $R^4$ bis $R^6$ wie zuvor definiert sind, mit Phosgen zu einem Isocyanat XIII umsetzt und dieses anschliessend mit einem Sulfonamid X, worin $R^2$ und $R^3$ wie zuvor definiert sind, zu V reagieren lässt:

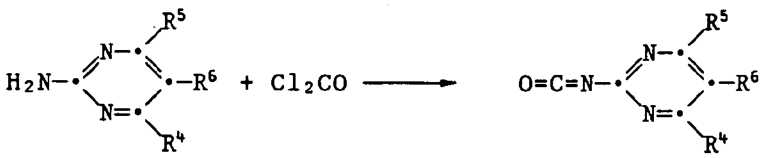

(XII) (XIII)

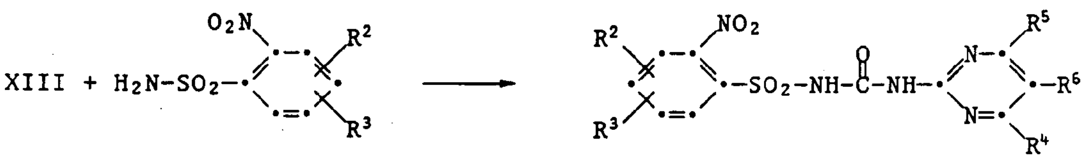

(X) (V)

Die Guanidine VI sind analog zu literaturbekannten Verfahren darstellbar, indem man

h) ein Anilin VIII, worin $R^1$ bis $R^3$ wie zuvor definiert sind, mit Cyanamid umsetzt:

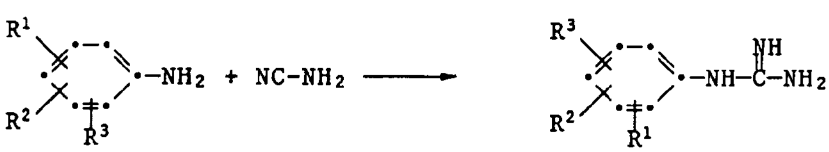

(VIII) (VI)

oder

i) einen Thioharnstoff ($R^7$=H) oder einen Isothioharnstoff ($R^7$=$C_1$-$C_4$-Alkyl) der Formel XIV, worin die Reste $R^1$ bis $R^3$ wie zuvor definiert sind und $R^7$ Wasserstoff oder $C_1$-$C_4$-Alkyl bedeutet, mit Ammoniak zum Guanidin VI umsetzt:

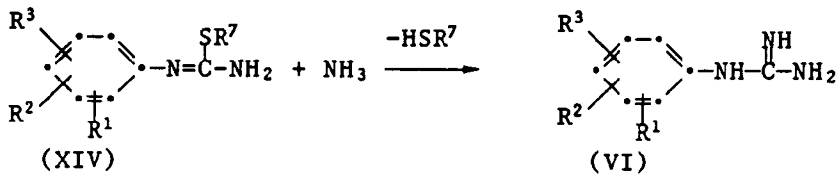


(XIV) (VI)

(Literatur: Verfahren h): Houben Weyl, Methoden der Org. Chemie, Thieme, Stuttgart, Bd. VIII S. 98, 180; Verfahren i): Houben Weyl, Methoden der Org. Chemie, Thieme, Stuttgart, Bd. VIII S. 183).

Die β-Diketone VII sind ebenfalls analog zu literaurbekannten Verfahren herstellbar, in dem man

j) ein Keton XV, worin $R^5$ und $R^6$ wie zuvor definiert sind, mit einer Verbindung der Formel XVI, worin $R^4$ wie zuvor definiert ist und Z für eine unter den Reaktionsbedingungen der Claisen-Kondensation abspaltbare Gruppe, wie $C_1$-$C_4$-Alkoxy, Phenoxy, Benzyloxy oder Halogen bedeutet, unter den Reaktionsbedingungen einer Claisen Kondensation umsetzt.

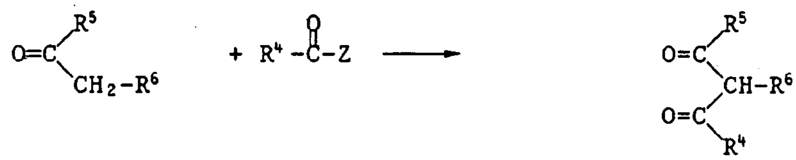

XV XVI VII

(Lit.: C. Ferry Reaktionen der organischen Synthese, Thieme Stuttgart, 1978 S. 312 sowie dort zitierte Literatur).

Die 2-Aminopyrimidine XII, die 2-Isocyanopyrimidine XIII, die 2-Halogenpyrimidine IX' (mit X=Halogen), die 2-Mercaptopyrimidine IX" (mit X=SH), die 2-Alkylthiopyrimidine IX‴ (mit X=$C_1$-$C_4$-Alkylthio) und die 2-Alkylsulfonylpyrimidine IX"" (mit X=$C_1$-$C_4$-Alkyl-$SO_2$-) in denen die Reste $R^4$ bis $R^6$ wie zuvor definiert sind, sind überwiegend neu.

Insbesondere sind die Verbindungen der Formeln XI, XIII und IX neu, in denen die Reste $R^5$ und $R^6$ gemeinsam für einen anellierten carbocyclischen oder heterocyclischen Rest stehen.

Zur Herstellung der Verbindungen der Formeln XIII, IX', IX" und IX‴ stehen dem Fachmann zahlreiche Synthesewege zur Verfügung. Diese sind dem Chemiker allgemein bekannt und in den einschlägigen Handbüchern umfassend beschrieben.

Nachstehendes Syntheseschema (Schema I) zeigt einen Ausschnitt der Möglichkeiten zur Herstellung dieser Verbindungen, wobei jeweils von dem β-Diketon VII ausgegangen wird:

Schema I

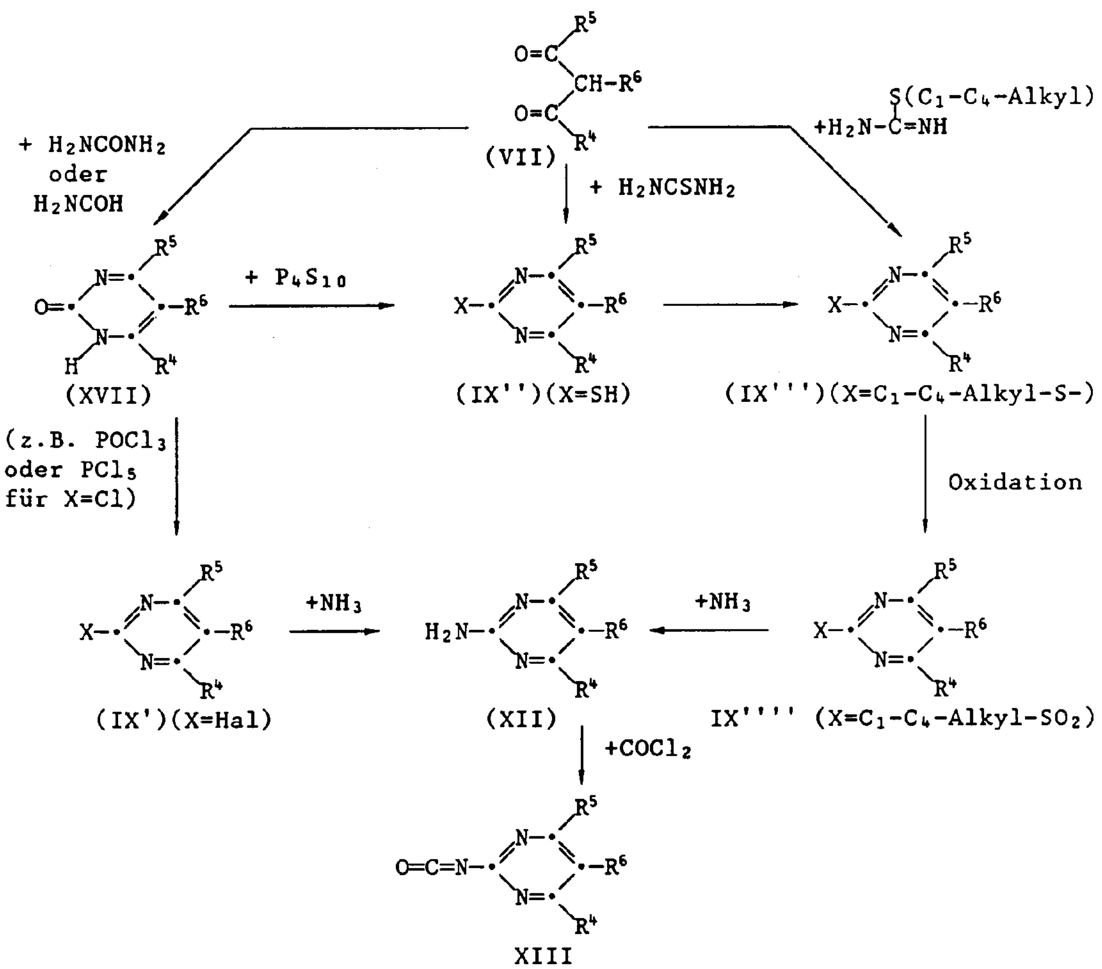


Die Pyridinone XVII, worin die Reste $R^4$ bis $R^6$ wie zuvor definiert sind, sind ebenfalls teilweise neu.

Insbesondere sind diejenigen Pyrimidinone der Formel XVII neu, in denen die Reste $R^5$ und $R^6$ gemeinsam für einen anellierten carbocyclischen oder heterocyclischen Rest stehen.

Des weiteren betrifft die Erfindung herbizide und pflanzenwuchsregulatorische Mittel enthaltend eine Verbindung der Formel I zusammen mit geeigneten Hilfs- und/oder Trägerstoffen.

Die Wirkstoffe der Formel I werden in der Regel mit Aufwandmengen von 0,005 bis 5 kg/ha insbesondere 0,1 bis 3 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und selektiv herbizide Eigenschaften aus, die sie ausgezeichnet zum Einsatz in Kulturen von Nutzpflanzen, insbesondere in Getreide, Baumwolle, Soja, Raps, Mais und Reis befähigen.

Die Verbindungen der Formel I haben ausserdem pflanzenwuchsregulatorische Eigenschaften. Es werden sowohl Monokotyledonen als auch Dikotyledonen in ihrem Wachstum beeinflusst.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumsregulatoren beruht darauf, dass die Nährstoffe in stärkerem Masse der Blüten- und Fruchtbildung zugute kommen, während das vegetative Wachstum eingeschränkt wird.

Bei grösseren Aufwandmengen werden Unkräuter und Gräser in ihrer Entwicklung so geschädigt, dass sie absterben.

In besonders vorteilhafter Weise können die wuchsregulatorischen Verbindungen der Formel I zur Wuchsregulation von Untersaaten in Maiskulturen verwendet werden.

Als Untersaaten in Maiskulturen sind prinzipiell diejenigen Pflanzen geeignet, die den Boden zwischen den einzelnen Maispflanzen bedecken und so in erster Linie der Bodenerosion in Maiskulturen entgegenwirken. Geeignete Pflanzen für die Untersaat sind unter anderem Raps, Klee, Gräser oder Leguminosen. In geeigneten Aufwandmengen hemmen die Verbindungen der Formel I den Neuzuwachs von Gräsern. Dies erlaubt es in Rasenkulturen (Parks, Gärten etc.) die Zahl der erforderlichen Schnitte zu vermindern, beziehungsweise die Zeiträume zwischen den einzelnen Schnitten zu verlängern. In besonders vorteilhafter Weise können hierzu Granulatformulierungen der Wirkstoffe der Formel I verwendet werden. Das Granulat kann entweder den Wirkstoff allein, neben den üblichen Hilfsund Trägerstoffen, enthalten oder der Wirkstoff wird zusammen mit einem Mineraldünger, und/oder gegebenenfalls weiteren Wirkstoffen zur Bekämpfung von Moos oder anderen in Rasenkulturen unerwünschten Pflanzenwuchses, als Granulat formuliert. Die Anwendung als Streugranulat erlaubt es, mit Hilfe der bei Rasenkulturen üblichen Bearbeitungsgeräte, während längerer Zeit den Neuzuwachs der Gräser zu hemmen. Das Granulat kann dabei in an sich bekannter Weise hergestellt werden, es weist vorzugsweise eine Korngrösse von 0,1 bis 2,0 mm, insbesondere 0,25 bis 1,0 mm auf.

Die Erfindung betrifft auch herbizide und pflanzenwachstumsregulierende Mittel, welche einen Wirkstoff der Formel I enthalten, sowie Verfahren zur pre- und post-emergenten Unkrautbekämpfung und zur Beinflussung des Pflanzenwuchses von monokotylen und dikotylen Pflanzen, insbesondere Gräsern, tropischen Bodenbedeckern und Geiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

So können die Aktivsubstanzen der Formel I auch auf mineralische Dünger aufgebracht (aufgebeizt) werden. Das so erhältliche Mittel ist in vorteilhafter Weise als Wuchsregulator bei Gräsern geeignet.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von

Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Klebstoffe sind insbesondere diejenigen Hilfsstoffe, welche beim Granulieren den Zusammenhalt des Trägermaterials, der Hilfsstoffe und der Wirkstoffe bewirken, wie Gummi arabicum oder Carboxymethylcellulose.

Die in der Formulierungstechnik gebräuchlichen Tenside sind u.a. in folgenden Publikationen beschrieben:

"1987 International Mc Cutcheon's Emulsifiers and Detergents", Glen Rock, N.J., USA.
Dr. Helmut Stache "Tensid Taschenbuch"
Carl Hanser Verlag, München/Wien 1981.

Die Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

Emulgierbare Konzentrate:

| | |
|---|---|
| Wirkstoff der Formel I: | 1 bis 20 % bevorzugt 5 bis 10 % |
| oberflächenaktives Mittel: | 5 bis 30 %,vorzugsweise 10 bis 20 % |
| flüssiges Trägermittel: | 50 bis 94 %,vorzugsweise 70 bis 85 %. |

Stäube:

| | |
|---|---|
| Wirkstoff der Formel I: | 0,1 bis 10 %,vorzugsweise 0,1 bis 1 % |
| festes Trägermittel: | 99,9 bis 90 %, vorzugsweise 99,9 bis 99 %. |

Suspensions-Konzentrate:

| | |
|---|---|
| Wirkstoff der Formel I: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

Benetzbare Pulver:

| | |
|---|---|
| Wirkstoff der Formel I: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 %. |

Granulate:

| | |
|---|---|
| Wirkstoff der Formel I: | 0,5 bis 30 %, vorzugsweise 3 bis 15 % |
| festes Trägermittel: | 99,5 bis 70 %, vorzugsweise 97 bis 85%. |

Streugranulat:

| | |
|---|---|
| Wirkstoff der Formel I: | 0,01 bis 30 %, vorzugsweise 0,05 bis 15% |
| Klebstoff: | 0,05 bis 5 %, vorzugsweise 0,1 bis 2 % |
| oberflächenaktives Mittel: | 0,5 bis 20%, vorzugsweise 1 bis 15 % |
| festes Trägermittel: | 99,44 bis 45 %, vorzugsweise 95 bis 65 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

Die - unkorrigierten - Schmelzpunkte in den nachfolgenden Herstellungsbeispielen sind in °C angegeben.

H. 1. Verbindungen der Formel I

H. 1.1. 2-[N-Carbamyl-N-(2-methyl-6-nitrophenyl)-amino]-5,6,7,8-tetrahydro-4-trifluormethyl-chinazolin

Zu einer Lösung von 7,0 g 2-(2-Methyl-6-nitrophenylamino)-5,6,7,8-tetrahydro-4-trifluormethyl-chinazolin in 100 ml Essigsäureethylester werden bei 3°C 2,2 ml Chlorsulfonylisocyanat gegeben. Es wird eine Stunde unter Kühlung auf dem Eisbad gerührt und danach mit 50 ml Essigsäureethylester und 30 ml Wasser versetzt. Die organische Phase wird abgetrennt mit Sole gewaschen, getrocknet und im Vakuum eingeengt. Der Rückstand wird mit Hexan verrieben, wobei das Produkt auskristallisiert.

Man isoliert 7,8 g der Titelverbindung der Formel

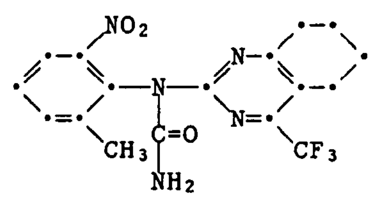

als Kristalle vom Smp. 161-162°C (Zers.) (Verb. No. 1.563).

Die Verbindungen der Tabelle 1 sind analog herstellbar:

Tabelle 1: Verbindungen der Formel

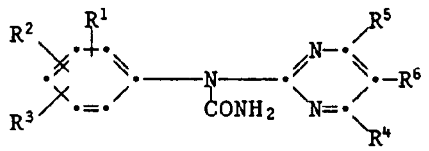

(I)

| Verb.No | $R^1$ | $R^2$ | $R^3$ | $R^4$ | $R^5$ | $R^6$ | Phys. Daten |
|---|---|---|---|---|---|---|---|
| 1.001 | 2-Cl | H | H | $CH_3$ | Cl | $CH_3$ | |
| 1.002 | 2-Cl | H | H | $CH_3$ | $CF_3$ | $CH_3$ | |
| 1.003 | 2-Cl | H | H | $C_2H_5$ | $CF_3$ | $CH_3$ | |
| 1.004 | 2-Cl | H | H | $OCH_3$ | $CF_3$ | $CH_3$ | |
| 1.005 | 2-Cl | H | H | $CH_3$ | $CF_3$ | $C_2H_5$ | |
| 1.006 | 2-F | H | H | $CH_3$ | $CF_3$ | $CH_3$ | |
| 1.007 | 2-F | H | H | $CH_3$ | Cl | $CH_3$ | |
| 1.008 | 2-F | H | H | $C_2H_5$ | $CF_3$ | $CH_3$ | |
| 1.009 | 2-F | H | H | $SCH_3$ | $CF_3$ | $CH_3$ | |
| 1.010 | 2-F | H | H | $OCH_3$ | $CH_3$ | CN | |
| 1.011 | 2-Br | H | H | $CH_3$ | Cl | $CH_3$ | |
| 1.012 | 2-Br | H | H | $CH_3$ | $CF_3$ | $CH_3$ | |
| 1.013 | 2-Br | H | H | $C_2H_5$ | $CF_3$ | $CH_3$ | |
| 1.014 | 2-Br | H | H | $C_2H_5$ | $CF_2Cl$ | $CH_3$ | |
| 1.015 | 2-Br | H | H | $C_2H_5$ | CHFCl | $CH_3$ | |
| 1.016 | 2-Br | H | H | $C_2H_5$ | $C_2F_5$ | $CH_3$ | |
| 1.017 | 2-Br | H | H | $C_2H_5$ | $CHCl_2$ | $CH_3$ | |
| 1.018 | 2-Br | H | H | $CH_3$ | $CF_2CF_3$ | $CH_3$ | |
| 1.019 | 2-Br | H | H | $OCH_3$ | $CF_3$ | $CH_3$ | |
| 1.020 | 2-Br | H | H | $SCH_3$ | $CF_3$ | $CH_3$ | |
| 1.021 | 2-Br | H | H | $SCH_3$ | $CH_3$ | $CH_3$ | |
| 1.022 | 2-Br | H | H | $SO_2CH_3$ | $CH_3$ | $CH_3$ | |
| 1.023 | 2-Br | H | H | Cyclopropyl | $CF_3$ | $CH_3$ | |
| 1.024 | 2-Br | H | H | $C_3H_7(i)$ | $CF_3$ | $CH_3$ | |
| 1.025 | 2-Br | H | H | $CH_3$ | Cl | $CH_2CH_2Cl$ | |
| 1.026 | 2-Br | H | H | $CH_3$ | Cl | $CH_2CH_2OCH_3$ | |
| 1.027 | 2-Br | H | H | $OCH_3$ | $CH_3$ | $CH_2CH_2OCH_3$ | |
| 1.028 | 2-J | H | H | $CH_3$ | Cl | $CH_3$ | |
| 1.029 | 2-J | H | H | $CH_3$ | $CF_3$ | $CH_3$ | |
| 1.030 | 2-J | H | H | $C_2H_5$ | $CF_3$ | $CH_3$ | |
| 1.031 | 2-J | H | H | $OCH_3$ | $CH_3$ | $CH_3$ | |
| 1.032 | 2-J | H | H | $CH_3$ | Cl | $C_2H_5$ | |
| 1.033 | 2-J | H | H | Cyclopropyl | $CF_3$ | $CH_3$ | |
| 1.034 | $2-CF_3$ | H | H | $CH_3$ | Cl | $CH_3$ | |
| 1.035 | $2-CF_3$ | H | H | $OCH_3$ | $CH_3$ | $CH_3$ | |
| 1.036 | $2-CF_3$ | H | H | $SCH_3$ | $CH_3$ | $CH_3$ | |
| 1.037 | $2-CF_3$ | H | H | $SO_2CH_3$ | $CH_3$ | $CH_3$ | |
| 1.038 | $2-CF_3$ | H | H | $CH_3$ | $CF_3$ | $CH_3$ | |
| 1.039 | $2-CF_3$ | H | H | $C_2H_5$ | $CF_3$ | $CH_3$ | |
| 1.040 | $2-CF_3$ | H | H | $C_2H_5$ | $CF_2Cl$ | $CH_3$ | |
| 1.041 | $2-CF_3$ | H | H | $C_2H_5$ | $CHCl_2$ | $CH_3$ | |

Tabelle 1 (Fortsetzung)

| Verb.No | $R^1$ | $R^2$ | $R^3$ | $R^4$ | $R^5$ | $R^6$ | Phys. Daten |
|---|---|---|---|---|---|---|---|
| 1.042 | 2-$CF_3$ | H | H | $C_2H_5$ | $CF_2CF_3$ | $CH_3$ | |
| 1.043 | 2-$CF_3$ | H | H | $OCH_3$ | $CH_3$ | CN | |
| 1.044 | 2-$CF_3$ | H | H | $OCH_3$ | $CH_3$ | $NO_2$ | |
| 1.045 | 2-$CF_3$ | H | H | $OCH_3$ | $CH_3$ | Cl | |
| 1.046 | 2-$NO_2$ | H | H | $CH_3$ | Cl | $CH_3$ | |
| 1.047 | 2-$NO_2$ | H | H | $CH_3$ | $CF_3$ | $CH_3$ | |
| 1.048 | 2-$NO_2$ | H | H | $OCH_3$ | $CF_3$ | $CH_3$ | |
| 1.049 | 2-$NO_2$ | H | H | $SCH_3$ | $CH_3$ | $CH_3$ | |
| 1.050 | 2-$NO_2$ | H | H | $SOCH_3$ | $CH_3$ | $CH_3$ | |
| 1.051 | 2-$NO_2$ | H | H | $SO_2CH_3$ | $CH_3$ | $CH_3$ | |
| 1.052 | 2-$NO_2$ | H | H | $SCH_3$ | $CF_3$ | $CH_3$ | |
| 1.053 | 2-$NO_2$ | H | H | $CH_3$ | $CF_3$ | Phenyl | |
| 1.054 | 2-$NO_2$ | H | H | $OCH_3$ | $CF_3$ | 4-Cl-Phenyl | |
| 1.055 | 2-$NO_2$ | H | H | Cl | $CF_3$ | 4-Cl-Phenyl | |
| 1.056 | 2-$NO_2$ | H | H | $CH_3$ | Cl | $CH_2CH_2Cl$ | Fp. 145-146°C |
| 1.057 | 2-$NO_2$ | H | H | $OCH_3$ | $CH_3$ | $CH_2CH_2OCH_3$ | |
| 1.058 | 2-$NO_2$ | H | H | $C_2H_5$ | $CF_3$ | $CH_3$ | Fp. 160-161°C |
| 1.059 | 2-$NO_2$ | H | H | $C_2H_5$ | $CF_2Cl$ | $CH_3$ | |
| 1.060 | 2-$NO_2$ | H | H | $C_2H_5$ | $CHCl_2$ | $CH_3$ | |
| 1.061 | 2-$NO_2$ | H | H | $C_2H_5$ | CHFCl | $CH_3$ | |
| 1.062 | 2-$NO_2$ | H | H | $C_2H_5$ | $C_2F_5$ | $CH_3$ | |
| 1.063 | 2-$NO_2$ | H | H | $C_2H_5$ | $C_3F_7$(n) | $CH_3$ | |
| 1.064 | 2-$NO_2$ | H | H | $C_2H_5$ | $CCl_2CF_3$ | $CH_3$ | |
| 1.065 | 2-$NO_2$ | H | H | $C_3H_7$(n) | $CF_3$ | $CH_3$ | |
| 1.066 | 2-$NO_2$ | H | H | $C_3H_7$(n) | $CF_3$ | $C_2H_5$ | |
| 1.067 | 2-$NO_2$ | H | H | $C_3H_7$(i) | $CF_3$ | $CH_3$ | Fp. 162-163°C |
| 1.068 | 2-$NO_2$ | H | H | Cyclopropyl | $CF_3$ | $CH_3$ | |
| 1.069 | 2-$NO_2$ | H | H | $CH_3$ | $OCHF_2$ | $CH_3$ | |
| 1.070 | 2-$NO_2$ | H | H | $OCH_3$ | $CH_3$ | $NO_2$ | |
| 1.071 | 2-$NO_2$ | H | H | $OCH_3$ | $CH_3$ | Cl | |
| 1.072 | 2-$NO_2$ | H | H | $OCH_3$ | $CH_3$ | CN | |
| 1.073 | 2-$NO_2$ | H | H | $CH_3$ | Cl | CN | |
| 1.074 | 2-$NO_2$ | H | H | $CH_3$ | Cl | $NO_2$ | |
| 1.075 | 2-$NO_2$ | H | H | $CH_3$ | Cl | Cl | |
| 1.076 | 2-$NO_2$ | H | H | $SCH_3$ | $CH_3$ | Cl | |
| 1.077 | 2-$NO_2$ | H | H | $SO_2CH_3$ | $CH_3$ | Cl | |
| 1.078 | 2-$NO_2$ | H | H | $OCH_3$ | $CH_3$ | $COOCH_3$ | |
| 1.079 | 2-$NO_2$ | H | H | $CH_3$ | $CH_3$ | $COOCH_3$ | |
| 1.080 | 2-$NO_2$ | H | H | $CH_3$ | $CF_2Cl$ | $CH_3$ | |
| 1.081 | 2-$OCH_3$ | H | H | $CH_3$ | Cl | $CH_3$ | |
| 1.082 | 2-$OCH_3$ | H | H | $CH_3$ | $CF_3$ | $CH_3$ | |
| 1.083 | 2-$OCH_3$ | H | H | $C_2H_5$ | $CF_3$ | $CH_3$ | |
| 1.084 | 2-$OCH_3$ | H | H | $OCH_3$ | $CH_3$ | $CH_3$ | |
| 1.085 | 2-$OCH_3$ | H | H | $OCH_3$ | $CF_3$ | $CH_3$ | |
| 1.086 | 2-$OCF_3$ | H | H | $CH_3$ | Cl | $CH_3$ | |
| 1.087 | 2-$OCF_3$ | H | H | $CH_3$ | $CF_3$ | $CH_3$ | |
| 1.088 | 2-$OCF_3$ | H | H | $OCH_3$ | $CH_3$ | $CH_3$ | |

Tabelle 1 (Fortsetzung)

| Verb.No | $R^1$ | $R^2$ | $R^3$ | $R^4$ | $R^5$ | $R^6$ | Phys. Daten |
|---|---|---|---|---|---|---|---|
| 1.089 | 2-$OCF_3$ | H | H | $SCH_3$ | $CH_3$ | $CH_3$ | |
| 1.090 | 2-$OCF_3$ | H | H | $SO_2CH_3$ | $CH_3$ | $CH_3$ | |
| 1.091 | 2-$OCF_3$ | H | H | $C_2H_5$ | $CF_3$ | $CH_3$ | |
| 1.092 | 2-$OCF_3$ | H | H | $C_2H_5$ | $CF_2Cl$ | $CH_3$ | |
| 1.093 | 2-$OCF_3$ | H | H | $SCH_3$ | $CF_3$ | $CH_3$ | |
| 1.094 | 2-$OCF_3$ | H | H | $OCH_3$ | $CF_3$ | $CH_3$ | |
| 1.095 | 2-$OCF_3$ | H | H | $OC_2H_5$ | $CF_3$ | $CH_3$ | |
| 1.096 | 2-$OCHF_2$ | H | H | $CH_3$ | Cl | $CH_3$ | |
| 1.097 | 2-$OCHF_2$ | H | H | $CH_3$ | Cl | $C_2H_5$ | |
| 1.098 | 2-$OCHF_2$ | H | H | $OCH_3$ | $CH_3$ | $C_2H_5$ | |
| 1.099 | 2-$OCHF_2$ | H | H | $SCH_3$ | $CH_3$ | $CH_3$ | |
| 1.100 | 2-$OCHF_2$ | H | H | $SO_2CH_3$ | $CH_3$ | $CH_3$ | |
| 1.101 | 2-$OCHF_2$ | H | H | $CH_3$ | $CF_3$ | $CH_3$ | |
| 1.102 | 2-$OCHF_2$ | H | H | $C_2H_5$ | $CF_3$ | $CH_3$ | |
| 1.103 | 2-$OCHF_2$ | H | H | $C_2H_5$ | $CF_2Cl$ | $CH_3$ | |
| 1.104 | 2-$OCHF_2$ | H | H | $CH_3$ | Cl | $CH_2CH_2Cl$ | |
| 1.105 | 2-$OCHF_2$ | H | H | $OCH_3$ | $CH_3$ | $CH_2CH_2OCH_3$ | |
| 1.106 | 2-$OCHF_2$ | H | H | $OC_2H_5$ | $CH_3$ | $CH_2CH_2OC_2H_5$ | |
| 1.107 | 2-$OCHF_2$ | H | H | Cyclopropyl | $CF_3$ | $CH_3$ | |
| 1.108 | 2-$OCHF_2$ | H | H | $C_3H_7$(i) | $CF_3$ | $CH_3$ | |
| 1.109 | 2-$OC_2H_5$ | H | H | $CH_3$ | Cl | $CH_3$ | |
| 1.110 | 2-$OC_2H_5$ | H | H | $CH_3$ | $CF_3$ | $CH_3$ | |
| 1.111 | 2-$OC_2H_5$ | H | H | $OCH_3$ | $CH_3$ | $CH_3$ | |
| 1.112 | 2-$OC_2H_5$ | H | H | $OCH_3$ | $CF_3$ | $CH_3$ | |
| 1.113 | 2-CN | H | H | $CH_3$ | $CF_3$ | $CH_3$ | |
| 1.114 | 2-CN | H | H | $C_2H_5$ | $CF_3$ | $CH_3$ | |
| 1.115 | 2-CN | H | H | $OCH_3$ | $CH_3$ | $CH_3$ | |
| 1.116 | 2-CN | H | H | $OCH_3$ | $CF_3$ | $CH_3$ | |
| 1.117 | 2-CN | H | H | $SCH_3$ | $CF_3$ | $CH_3$ | |
| 1.118 | 2-CN | H | H | Cyclopropyl | $CF_3$ | $CH_3$ | |
| 1.119 | 2-CN | H | H | $OCH_3$ | $CH_3$ | Cl | |
| 1.120 | 2-$COOCH_3$ | H | H | $CH_3$ | $CF_3$ | $CH_3$ | |
| 1.121 | 2-$COOCH_3$ | H | H | $OCH_3$ | $CF_3$ | $CH_3$ | |
| 1.122 | 2-$COOCH_3$ | H | H | $C_2H_5$ | $CF_3$ | $CH_3$ | |
| 1.123 | 2-$COOCH_3$ | H | H | $CH_3$ | $CF_3$ | Phenyl | |
| 1.124 | 2-$CON(CH_3)_2$ | H | H | $CH_3$ | Cl | $CH_3$ | |
| 1.125 | 2-$CON(CH_3)_2$ | H | H | $CH_3$ | $CF_3$ | $CH_3$ | |
| 1.126 | 2-$CON(CH_3)_2$ | H | H | $OCH_3$ | $CF_3$ | $CH_3$ | |
| 1.127 | 2-$CON(CH_3)_2$ | H | H | $OCH_3$ | $CF_3$ | $C_2H_5$ | |
| 1.128 | 2-$CH_3$ | H | H | $CH_3$ | Cl | $CH_3$ | |
| 1.129 | 2-$CH_3$ | H | H | $CH_3$ | $CF_3$ | $CH_3$ | |
| 1.130 | 2-$CH_3$ | H | H | $C_2H_5$ | $CF_3$ | $CH_3$ | |
| 1.131 | 2-$CH_3$ | H | H | $CH_3$ | $CF_2Cl$ | $CH_3$ | |
| 1.132 | 2-$CH_3$ | H | H | $OCH_3$ | $CF_3$ | $CH_3$ | |
| 1.133 | 2-$C_2H_5$ | H | H | $CH_3$ | Cl | $CH_3$ | |
| 1.134 | 2-$C_2H_5$ | H | H | $CH_3$ | $CF_3$ | $CH_3$ | |
| 1.135 | 2-$C_2H_5$ | H | H | $OCH_3$ | $CF_3$ | $CH_3$ | |

Tabelle 1 (Fortsetzung)

| Verb.No | $R^1$ | $R^2$ | $R^3$ | $R^4$ | $R^5$ | $R^6$ | Phys. Daten |
|---|---|---|---|---|---|---|---|
| 1.136 | 2-$C_2H_5$ | H | H | $SCH_3$ | $CF_3$ | $CH_3$ | |
| 1.137 | 3-Cl | H | H | $CH_3$ | $CF_3$ | $CH_3$ | |
| 1.138 | 3-Br | H | H | $CH_3$ | $CF_3$ | $CH_3$ | |
| 1.139 | 3-$CF_3$ | H | H | $C_2H_5$ | $CF_3$ | $CH_3$ | |
| 1.140 | 3-Cl | 5-Cl | H | $CH_3$ | Cl | $CH_3$ | |
| 1.141 | 3-Cl | 5-Cl | H | $CH_3$ | $CF_3$ | $CH_3$ | |
| 1.142 | 3-Cl | 5-Cl | H | $C_2H_5$ | $CF_3$ | $CH_3$ | |
| 1.143 | 3-Cl | 5-Cl | H | $OCH_3$ | $CF_3$ | $CH_3$ | |
| 1.144 | 2-Cl | 5-Cl | H | $CH_3$ | Cl | $CH_3$ | |
| 1.145 | 2-Cl | 5-Cl | H | $CH_3$ | $CF_3$ | $CH_3$ | |
| 1.146 | 2-Cl | 5-Cl | H | $OCH_3$ | $CF_3$ | $CH_3$ | |
| 1.147 | 2-Cl | 5-Cl | H | $SCH_3$ | $CH_3$ | $CH_3$ | |
| 1.148 | 2-Cl | 5-Cl | H | $SOCH_3$ | $CH_3$ | $CH_3$ | |
| 1.149 | 2-Cl | 5-Cl | H | $SO_2CH_3$ | $CH_3$ | $CH_3$ | |
| 1.150 | 2-Cl | 5-Cl | H | $SC_2H_5$ | $CF_3$ | $CH_3$ | |
| 1.151 | 2-Cl | 5-Cl | H | $SC_3H_7$(i) | $CF_3$ | $CH_3$ | |
| 1.152 | 2-Cl | 5-Cl | H | $OC_2H_5$ | $CF_3$ | $CH_3$ | |
| 1.153 | 2-Cl | 5-Cl | H | $SOC_2H_5$ | $CF_3$ | $CH_3$ | |
| 1.154 | 2-Cl | 5-Cl | H | $OC_3H_7$(i) | $CF_3$ | $CH_3$ | |
| 1.155 | 2-Cl | 5-Cl | H | $CH_3$ | Cl | Phenyl | |
| 1.156 | 2-Cl | 5-Cl | H | $OCH_3$ | $CH_3$ | Phenyl | |
| 1.157 | 2-Cl | 5-Cl | H | $CH_3$ | Cl | $CH_2CH_2Cl$ | |
| 1.158 | 2-Cl | 5-Cl | H | $OCH_3$ | $CH_3$ | $CH_2CH_2OCH_3$ | |
| 1.159 | 2-Cl | 5-Cl | H | $C_2H_5$ | $CF_3$ | $CH_3$ | |
| 1.160 | 2-Cl | 5-Cl | H | $CH_3$ | $CF_2Cl$ | $CH_3$ | |
| 1.161 | 2-Cl | 5-Cl | H | $CH_3$ | $C_2H_5$ | $CH_3$ | |
| 1.162 | 2-Cl | 5-Cl | H | $CH_3$ | $C_3F_7$(n) | $CH_3$ | |
| 1.163 | 2-Cl | 5-Cl | H | $C_2H_5$ | $CF_2Cl$ | $CH_3$ | |
| 1.164 | 2-Cl | 5-Cl | H | $C_2H_5$ | $CHF_2$ | $CH_3$ | |
| 1.165 | 2-Cl | 5-Cl | H | $CH_3$ | $CCl_2CF_3$ | $CH_3$ | |
| 1.166 | 2-Cl | 5-Cl | H | $C_3H_7$(n) | $CF_3$ | $CH_3$ | |
| 1.167 | 2-Cl | 5-Cl | H | $C_3H_7$(i) | $CF_3$ | $CH_3$ | |
| 1.168 | 2-Cl | 5-Cl | H | Cyclopropyl | $CF_3$ | $CH_3$ | |
| 1.169 | 2-Cl | 5-Cl | H | $CH_3$ | $CF_3$ | $C_2H_5$ | |
| 1.170 | 2-Cl | 5-Cl | H | $CH_3$ | $CF_3$ | $C_3H_7$(i) | |
| 1.171 | 2-Cl | 5-Cl | H | $CH_3$ | $OCHF_2$ | $CH_3$ | |
| 1.172 | 2-Cl | 5-Cl | H | $OCH_3$ | $CH_3$ | $NO_2$ | |
| 1.173 | 2-Cl | 5-Cl | H | $OCH_3$ | $CH_3$ | Cl | |
| 1.174 | 2-Cl | 5-Cl | H | $OCH_3$ | $CH_3$ | CN | |
| 1.175 | 2-Cl | 5-Cl | H | $OC_2H_5$ | $CH_3$ | CN | |
| 1.176 | 2-Cl | 5-Cl | H | $CH_3$ | Cl | CN | |
| 1.177 | 2-Cl | 5-Cl | H | $CH_3$ | Cl | $NO_2$ | |
| 1.178 | 2-Cl | 5-Cl | H | $CH_3$ | Cl | Cl | |
| 1.179 | 2-Cl | 5-Cl | H | $C_2H_5$ | Cl | Cl | |
| 1.180 | 2-Cl | 5-Cl | H | $C_2H_5$ | Cl | Br | |
| 1.181 | 2-Cl | 5-Cl | H | $OCH_3$ | $CH_3$ | $COOCH_3$ | |
| 1.182 | 2-Cl | 5-Cl | H | $CH_3$ | $CH_3$ | $COOC_2H_5$ | |

Tabelle 1 (Fortsetzung)

| Verb.No | $R^1$ | $R^2$ | $R^3$ | $R^4$ | $R^5$ | $R^6$ | Phys. Daten |
|---|---|---|---|---|---|---|---|
| 1.183 | 2-Cl | 6-Cl | H | $CH_3$ | Cl | $CH_3$ | |
| 1.184 | 2-Cl | 6-Cl | H | $CH_3$ | $CF_3$ | $CH_3$ | |
| 1.185 | 2-Cl | 6-Cl | H | $CH_3$ | $CF_3$ | $C_2H_5$ | |
| 1.186 | 2-Cl | 6-Cl | H | $OCH_3$ | $CF_3$ | $CH_3$ | |
| 1.187 | 2-Cl | 6-Cl | H | $SCH_3$ | $CF_3$ | $CH_3$ | |
| 1.188 | 2-Cl | 6-Cl | H | $CF_3$ | Cl | $CH_3$ | Fp. 182-183°C |
| 1.189 | 2-Cl | 6-Cl | H | $SC_4H_9(n)$ | $CF_3$ | $CH_3$ | |
| 1.190 | 2-Cl | 6-Cl | H | $SC_2H_5$ | $CH_3$ | $CH_3$ | |
| 1.191 | 2-Cl | 6-Cl | H | $SOC_2H_5$ | $CH_3$ | $CH_3$ | |
| 1.192 | 2-Cl | 6-Cl | H | $SO_2C_2H_5$ | $CH_3$ | $CH_3$ | |
| 1.193 | 2-Cl | 6-Cl | H | $C_2H_5$ | $CF_3$ | $CH_3$ | |
| 1.194 | 2-Cl | 6-Cl | H | $C_3H_7(n)$ | $CF_3$ | $CH_3$ | |
| 1.195 | 2-Cl | 6-Cl | H | $C_4H_9(n)$ | $CF_3$ | $CH_3$ | |
| 1.196 | 2-Cl | 6-Cl | H | Cyclopropyl | $CF_3$ | $CH_3$ | |
| 1.197 | 2-Cl | 6-Cl | H | $C_3H_7(i)$ | $CF_3$ | $CH_3$ | Fp. 164-165°C |
| 1.198 | 2-Cl | 6-Cl | H | $CH_3$ | $CF_2Cl$ | $CH_3$ | |
| 1.199 | 2-Cl | 6-Cl | H | $CH_3$ | $CHF_2$ | $CH_3$ | |
| 1.200 | 2-Cl | 6-Cl | H | $CH_3$ | $CHCl_2$ | $CH_3$ | |
| 1.201 | 2-Cl | 6-Cl | H | $CH_3$ | CHFCl | $CH_3$ | |
| 1.202 | 2-Cl | 6-Cl | H | $CH_3$ | $CCl_2CF_3$ | $CH_3$ | |
| 1.203 | 2-Cl | 6-Cl | H | $CH_3$ | $OCHF_2$ | $CH_3$ | |
| 1.204 | 2-Cl | 6-Cl | H | $OCH_3$ | $CH_3$ | $NO_2$ | |
| 1.205 | 2-Cl | 6-Cl | H | $OCH_3$ | $CF_3$ | $NO_2$ | |
| 1.206 | 2-Cl | 6-Cl | H | $OCH_3$ | $CF_3$ | Cl | |
| 1.207 | 2-Cl | 6-Cl | H | $OCH_3$ | $CH_3$ | CN | |
| 1.208 | 2-Cl | 6-Cl | H | $CH_3$ | Cl | CN | |
| 1.209 | 2-Cl | 6-Cl | H | $CH_3$ | Cl | $NO_2$ | |
| 1.210 | 2-Cl | 6-Cl | H | $CH_3$ | Cl | Cl | |
| 1.211 | 2-Cl | 6-Cl | H | $CH_3$ | Cl | Br | |
| 1.212 | 2-Cl | 6-Cl | H | $OCH_3$ | $CH_3$ | $COOCH_3$ | |
| 1.213 | 2-Cl | 3-Cl | H | $CH_3$ | Cl | $CH_3$ | |
| 1.214 | 2-Cl | 3-Cl | H | $CH_3$ | $CF_3$ | $CH_3$ | |
| 1.215 | 2-Cl | 3-Cl | H | $C_2H_5$ | $CF_3$ | $CH_3$ | |
| 1.216 | 2-Cl | 3-Cl | H | $C_2H_5$ | $CF_2Cl$ | $CH_3$ | |
| 1.217 | $2-NO_2$ | $3-CH_3$ | H | $CH_3$ | Cl | $CH_3$ | |
| 1.218 | $2-NO_2$ | $3-CH_3$ | H | $CH_3$ | $CF_3$ | $CH_3$ | |
| 1.219 | $2-NO_2$ | $3-CH_3$ | H | $C_2H_5$ | $CF_3$ | $CH_3$ | |
| 1.220 | $2-NO_2$ | $3-CH_3$ | H | $C_2H_5$ | $CF_2Cl$ | $CH_3$ | |
| 1.221 | $2-NO_2$ | 3-Cl | H | $CH_3$ | Cl | $CH_3$ | |
| 1.222 | $2-NO_2$ | 3-Cl | H | $CH_3$ | $CF_3$ | $CH_3$ | |
| 1.223 | $2-NO_2$ | 3-Cl | H | $OCH_3$ | $CH_3$ | $CH_3$ | |
| 1.224 | $2-NO_2$ | 3-Cl | H | $C_2H_5$ | $CF_3$ | $CH_3$ | |
| 1.225 | $2-NO_2$ | 3-Cl | H | $C_2H_5$ | $CF_2Cl$ | $CH_3$ | |
| 1.226 | $2-NO_2$ | 5-Cl | H | $CH_3$ | Cl | $CH_3$ | |
| 1.227 | $2-NO_2$ | 5-Cl | H | $CH_3$ | $CF_3$ | $CH_3$ | |
| 1.228 | $2-NO_2$ | 5-Cl | H | $C_2H_5$ | $CF_3$ | $CH_3$ | |
| 1.229 | $2-NO_2$ | 5-Cl | H | $CH_3$ | $CF_2Cl$ | $CH_3$ | |

Tabelle 1 (Fortsetzung)

| Verb.No | $R^1$ | $R^2$ | $R^3$ | $R^4$ | $R^5$ | $R^6$ | Phys. Daten |
|---|---|---|---|---|---|---|---|
| 1.230 | 2-$NO_2$ | 5-Cl | H | $SCH_3$ | $CH_3$ | $CH_3$ | |
| 1.231 | 2-$NO_2$ | 5-Cl | H | $SOCH_3$ | $CH_3$ | $CH_3$ | |
| 1.232 | 2-$NO_2$ | 5-Cl | H | $SO_2CH_3$ | $CH_3$ | $CH_3$ | |
| 1.233 | 2-$NO_2$ | 5-Cl | H | CN | $CH_3$ | $CH_3$ | |
| 1.234 | 2-$NO_2$ | 5-Cl | H | $OCH_3$ | $CF_3$ | $CH_3$ | |
| 1.235 | 2-$NO_2$ | 5-Cl | H | $SCH_3$ | $CF_3$ | $CH_3$ | |
| 1.236 | 2-$NO_2$ | 5-Cl | H | $OC_3H_7$(i) | $CF_3$ | $CH_3$ | |
| 1.237 | 2-$NO_2$ | 5-Cl | H | $SC_2H_5$ | $CF_3$ | $CH_3$ | |
| 1.238 | 2-$NO_2$ | 5-Cl | H | $C_3H_7$(i) | $CF_3$ | $CH_3$ | |
| 1.239 | 2-$NO_2$ | 6-Cl | H | $CH_3$ | Cl | $CH_3$ | |
| 1.240 | 2-$NO_2$ | 6-Cl | H | $CH_3$ | $CF_3$ | $CH_3$ | |
| 1.241 | 2-$NO_2$ | 6-Cl | H | $OCH_3$ | $CF_3$ | $CH_3$ | |
| 1.242 | 2-$NO_2$ | 6-Cl | H | $SCH_3$ | $CF_3$ | $CH_3$ | |
| 1.243 | 2-$NO_2$ | 6-Cl | H | $SOCH_3$ | $CF_3$ | $CH_3$ | |
| 1.244 | 2-$NO_2$ | 6-Cl | H | $SO_2CH_3$ | $CF_3$ | $CH_3$ | |
| 1.245 | 2-$NO_2$ | 6-Cl | H | $C_2H_5$ | $CF_3$ | $CH_3$ | |
| 1.246 | 2-$NO_2$ | 6-Cl | H | Cyclopropyl | $CF_3$ | $CH_3$ | |
| 1.247 | 2-$NO_2$ | 6-Cl | H | $C_3H_7$(i) | $CF_3$ | $CH_3$ | |
| 1.248 | 2-$NO_2$ | 6-Cl | H | $CH_3$ | $CF_2Cl$ | $CH_3$ | |
| 1.249 | 2-$NO_2$ | 5-$CH_3$ | H | $CH_3$ | Cl | $CH_3$ | |
| 1.250 | 2-$NO_2$ | 5-$CH_3$ | H | $CH_3$ | $CF_3$ | $CH_3$ | |
| 1.251 | 2-$NO_2$ | 5-$CH_3$ | H | $OCH_3$ | $CF_3$ | $CH_3$ | |
| 1.252 | 2-$NO_2$ | 5-$CH_3$ | H | $CF_3$ | Cl | $CH_3$ | |
| 1.253 | 2-$NO_2$ | 5-$CH_3$ | H | $SCH_3$ | $CF_3$ | $CH_3$ | |
| 1.254 | 2-$NO_2$ | 5-$CH_3$ | H | $C_2H_5$ | $CF_3$ | $CH_3$ | |
| 1.255 | 2-$NO_2$ | 5-$CH_3$ | H | $C_3H_7$(n) | $CF_3$ | $CH_3$ | |
| 1.256 | 2-$NO_2$ | 5-$CH_3$ | H | $C_3H_7$(i) | $CF_3$ | $CH_3$ | |
| 1.257 | 2-$NO_2$ | 5-$CH_3$ | H | Cyclopropyl | $CF_3$ | $CH_3$ | |
| 1.258 | 2-$NO_2$ | 5-$CH_3$ | H | $CH_3$ | $CF_2Cl$ | $CH_3$ | |
| 1.259 | 2-$NO_2$ | 5-$CH_3$ | H | $CH_3$ | CHFCl | $CH_3$ | |
| 1.260 | 2-$NO_2$ | 5-$CH_3$ | H | $OCH_3$ | $CH_3$ | $NO_2$ | |
| 1.261 | 2-$NO_2$ | 5-$CH_3$ | H | $OCH_3$ | $CH_3$ | CN | |
| 1.262 | 2-$NO_2$ | 5-$CH_3$ | H | $OCH_3$ | $CH_3$ | Cl | |
| 1.263 | 2-$NO_2$ | 5-$CH_3$ | H | $OCH_3$ | $CH_3$ | $COOCH_3$ | |
| 1.264 | 2-$NO_2$ | 5-$CH_3$ | H | $OC_3H_7$(n) | $CF_3$ | $CH_3$ | |
| 1.265 | 2-$NO_2$ | 5-$CH_3$ | H | $SC_2H_5$ | $CF_3$ | $CH_3$ | |
| 1.266 | 2-$NO_2$ | 6-$CH_3$ | H | $CH_3$ | Cl | $CH_3$ | |
| 1.267 | 2-$NO_2$ | 6-$CH_3$ | H | $OCH_3$ | $CH_3$ | $CH_3$ | |
| 1.268 | 2-$NO_2$ | 6-$CH_3$ | H | $SCH_3$ | $CH_3$ | $CH_3$ | |
| 1.269 | 2-$NO_2$ | 6-$CH_3$ | H | $SOCH_3$ | $CH_3$ | $CH_3$ | |
| 1.270 | 2-$NO_2$ | 6-$CH_3$ | H | $SO_2CH_3$ | $CH_3$ | $CH_3$ | |
| 1.271 | 2-$NO_2$ | 6-$CH_3$ | H | $CF_3$ | Cl | $CH_3$ | |
| 1.272 | 2-$NO_2$ | 6-$CH_3$ | H | $OCH_3$ | $CF_3$ | $CH_3$ | |
| 1.273 | 2-$NO_2$ | 6-$CH_3$ | H | $OC_2H_5$ | $CF_3$ | $CH_3$ | |
| 1.274 | 2-$NO_2$ | 6-$CH_3$ | H | $SCH_3$ | $CF_3$ | $CH_3$ | |
| 1.275 | 2-$NO_2$ | 6-$CH_3$ | H | $SOCH_3$ | $CF_3$ | $CH_3$ | |
| 1.276 | 2-$NO_2$ | 6-$CH_3$ | H | $SO_2CH_3$ | $CF_3$ | $CH_3$ | |

Tabelle 1 (Fortsetzung)

| Verb.No | $R^1$ | $R^2$ | $R^3$ | $R^4$ | $R^5$ | $R^6$ | Phys. Daten |
|---|---|---|---|---|---|---|---|
| 1.277 | 2-$NO_2$ | 6-$CH_3$ | H | $SC_3H_7$(i) | $CF_3$ | $CH_3$ | |
| 1.278 | 2-$NO_2$ | 6-$CH_3$ | H | $CF_3$ | Cl | $C_2H_5$ | |
| 1.279 | 2-$NO_2$ | 6-$CH_3$ | H | $OCH_3$ | $CF_3$ | $C_2H_5$ | |
| 1.280 | 2-$NO_2$ | 6-$CH_3$ | H | $SCH_3$ | $CF_3$ | $C_2H_5$ | |
| 1.281 | 2-$NO_2$ | 6-$CH_3$ | H | $CF_3$ | Cl | $C_3H_7$(i) | |
| 1.282 | 2-$NO_2$ | 6-$CH_3$ | H | $OCH_3$ | $CF_3$ | $C_3H_7$(i) | |
| 1.283 | 2-$NO_2$ | 6-$CH_3$ | H | $SC_2H_5$ | $CF_3$ | $C_3H_7$(i) | |
| 1.284 | 2-$NO_2$ | 6-$CH_3$ | H | $CH_3$ | $CF_3$ | $CH_3$ | Fp. 165-166°C |
| 1.285 | 2-$NO_2$ | 6-$CH_3$ | H | $C_2H_5$ | $CF_3$ | $CH_3$ | Fp. 158-159°C |
| 1.286 | 2-$NO_2$ | 6-$CH_3$ | H | $C_3H_7$(n) | $CF_3$ | $CH_3$ | |
| 1.287 | 2-$NO_2$ | 6-$CH_3$ | H | $C_3H_7$(i) | $CF_3$ | $CH_3$ | |
| 1.288 | 2-$NO_2$ | 6-$CH_3$ | H | Cyclopropyl | $CF_3$ | $CH_3$ | |
| 1.289 | 2-$NO_2$ | 6-$CH_3$ | H | $C_4H_9$(n) | $CF_3$ | $CH_3$ | |
| 1.290 | 2-$NO_2$ | 6-$CH_3$ | H | $CH_3$ | $CF_2Cl$ | $CH_3$ | |
| 1.291 | 2-$NO_2$ | 6-$CH_3$ | H | $CH_3$ | $CHF_2$ | $CH_3$ | |
| 1.292 | 2-$NO_2$ | 6-$CH_3$ | H | $CH_3$ | $CHCl_2$ | $CH_3$ | |
| 1.293 | 2-$NO_2$ | 6-$CH_3$ | H | $CH_3$ | $CHFCl$ | $CH_3$ | |
| 1.294 | 2-$NO_2$ | 6-$CH_3$ | H | $CH_3$ | $C_2F_5$ | $CH_3$ | |
| 1.295 | 2-$NO_2$ | 6-$CH_3$ | H | $CH_3$ | $C_3F_7$(n) | $CH_3$ | |
| 1.296 | 2-$NO_2$ | 6-$CH_3$ | H | $C_2H_5$ | $CF_2Cl$ | $CH_3$ | |
| 1.297 | 2-$NO_2$ | 6-$CH_3$ | H | $C_3H_7$(i) | $CF_2Cl$ | $CH_3$ | |
| 1.298 | 2-$NO_2$ | 6-$CH_3$ | H | Cyclopropyl | $CF_2Cl$ | $CH_3$ | |
| 1.299 | 2-$NO_2$ | 6-$CH_3$ | H | $C_2H_5$ | $C_2F_5$ | $CH_3$ | |
| 1.300 | 2-$NO_2$ | 6-$CH_3$ | H | $C_2H_5$ | $CHCl_2$ | $CH_3$ | |
| 1.301 | 2-$NO_2$ | 6-$CH_3$ | H | $CH_3$ | $CCl_2CF_3$ | $CH_3$ | |
| 1.302 | 2-$NO_2$ | 6-$CH_3$ | H | $CH_3$ | $OCHF_2$ | $CH_3$ | |
| 1.303 | 2-$NO_2$ | 6-$CH_3$ | H | $CH_3$ | Cl | $CH_2CH_2Cl$ | |
| 1.304 | 2-$NO_2$ | 6-$CH_3$ | H | $OCH_3$ | $CH_3$ | $CH_2CH_2OCH_3$ | |
| 1.305 | 2-$NO_2$ | 6-$CH_3$ | H | $SCH_3$ | $CH_3$ | $CH_2CH_2SCH_3$ | |
| 1.306 | 2-$NO_2$ | 6-$CH_3$ | H | CN | $CH_3$ | $CH_3$ | |
| 1.307 | 2-$NO_2$ | 6-$CH_3$ | H | $CH_3$ | $CF_3$ | Phenyl | |
| 1.308 | 2-$NO_2$ | 6-$CH_3$ | H | $CH_3$ | Cl | Phenyl | |
| 1.309 | 2-$NO_2$ | 6-$CH_3$ | H | $OCH_3$ | $CH_3$ | $NO_2$ | |
| 1.310 | 2-$NO_2$ | 6-$CH_3$ | H | $OCH_3$ | $CH_3$ | Cl | |
| 1.311 | 2-$NO_2$ | 6-$CH_3$ | H | $OCH_3$ | $CH_3$ | CN | |
| 1.312 | 2-$NO_2$ | 6-$CH_3$ | H | $CH_3$ | Cl | CN | |
| 1.313 | 2-$NO_2$ | 6-$CH_3$ | H | $CH_3$ | Cl | $NO_2$ | |
| 1.314 | 2-$NO_2$ | 6-$CH_3$ | H | $CH_3$ | Cl | Cl | |
| 1.315 | 2-$NO_2$ | 6-$CH_3$ | H | $SCH_3$ | $CH_3$ | Cl | |
| 1.316 | 2-$NO_2$ | 6-$CH_3$ | H | $CH_3$ | Cl | $OCH_3$ | |
| 1.317 | 2-$NO_2$ | 6-$CH_3$ | H | $OCH_3$ | $CH_3$ | $COOC_2H_5$ | |
| 1.318 | 2-$NO_2$ | 6-$CH_3$ | H | $CH_3$ | $CH_3$ | $COOCH_3$ | |
| 1.319 | 2-$NO_2$ | 5-F | H | $CH_3$ | Cl | $CH_3$ | |
| 1.320 | 2-$NO_2$ | 5-F | H | $CH_3$ | $CF_3$ | $CH_3$ | |
| 1.321 | 2-$NO_2$ | 5-F | H | $C_2H_5$ | $CF_3$ | $CH_3$ | |
| 1.322 | 2-$NO_2$ | 6-F | H | $CH_3$ | Cl | $CH_3$ | |
| 1.323 | 2-$NO_2$ | 6-F | H | $OCH_3$ | $CH_3$ | $CH_3$ | |

Tabelle 1 (Fortsetzung)

| Verb.No | $R^1$ | $R^2$ | $R^3$ | $R^4$ | $R^5$ | $R^6$ | Phys. Daten |
|---|---|---|---|---|---|---|---|
| 1.324 | 2-$NO_2$ | 6-F | H | $CH_3$ | $CF_3$ | $CH_3$ | |
| 1.325 | 2-$NO_2$ | 6-F | H | $CH_3$ | $CF_2Cl$ | $CH_3$ | |
| 1.326 | 2-$NO_2$ | 6-Br | H | $CH_3$ | Cl | $CH_3$ | |
| 1.327 | 2-$NO_2$ | 6-Br | H | $CH_3$ | $CF_3$ | $CH_3$ | |
| 1.328 | 2-$NO_2$ | 6-Br | H | $C_2H_5$ | $CF_3$ | $CH_3$ | |
| 1.329 | 2-$NO_2$ | 6-Br | H | Cyclopropyl | $CF_3$ | $CH_3$ | |
| 1.330 | 2-$NO_2$ | 6-Br | H | $CH_3$ | CHFCl | $CH_3$ | |
| 1.331 | 2-$NO_2$ | 6-Br | H | $CH_3$ | $CF_2Cl$ | $CH_3$ | |
| 1.332 | 2-$NO_2$ | 6-Br | H | $CH_3$ | $CHCl_2$ | $CH_3$ | |
| 1.333 | 2-F | 5-F | H | $CH_3$ | Cl | $CH_3$ | |
| 1.334 | 2-F | 5-F | H | $CH_3$ | $CF_3$ | $CH_3$ | |
| 1.335 | 2-F | 5-F | H | $C_2H_5$ | $CF_3$ | $CH_3$ | |
| 1.336 | 2-F | 5-F | H | $C_2H_5$ | $C_2F_5$ | $CH_3$ | |
| 1.337 | 2-F | 5-F | H | $C_2H_5$ | $CF_2Cl$ | $CH_3$ | |
| 1.338 | 2-F | 6-F | H | $CH_3$ | Cl | $CH_3$ | |
| 1.339 | 2-F | 6-F | H | $CH_3$ | $CF_3$ | $CH_3$ | |
| 1.340 | 2-F | 6-F | H | $C_2H_5$ | $CF_3$ | $CH_3$ | Fp. 144-145°C |
| 1.341 | 2-F | 6-F | H | $OCH_3$ | $CF_3$ | $CH_3$ | |
| 1.342 | 2-F | 6-F | H | $SCH_3$ | $CF_3$ | $CH_3$ | |
| 1.343 | 2-F | 6-Cl | H | $CH_3$ | Cl | $CH_3$ | |
| 1.344 | 2-F | 6-Cl | H | $CH_3$ | $CF_3$ | $CH_3$ | |
| 1.345 | 2-F | 6-Cl | H | $CH_3$ | $C_2F_5$ | $CH_3$ | |
| 1.346 | 2-F | 6-Cl | H | $C_2H_5$ | $CF_3$ | $CH_3$ | |
| 1.347 | 2-F | 6-Cl | H | $C_2H_5$ | $CF_2Cl$ | $CH_3$ | |
| 1.348 | 2-$CF_3$ | 6-Cl | H | $CH_3$ | Cl | $CH_3$ | |
| 1.349 | 2-$CF_3$ | 6-Cl | H | $CH_3$ | $CF_3$ | $CH_3$ | |
| 1.350 | 2-$CF_3$ | 6-Cl | H | $C_2H_5$ | $CF_3$ | $CH_3$ | |
| 1.351 | 2-$CF_3$ | 6-Cl | H | $C_2H_5$ | $CF_2Cl$ | $CH_3$ | |
| 1.352 | 2-$CF_3$ | 6-Cl | H | $CH_3$ | $C_2F_5$ | $CH_3$ | |
| 1.353 | 2-$CF_3$ | 6-$CH_3$ | H | $CH_3$ | Cl | $CH_3$ | |
| 1.354 | 2-$CF_3$ | 6-$CH_3$ | H | $CH_3$ | $CF_3$ | $CH_3$ | |
| 1.355 | 2-$CF_3$ | 6-$CH_3$ | H | $C_2H_5$ | $CF_3$ | $CH_3$ | |
| 1.356 | 2-$CF_3$ | 6-$CH_3$ | H | Cyclopropyl | $CF_3$ | $CH_3$ | |
| 1.357 | 2-$OCHF_2$ | 5-Cl | H | $CH_3$ | Cl | $CH_3$ | |
| 1.358 | 2-$OCHF_2$ | 5-Cl | H | $CH_3$ | $CF_3$ | $CH_3$ | |
| 1.359 | 2-$OCHF_2$ | 5-Cl | H | $C_2H_5$ | $CF_3$ | $CH_3$ | |
| 1.360 | 2-$OCHF_2$ | 5-Cl | H | $CH_3$ | $CF_2Cl$ | $CH_3$ | |
| 1.361 | 2-$OCHF_2$ | 5-Cl | H | $CH_3$ | $CHCl_2$ | $CH_3$ | |
| 1.362 | 2-$OCHF_2$ | 5-Cl | H | $CF_3$ | Cl | $CH_3$ | |
| 1.363 | 2-$OCHF_2$ | 5-Cl | H | $OCH_3$ | $CF_3$ | $CH_3$ | |
| 1.364 | 2-$OCHF_2$ | 5-Cl | H | $SC_2H_5$ | $CF_3$ | $CH_3$ | |
| 1.365 | 2-$OCHF_2$ | 5-Cl | H | $CF_3$ | Cl | $C_2H_5$ | |
| 1.366 | 2-$OCHF_2$ | 5-Cl | H | $OCH_3$ | $CF_3$ | $C_2H_5$ | |
| 1.367 | 2-$OCHF_2$ | 5-Cl | H | $OC_2H_5$ | $CF_3$ | $C_2H_5$ | |
| 1.368 | 2-$OCHF_2$ | 5-Cl | H | $SCH_3$ | $CF_3$ | $C_2H_5$ | |
| 1.369 | 2-$OCF_3$ | 5-Cl | H | $CH_3$ | Cl | $CH_3$ | |
| 1.370 | 2-$OCF_3$ | 5-Cl | H | $CH_3$ | $CF_3$ | $CH_3$ | |

Tabelle 1 (Fortsetzung)

| Verb.No | $R^1$ | $R^2$ | $R^3$ | $R^4$ | $R^5$ | $R^6$ | Phys. Daten |
|---|---|---|---|---|---|---|---|
| 1.371 | 2-$OCF_3$ | 5-Cl | H | $C_2H_5$ | $CF_3$ | $CH_3$ | |
| 1.372 | 2-$OCF_3$ | 5-Cl | H | $CH_3$ | $C_2H_5$ | $CH_3$ | |
| 1.373 | 2-$OCF_3$ | 5-Cl | H | $CH_3$ | $CF_2Cl$ | $CH_3$ | |
| 1.374 | 2-$C_2H_5$ | 6-$CH_3$ | H | $CH_3$ | Cl | $CH_3$ | |
| 1.375 | 2-$C_2H_5$ | 6-$CH_3$ | H | $CH_3$ | $CF_3$ | $CH_3$ | |
| 1.376 | 2-$C_2H_5$ | 6-$CH_3$ | H | $OCH_3$ | $CF_3$ | $CH_3$ | |
| 1.377 | 2-$C_2H_5$ | 6-$CH_3$ | H | $SCH_3$ | $CF_3$ | $CH_3$ | |
| 1.378 | 2-$C_2H_5$ | 6-$CH_3$ | H | $OC_3H_7$(i) | $CF_3$ | $CH_3$ | |
| 1.379 | 2-$CF_3$ | 6-$NO_2$ | H | $CH_3$ | Cl | $CH_3$ | |
| 1.380 | 2-$CF_3$ | 6-$NO_2$ | H | $CH_3$ | $CF_3$ | $CH_3$ | |
| 1.381 | 2-$NO_2$ | 6-$NO_2$ | H | $CH_3$ | $CF_3$ | $CH_3$ | |
| 1.382 | 2-$NO_2$ | 6-$NO_2$ | H | $OCH_3$ | $CF_3$ | $CH_3$ | |
| 1.383 | 2-Cl | 6-Cl | 3-$CH_3$ | $CH_3$ | Cl | $CH_3$ | |
| 1.384 | 2-Cl | 6-Cl | 3-$CH_3$ | $CH_3$ | $CF_3$ | $CH_3$ | |
| 1.385 | 2-Cl | 6-Cl | 3-$CH_3$ | $CH_3$ | $CF_3$ | $C_2H_5$ | |
| 1.386 | 2-Cl | 6-Cl | 3-Cl | $CH_3$ | Cl | $CH_3$ | |
| 1.387 | 2-Cl | 6-Cl | 3-Cl | $CH_3$ | $CF_3$ | $CH_3$ | |
| 1.388 | 2-Cl | 6-Cl | 3-Cl | $C_2H_5$ | $CF_3$ | $CH_3$ | |
| 1.389 | 2-$CH_3$ | 3-Cl | H | $CH_3$ | Cl | $CH_3$ | |
| 1.390 | 2-$CH_3$ | 3-Cl | H | $CH_3$ | $CF_3$ | $CH_3$ | |
| 1.391 | 2-$CH_3$ | 3-Cl | H | $C_2H_5$ | $CF_3$ | $CH_3$ | |
| 1.392 | 2-$CH_3$ | 5-Cl | H | $CH_3$ | Cl | $CH_3$ | |
| 1.393 | 2-$CH_3$ | 5-Cl | H | $CH_3$ | $CF_3$ | $CH_3$ | |
| 1.394 | 2-$CH_3$ | 5-Cl | H | $C_2H_5$ | $CF_3$ | $CH_3$ | |
| 1.395 | 2-$CH_3$ | 5-Cl | H | $C_2H_5$ | $CF_2Cl$ | $CH_3$ | |
| 1.396 | 2-$CH_3$ | 5-Cl | H | $OCH_3$ | $CF_3$ | $CH_3$ | |
| 1.397 | 2-$CH_3$ | 6-Cl | H | $CH_3$ | Cl | $CH_3$ | |
| 1.398 | 2-$CH_3$ | 6-Cl | H | $CH_3$ | $CF_3$ | $CH_3$ | |
| 1.399 | 2-$CH_3$ | 6-Cl | H | $C_2H_5$ | $CF_3$ | $CH_3$ | |
| 1.400 | 2-$CH_3$ | 6-Cl | H | $C_2H_5$ | $CHCl_2$ | $CH_3$ | |
| 1.401 | 2-$CH_3$ | 6-Cl | H | $C_2H_5$ | $CF_2Cl$ | $CH_3$ | |
| 1.402 | 2-$CH_3$ | 6-Cl | H | $OC_2H_5$ | $CF_3$ | $CH_3$ | |
| 1.403 | 2-$CH_3$ | 6-Cl | H | $SCH_3$ | $CF_3$ | $CH_3$ | |
| 1.404 | 2-$CH_3$ | 6-Cl | H | $OC_4H_9$(n) | $CF_3$ | $CH_3$ | |
| 1.405 | 2-$CH_3$ | 6-Cl | H | $CH_3$ | Cl | $SCH_3$ | |
| 1.406 | 2-$CH_3$ | 6-$CH_3$ | H | $CH_3$ | Cl | $CH_3$ | |
| 1.407 | 2-$CH_3$ | 6-$CH_3$ | H | $CH_3$ | $CF_3$ | $CH_3$ | |
| 1.408 | 2-$CH_3$ | 6-$CH_3$ | H | $C_2H_5$ | $CF_3$ | $CH_3$ | |
| 1.409 | 2-$CH_3$ | 6-$CH_3$ | H | Cyclopropyl | $CF_3$ | $CH_3$ | |
| 1.410 | 2-$CH_3$ | 6-$CH_3$ | H | $C_2H_5$ | $CF_2Cl$ | $CH_3$ | |
| 1.411 | 2-Cl | 3-$CH_3$ | H | $CH_3$ | Cl | $CH_3$ | |
| 1.412 | 2-Cl | 3-$CH_3$ | H | $CH_3$ | $CF_3$ | $CH_3$ | |
| 1.413 | 2-Cl | 3-$CH_3$ | H | $C_2H_5$ | $CF_3$ | $CH_3$ | |
| 1.414 | 2-Cl | 5-$CH_3$ | H | $CH_3$ | Cl | $CH_3$ | |
| 1.415 | 2-Cl | 5-$CH_3$ | H | $CH_3$ | $CF_3$ | $CH_3$ | |
| 1.416 | 2-Cl | 5-$CH_3$ | H | $C_2H_5$ | $CF_3$ | $CH_3$ | |
| 1.417 | 2-Cl | 5-$CH_3$ | H | $C_2H_5$ | $CF_2Cl$ | $CH_3$ | |

Tabelle 1 (Fortsetzung)

| Verb.No | $R^1$ | $R^2$ | $R^3$ | $R^4$ | $R^5$ | $R^6$ | Phys. Daten |
|---|---|---|---|---|---|---|---|
| 1.418 | $2\text{-}NO_2$ | H | H | $CH_2Cl$ | Cl | $CH_3$ | |
| 1.419 | $2\text{-}NO_2$ | H | H | $CH_2OCH_3$ | Cl | $CH_3$ | |
| 1.420 | $2\text{-}NO_2$ | H | H | $CH_3$ | Cl | $SCH_3$ | |
| 1.421 | $2\text{-}NO_2$ | H | H | $CH_3$ | Cl | $SC_2H_5$ | |
| 1.422 | $2\text{-}NO_2$ | H | H | $CH_3$ | Cl | $OCH_3$ | |
| 1.423 | $2\text{-}NO_2$ | H | H | $CH_3$ | Cl | $OC_2H_5$ | |
| 1.424 | $2\text{-}NO_2$ | $6\text{-}CH_3$ | H | $CH_2OCH_3$ | Cl | $CH_3$ | |
| 1.425 | $2\text{-}NO_2$ | $6\text{-}CH_3$ | H | $CH_3$ | Cl | $SCH_3$ | |
| 1.426 | $2\text{-}NO_2$ | $6\text{-}CH_3$ | H | $CH_3$ | Cl | $OC_2H_5$ | |
| 1.427 | $2\text{-}NO_2$ | $6\text{-}CH_3$ | H | $CH_3$ | Cl | $SC_2H_5$ | |
| 1.428 | $2\text{-}NO_2$ | $6\text{-}CH_3$ | H | $CH_3$ | Cl | $SC_3H_7(i)$ | |
| 1.429 | 2-Cl | 5-Cl | H | $CH_3$ | Cl | $OCH_3$ | |
| 1.430 | 2-Cl | 5-Cl | H | $CH_3$ | Cl | $SCH_3$ | |
| 1.431 | 2-Cl | 5-Cl | H | $CH_3$ | Cl | $OC_2H_5$ | |
| 1.432 | 2-Cl | 6-Cl | H | $CH_2OCH_3$ | $CF_3$ | $CH_3$ | |
| 1.433 | 2-Cl | 6-Cl | H | $CH_2OCH_3$ | Cl | $CH_3$ | |
| 1.434 | 2-Cl | 6-Cl | H | $CH_3$ | Cl | $OCH_3$ | |
| 1.435 | 2-Cl | 6-Cl | H | $CH_3$ | Cl | $SCH_3$ | |
| 1.436 | 2-Cl | 6-Cl | H | $CH_3$ | Cl | $OC_2H_5$ | |
| 1.437 | 2-Cl | 6-Cl | H | $CH_3$ | Cl | $SC_2H_5$ | |
| 1.438 | $2\text{-}NO_2$ | H | H | $CH_2COOCH_3$ | $CF_3$ | $CH_3$ | |
| 1.439 | $2\text{-}NO_2$ | H | H | $CH_2\overset{O}{\overset{\|}{C}}CH_3$ | $CF_3$ | $CH_3$ | |
| 1.440 | $2\text{-}OCH_3$ | 5-Cl | H | $CH_3$ | Cl | $CH_3$ | |
| 1.441 | $2\text{-}OCH_3$ | 5-Cl | H | $CH_3$ | $CF_3$ | $CH_3$ | |
| 1.442 | $2\text{-}OCH_3$ | 5-Cl | H | $C_2H_5$ | $CF_3$ | $CH_3$ | |
| 1.443 | $2\text{-}OCH_3$ | 5-Cl | H | $CH_3$ | $CF_2Cl$ | $CH_3$ | |
| 1.444 | $2\text{-}OCH_3$ | 5-Cl | H | $CH_3$ | $C_2F_5$ | $CH_3$ | |
| 1.445 | $2\text{-}OCH_3$ | 5-Cl | H | Cyclopropyl | $CF_3$ | $CH_3$ | |

| Verb.No | $R^1$ | $R^2$ | $R^3$ | $-R^5 - R^6 -$ | $R^4$ | Phys. Daten |
|---|---|---|---|---|---|---|
| 1.446 | $2\text{-}NO_2$ | H | H | $-CH_2\text{-}CH_2-$ | $CF_3$ | |
| 1.447 | $2\text{-}NO_2$ | H | H | $-CHCH_3\text{-}CH_2-$ | $CF_3$ | |
| 1.448 | $2\text{-}NO_2$ | H | H | $-CH_2\text{-}CH_2-$ | $CF_2Cl$ | |
| 1.449 | $2\text{-}NO_2$ | H | H | $-(CH_2)_3-$ | $CF_3$ | Fp.160-161°C |
| 1.450 | $2\text{-}NO_2$ | H | H | $-(CH_2)_3-$ | Cl | |
| 1.451 | $2\text{-}NO_2$ | H | H | $-(CH_2)_3-$ | $OCH_3$ | |
| 1.452 | $2\text{-}NO_2$ | H | H | $-(CH_2)_3-$ | $SCH_3$ | |
| 1.453 | $2\text{-}NO_2$ | H | H | $-(CH_2)_3-$ | $SOCH_3$ | |
| 1.454 | $2\text{-}NO_2$ | H | H | $-(CH_2)_3-$ | $SO_2CH_3$ | |
| 1.455 | $2\text{-}NO_2$ | H | H | $-CHCH_3\text{-}CH_2\text{-}CH_2-$ | $CF_3$ | |
| 1.456 | $2\text{-}NO_2$ | H | H | $-CHCH_3\text{-}CH_2\text{-}CH_2-$ | Cl | |
| 1.457 | $2\text{-}NO_2$ | H | H | $-CHCH_3\text{-}CH_2\text{-}CH_2-$ | $CHCl_2$ | |
| 1.458 | $2\text{-}NO_2$ | H | H | $-CHCH_3\text{-}CH_2\text{-}CH_2-$ | CHFCl | |
| 1.459 | $2\text{-}NO_2$ | H | H | $-CHCH_3\text{-}CH_2\text{-}CH_2-$ | $CF_2Cl$ | |
| 1.460 | $2\text{-}NO_2$ | H | H | $-C(CH_3)_2\text{-}CH_2\text{-}CH_2-$ | $CF_3$ | |
| 1.461 | $2\text{-}NO_2$ | H | H | $-C(CH_3)_2\text{-}CH_2CH_2$ | Cl | |

Tabelle 1 (Fortsetzung)

| Verb.No | $R^1$ | $R^2$ | $R^3$ | $-R^5-R^6-$ | $R^4$ | Phys. Daten |
|---|---|---|---|---|---|---|
| 1.462 | $2-NO_2$ | H | H | $-C(CH_3)_2-CH_2-CH_2$ | $CF_2CF_3$ | |
| 1.463 | $2-NO_2$ | H | H | $-CH{=}CCH_3-CH_2-$ | $CF_3$ | |
| 1.464 | $2-NO_2$ | H | H | $-CH{=}CCH_3-CH_2-$ | Cl | |
| 1.465 | $2-NO_2$ | H | H | $-CH{=}CCH_3-CH_2-$ | $CHF_2$ | |
| 1.466 | $2-NO_2$ | H | H | $-CH_2-CHCH_3-CH_2-$ | Cl | |
| 1.467 | $2-NO_2$ | H | H | $-CH_2-CHCH_3-CH_2-$ | $CF_3$ | |
| 1.468 | $2-NO_2$ | H | H | $-CH_2-CH_2-CHCH_3-$ | Cl | |
| 1.469 | $2-NO_2$ | H | H | $-CH_2-CH_2-CHCH_3-$ | $CF_3$ | |
| 1.470 | $2-NO_2$ | H | H | $-CH(COOCH_3)-CH_2-CH_2-$ | Cl | |
| 1.471 | $2-NO_2$ | H | H | $-CH(COOC_2H_5)-CH_2-CH_2-$ | Cl | |
| 1.472 | $2-NO_2$ | H | H | $-CH_2-CH_2-S-$ | Cl | |
| 1.473 | $2-NO_2$ | H | H | $-CH_2-CH_2-S-$ | $CF_3$ | |
| 1.474 | $2-NO_2$ | H | H | $-CH_2-CH_2-S-$ | $CF_2Cl$ | |
| 1.475 | $2-NO_2$ | H | H | $-CH_2-CH_2-S-$ | $CHCl_2$ | |
| 1.476 | $2-NO_2$ | H | H | $-CH_2-C(CH_3)_2-CH_2-$ | Cl | |
| 1.477 | $2-NO_2$ | H | H | $-CH_2-C(CH_3)_2-CH_2-$ | $CF_3$ | |
| 1.478 | $2-NO_2$ | H | H | $-CH_2-C(CH_3)_2-CH_2-$ | $CF_2CF_3$ | |
| 1.479 | $2-NO_2$ | H | H | $-CH_2-CH_2-C(CH_3)_2-$ | Cl | |
| 1.480 | $2-NO_2$ | H | H | $-CH_2-CH_2-C(CH_3)_2-$ | $CF_3$ | |
| 1.481 | $2-NO_2$ | H | H | $-CH_2-CH_2-C(CH_3)_2-$ | $CF_2CF_3$ | |
| 1.482 | $2-NO_2$ | H | H | $-CHCH_3-CHCH_3-CH_2-$ | Cl | |
| 1.483 | $2-NO_2$ | H | H | $-CHCH_3-CHCH_3-CH_2-$ | $CF_3$ | |
| 1.484 | $2-NO_2$ | H | H | $-CHCH_3-CHCH_3-CH_2-$ | $CF_2Cl$ | |
| 1.485 | $2-NO_2$ | H | H | $-CH(C_2H_5)-CH_2-CH_2-$ | Cl | |
| 1.486 | $2-NO_2$ | H | H | $-CH(C_2H_5)-CH_2-CH_2-$ | $CF_3$ | |
| 1.487 | $2-NO_2$ | H | H | $-C(CH_3)(C_2H_5)-CH_2-CH_2$ | Cl | |
| 1.488 | $2-NO_2$ | H | H | $-C(CH_3)(C_2H_5)-CH_2CH_2$ | $CF_3$ | |
| 1.489 | $2-NO_2$ | H | H | $-CHCH_3-(CH_2)_3-$ | Cl | |
| 1.490 | $2-NO_2$ | H | H | $-CHCH_3-(CH_2)_3-$ | $CF_3$ | Fp. 170-171°C |
| 1.491 | $2-NO_2$ | H | H | $-CHCH_3-(CH_2)_3-$ | $CF_2CF_3$ | |
| 1.492 | $2-NO_2$ | H | H | $-CHCH_3-(CH_2)_3-$ | $CF_2Cl$ | |
| 1.493 | $2-NO_2$ | H | H | $-CHCH_3-(CH_2)_3-$ | $CHF_2$ | |
| 1.494 | $2-NO_2$ | H | H | $-(CH_2)_4-$ | Cl | Fp. 168-170°C |
| 1.495 | $2-NO_2$ | H | H | $-(CH_2)_4-$ | $OCH_3$ | |
| 1.496 | $2-NO_2$ | H | H | $-(CH_2)_4-$ | $CF_3$ | Fp. 163-164°C |
| 1.497 | $2-NO_2$ | H | H | $-(CH_2)_4-$ | $CHF_2$ | Fp. 154-155°C |
| 1.498 | $2-NO_2$ | H | H | $-(CH_2)_4-$ | $CHCl_2$ | |
| 1.499 | $2-NO_2$ | H | H | $-(CH_2)_4-$ | $CHFCl$ | |
| 1.500 | $2-NO_2$ | H | H | $-(CH_2)_4-$ | $CF_2CF_3$ | |
| 1.501 | $2-NO_2$ | H | H | $-(CH_2)_4-$ | $C_3F_7(n)$ | |
| 1.502 | $2-NO_2$ | H | H | $-(CH_2)_4-$ | $CCl_2CF_3$ | |
| 1.503 | $2-NO_2$ | H | H | $-(CH_2)_4-$ | $SCH_3$ | |
| 1.504 | $2-NO_2$ | H | H | $-(CH_2)_4-$ | $SOCH_3$ | |
| 1.505 | $2-NO_2$ | H | H | $-(CH_2)_4-$ | $SO_2CH_3$ | |
| 1.506 | $2-NO_2$ | H | H | $-C(CH_3)_2-(CH_2)_3-$ | Cl | |
| 1.507 | $2-NO_2$ | H | H | $-C(CH_3)_2-(CH_2)_3-$ | $CF_3$ | |
| 1.508 | $2-NO_2$ | H | H | $-C(CH_3)_2-(CH_2)_3-$ | $CF_2Cl$ | |
| 1.509 | $2-NO_2$ | H | H | $-C(CH_3)_2-(CH_2)_3-$ | $CHF_2$ | |

Tabelle 1 (Fortsetzung)

| Verb.No | $R^1$ | $R^2$ | $R^3$ | $-R^5-R^6-$ | $R^4$ | Phys. Daten |
|---|---|---|---|---|---|---|
| 1.510 | $2-NO_2$ | H | H | $-C(CH_3)_2-(CH_2)_3-$ | $CF_2CF_3$ | |
| 1.511 | $2-NO_2$ | H | H | $-CH_2-CHCH_3-(CH_2)_2-$ | Cl | |
| 1.512 | $2-NO_2$ | H | H | $-CH_2-CHCH_3-(CH_2)_2-$ | $CF_3$ | Fp. 147-148°C |
| 1.513 | $2-NO_2$ | H | H | $-(CH_2)_3-CHCH_3-$ | Cl | |
| 1.514 | $2-NO_2$ | H | H | $-(CH_2)_3-CHCH_3-$ | $CF_3$ | |
| 1.515 | $2-NO_2$ | H | H | $-(CH_2)_2-CHCH_3-CH_2-$ | Cl | |
| 1.516 | $2-NO_2$ | H | H | $-(CH_2)_2-CHCH_3-CH_2-$ | $OCH_3$ | |
| 1.517 | $2-NO_2$ | H | H | $-(CH_2)_2-CHCH_3-CH_2-$ | $CF_3$ | Fp.184°C(Zers.) |
| 1.518 | $2-NO_2$ | H | H | $-(CH_2)_2-CHCH_3-CH_2-$ | $CF_2Cl$ | |
| 1.519 | $2-NO_2$ | H | H | $-(CH_2)_2-CHCH_3-CH_2-$ | $CHCl_2$ | |
| 1.520 | $2-NO_2$ | H | H | $-(CH_2)_2-C(CH_3)_2-CH_2-$ | Cl | |
| 1.521 | $2-NO_2$ | H | H | $-(CH_2)_2-C(CH_3)_2-CH_3-$ | $CF_3$ | |
| 1.522 | $2-NO_2$ | H | H | $-CH=CCH_3-CH_2-C(CH_3)_2-$ | Cl | |
| 1.523 | $2-NO_2$ | H | H | $-CH=CCH_3-CH_2-C(CH_3)_2-$ | $CF_3$ | |
| 1.524 | $2-NO_2$ | H | H | $-CH_2-CH(OCH_3)-(CH_2)_2$ | Cl | |
| 1.525 | $2-NO_2$ | H | H | $-CH_2-CH(OCH_3)-(CH_2)_2$ | $CF_3$ | |
| 1.526 | $2-NO_2$ | H | H | $-(CH_2)_2-S-CH_2-$ | Cl | |
| 1.527 | $2-NO_2$ | H | H | $-(CH_2)_2-S-CH_2-$ | $CF_3$ | |
| 1.528 | $2-NO_2$ | H | H | $-(CH_2)_2-O-CH_2-$ | Cl | |
| 1.529 | $2-NO_2$ | H | H | $-(CH_2)_2-NCH_3-CH_2-$ | $CF_3$ | |
| 1.530 | $2-NO_2$ | H | H | $-O-(CH_2)_2-$ | $CF_3$ | |
| 1.531 | $2-NO_2$ | H | H | $-O-(CH_2)_3-$ | $CF_3$ | |
| 1.532 | $2-NO_2$ | H | H | $-CH(COOC_2H_5)-(CH_2)_3$ | Cl | |
| 1.533 | $2-NO_2$ | H | H | $-CH(COOC_2H_5)-(CH_2)_3$ | $CF_3$ | |
| 1.534 | $2-NO_2$ | H | H | $-CHCH_3-CH_2-C(CH_3)_2-CH_2-$ | Cl | |
| 1.535 | $2-NO_2$ | H | H | $-CHCH_3-CH_2-C(CH_3)_2-CH_2-$ | $CF_3$ | |
| 1.536 | $2-NO_2$ | H | H | $-CHCH_3-CH_2-C(CH_3)_2-CH_2-$ | $CF_2CF_3$ | |
| 1.537 | $2-NO_2$ | H | H | $-(CH_2)_5-$ | Cl | |
| 1.538 | $2-NO_2$ | H | H | $-(CH_2)_5-$ | $CF_3$ | Fp. 156-158°C |
| 1.539 | $2-NO_2$ | H | H | $-CHCH_3-(CH_2)_4-$ | Cl | |
| 1.540 | $2-NO_2$ | H | H | $-CHCH_3-(CH_2)_4-$ | $CF_3$ | |
| 1.541 | $2-NO_2$ | H | H | $-C(CH_3)_2-(CH_2)_4-$ | Cl | |
| 1.542 | $2-NO_2$ | H | H | $-C(CH_3)_2-(CH_2)_4-$ | $CF_3$ | |
| 1.543 | $2-NO_2$ | H | H | $-(CH_2)_6-$ | Cl | |
| 1.544 | $2-NO_2$ | H | H | $-(CH_2)_6-$ | $CF_3$ | Fp. 154-156°C |
| 1.545 | $2-NO_2$ | $6-CH_3$ | H | $-(CH_2)_3-$ | $CF_3$ | Fp. 167-168°C |
| 1.546 | $2-NO_2$ | $6-CH_3$ | H | $-(CH_2)_3-$ | Cl | |
| 1.547 | $2-NO_2$ | $6-CH_3$ | H | $-(CH_2)_3-$ | $OCH_3$ | |
| 1.548 | $2-NO_2$ | $6-CH_3$ | H | $-(CH_2)_3-$ | $CF_2Cl$ | Fp. 177-178°C |
| 1.549 | $2-NO_2$ | $6-CH_3$ | H | $-(CH_2)_3-$ | $CF_2CF_3$ | |
| 1.550 | $2-NO_2$ | $6-CH_3$ | H | $-(CH_2)_3-$ | $CHCl_2$ | |
| 1.551 | $2-NO_2$ | $6-CH_3$ | H | $-CHCH_3-CH_2-CH_2-$ | Cl | |
| 1.552 | $2-NO_2$ | $6-CH_3$ | H | $-CHCH_3-CH_2-CH_2$ | $CF_3$ | |
| 1.553 | $2-NO_2$ | $6-CH_3$ | H | $-CHCH_3-CH_2-CH_2$ | $CF_2Cl$ | |
| 1.554 | $2-NO_2$ | $6-CH_3$ | H | $-C(CH_3)_2-CH_2-CH_2-$ | Cl | |
| 1.555 | $2-NO_2$ | $6-CH_3$ | H | $-C(CH_3)_2-CH_2-CH_2-$ | $CF_3$ | |
| 1.556 | $2-NO_2$ | $6-CH_3$ | H | $-C(CH_3)_2-CH_2-CH_2-$ | $CF_2CF_3$ | |
| 1.557 | $2-NO_2$ | $6-CH_3$ | H | $-C(CH_3)_2-CH_2-CH_2-$ | $CF_2Cl$ | |

Tabelle 1 (Fortsetzung)

| Verb.No | $R^1$ | $R^2$ | $R^3$ | $-R^5 - R^6 -$ | $R^4$ | Phys. Daten |
|---|---|---|---|---|---|---|
| 1.558 | $2-NO_2$ | $6-CH_3$ | H | $-C(CH_3)_2-CH_2-CH_2-$ | $CHCl_2$ | |
| 1.559 | $2-NO_2$ | $6-CH_3$ | H | $-CH_2-CH_2-S-$ | Cl | |
| 1.560 | $2-NO_2$ | $6-CH_3$ | H | $-CH_2-CH_2-S-$ | $CF_3$ | |
| 1.561 | $2-NO_2$ | $6-CH_3$ | H | $-(CH_2)_4-$ | Cl | |
| 1.562 | $2-NO_2$ | $6-CH_3$ | H | $-(CH_2)_4-$ | $OCH_3$ | |
| 1.563 | $2-NO_2$ | $6-CH_3$ | H | $-(CH_2)_4-$ | $CF_3$ | Fp. 161-162°C |
| 1.564 | $2-NO_2$ | $6-CH_3$ | H | $-(CH_2)_4-$ | $CF_2Cl$ | Fp. 175-177°C |
| 1.565 | $2-NO_2$ | $6-CH_3$ | H | $-(CH_2)_4-$ | $CHF_2$ | Fp. 148-149°C |
| 1.566 | $2-NO_2$ | $6-CH_3$ | H | $-(CH_2)_4-$ | $CHFCl$ | |
| 1.567 | $2-NO_2$ | $6-CH_3$ | H | $-(CH_2)_4-$ | $CHCl_2$ | |
| 1.568 | $2-NO_2$ | $6-CH_3$ | H | $-(CH_2)_4-$ | $CF_2CF_3$ | |
| 1.569 | $2-NO_2$ | $6-CH_3$ | H | $-(CH_2)_4-$ | $C_3F_7(n)$ | |
| 1.570 | $2-NO_2$ | $6-CH_3$ | H | $-(CH_2)_4-$ | $CCl_2CF_3$ | |
| 1.571 | $2-NO_2$ | $6-CH_3$ | H | $-(CH_2)_4-$ | $SCH_3$ | |
| 1.572 | $2-NO_2$ | $6-CH_3$ | H | $-(CH_2)_4$ | $SOCH_3$ | |
| 1.573 | $2-NO_2$ | $6-CH_3$ | H | $-(CH_2)_4$ | $SO_2CH_3$ | |
| 1.574 | $2-NO_2$ | $6-CH_3$ | H | $-C(CH_3)_2-(CH_2)_3-$ | Cl | |
| 1.575 | $2-NO_2$ | $6-CH_3$ | H | $-C(CH_3)_2-(CH_2)_3-$ | $CF_3$ | |
| 1.576 | $2-NO_2$ | $6-CH_3$ | H | $-CH_2-CHCH_3-(CH_2)_2-$ | Cl | |
| 1.577 | $2-NO_2$ | $6-CH_3$ | H | $-CH_2-CHCH_3-(CH_2)_2-$ | $CF_3$ | Fp. 156-157°C |
| 1.578 | $2-NO_2$ | $6-CH_3$ | H | $-CH_2-CHCH_3-(CH_2)_2-$ | $CF_2Cl$ | |
| 1.579 | $2-NO_2$ | $6-CH_3$ | H | $-CH_2-CHCH_3-(CH_2)_2-$ | $CHCl_2$ | |
| 1.580 | $2-NO_2$ | $6-CH_3$ | H | $-(CH_2)_2-NCH_3-CH_2-$ | $CF_3$ | |
| 1.581 | $2-NO_2$ | $6-CH_3$ | H | $-(CH_2)_5-$ | Cl | |
| 1.582 | $2-NO_2$ | $6-CH_3$ | H | $-(CH_2)_5-$ | $CF_3$ | Fp. 168-170°C |
| 1.583 | $2-NO_2$ | $6-CH_3$ | H | $-CH_2-CH_2-CH_2-O-$ | Cl | |
| 1.584 | $2-NO_2$ | $6-CH_3$ | H | $-CH_2-CH_2-O-$ | $CF_3$ | |
| 1.585 | 2-Cl | H | H | $-(CH_2)_3-$ | $CF_3$ | |
| 1.586 | 2-Cl | H | H | $-(CH_2)_4-$ | $CF_3$ | |
| 1.587 | 2-Cl | 5-Cl | H | $-(CH_2)_3-$ | Cl | |
| 1.588 | 2-Cl | 5-Cl | H | $-(CH_2)_3-$ | $CF_3$ | Fp. 165-166°C |
| 1.589 | 2-Cl | 5-Cl | H | $-(CH_2)_3-$ | $OCH_3$ | |
| 1.590 | 2-Cl | 5-Cl | H | $-(CH_2)_3-$ | $CF_2Cl$ | |
| 1.591 | 2-Cl | 5-Cl | H | $-(CH_2)_3-$ | $CF_2CF_3$ | |
| 1.592 | 2-Cl | 5-Cl | H | $-(CH_2)_3-$ | $CHCl_2$ | |
| 1.593 | 2-Cl | 5-Cl | H | $-C(CH_3)_2-CH_2-CH_2-$ | Cl | |
| 1.594 | 2-Cl | 5-Cl | H | $-C(CH_3)_2-CH_2-CH_2-$ | $OCH_3$ | |
| 1.595 | 2-Cl | 5-Cl | H | $-C(CH_3)_2-CH_2-CH_2-$ | $CF_3$ | |
| 1.596 | 2-Cl | 5-Cl | H | $-C(CH_3)_2-CH_2-CH_2-$ | $CF_2Cl$ | |
| 1.597 | 2-Cl | 5-Cl | H | $-C(CH_3)_2-CH_2-CH_2-$ | $CHCl_2$ | |
| 1.598 | 2-Cl | 5-Cl | H | $-C(CH_3)_2-CH_2-CH_2-$ | $CF_2CF_3$ | |
| 1.599 | 2-Cl | 5-Cl | H | $-CH_2-CH_2-S-$ | Cl | |
| 1.600 | 2-Cl | 5-Cl | H | $-CH_2-CH_2-S-$ | $CF_3$ | |
| 1.601 | 2-Cl | 5-Cl | H | $-(CH_2)_4-$ | Cl | Fp. 168-169°C |
| 1.602 | 2-Cl | 5-Cl | H | $-(CH_2)_4-$ | $OCH_3$ | |
| 1.603 | 2-Cl | 5-Cl | H | $-(CH_2)_4-$ | $SCH_3$ | |
| 1.604 | 2-Cl | 5-Cl | H | $-(CH_2)_4-$ | $SOCH_3$ | |
| 1.605 | 2-Cl | 5-Cl | H | $-(CH_2)_4-$ | $SO_2CH_3$ | |

Tabelle 1 (Fortsetzung)

| Verb.No | $R^1$ | $R^2$ | $R^3$ | $-R^5 - R^6 -$ | $R^4$ | Phys. Daten |
|---|---|---|---|---|---|---|
| 1.606 | 2-Cl | 5-Cl | H | $-(CH_2)_4-$ | $CF_3$ | Fp. 171-172°C |
| 1.607 | 2-Cl | 5-Cl | H | $-(CH_2)_4-$ | $CF_2Cl$ | |
| 1.608 | 2-Cl | 5-Cl | H | $-(CH_2)_4-$ | $CHF_2$ | |
| 1.609 | 2-Cl | 5-Cl | H | $-(CH_2)_4-$ | $CHCl_2$ | |
| 1.610 | 2-Cl | 5-Cl | H | $-(CH_2)_4-$ | $CHFCl$ | |
| 1.611 | 2-Cl | 5-Cl | H | $-(CH_2)_4-$ | $CF_2CF_3$ | |
| 1.612 | 2-Cl | 5-Cl | H | $-(CH_2)_4-$ | $C_3F_7n$ | |
| 1.613 | 2-Cl | 5-Cl | H | $-C(CH_3)_2-(CH_2)_3-$ | Cl | |
| 1.614 | 2-Cl | 5-Cl | H | $-C(CH_3)_2-(CH_2)_3-$ | $OCH_3$ | |
| 1.615 | 2-Cl | 5-Cl | H | $-C(CH_3)_2-(CH_2)_3-$ | $CF_3$ | |
| 1.616 | 2-Cl | 5-Cl | H | $-C(CH_3)_2-(CH_2)_3-$ | $CF_2Cl$ | |
| 1.617 | 2-Cl | 5-Cl | H | $-(CH_2)_5-$ | Cl | |
| 1.618 | 2-Cl | 5-Cl | H | $-(CH_2)_5-$ | $OCH_3$ | |
| 1.619 | 2-Cl | 5-Cl | H | $-(CH_2)_5-$ | $CF_3$ | Fp. 145-146°C |
| 1.620 | 2-Cl | 5-Cl | H | $-(CH_2)_5-$ | $CF_2Cl$ | |
| 1.621 | 2-Cl | 5-Cl | H | $-(CH_2)_6-$ | Cl | |
| 1.622 | 2-Cl | 5-Cl | H | $-(CH_2)_6-$ | $OCH_3$ | |
| 1.623 | 2-Cl | 5-Cl | H | $-(CH_2)_6-$ | $CF_3$ | Fp. 152-153°C |
| 1.624 | 2-Cl | 5-Cl | H | $-(CH_2)_6-$ | $CF_2Cl$ | |
| 1.625 | 2-Cl | 6-Cl | H | $-(CH_2)_3-$ | Cl | |
| 1.626 | 2-Cl | 6-Cl | H | $-(CH_2)_3-$ | $OCH_3$ | |
| 1.627 | 2-Cl | 6-Cl | H | $-(CH_2)_2-$ | $CF_3$ | Fp. 185-186°C |
| 1.628 | 2-Cl | 6-Cl | H | $-(CH_2)_3-$ | $CF_2Cl$ | |
| 1.629 | 2-Cl | 6-Cl | H | $-(CH_2)_3-$ | $CHF_2$ | Fp. 182-183°C |
| 1.630 | 2-Cl | 6-Cl | H | $-(CH_2)_3-$ | $CF_2CF_3$ | |
| 1.631 | 2-Cl | 6-Cl | H | $-(CH_2)_4-$ | Cl | |
| 1.632 | 2-Cl | 6-Cl | H | $-(CH_2)_4-$ | $OCH_3$ | |
| 1.633 | 2-Cl | 6-Cl | H | $-(CH_2)_4-$ | $CF_3$ | Fp. 179-180°C |
| 1.634 | 2-Cl | 6-Cl | H | $-(CH_2)_4-$ | $CF_2Cl$ | |
| 1.635 | 2-Cl | 6-Cl | H | $-(CH_2)_4-$ | $CHCl_2$ | |
| 1.636 | 2-Cl | 6-Cl | H | $-(CH_2)_4-$ | $CHF_2$ | |
| 1.637 | 2-Cl | 6-Cl | H | $-CH_2-CH_2-CHCH_3-CH_2-$ | $CF_3$ | |
| 1.638 | 2-Cl | 6-Cl | H | $-CH_2-CH_2-CHCH_3-CH_2-$ | $CF_2Cl$ | |
| 1.639 | 2-Cl | 6-Cl | H | $-CH_2-CH_2-CHCH_3-CH_2-$ | $CHF_3$ | |
| 1.640 | 2-Cl | 6-Cl | H | $-(CH_2)_5-$ | $CF_3$ | |
| 1.641 | 2-Cl | $6-CH_3$ | H | $-(CH_2)_3-$ | Cl | |
| 1.642 | 2-Cl | $6-CH_3$ | H | $-(CH_2)_3-$ | $CF_3$ | Fp. 165-167°C |
| 1.643 | 2-Cl | $6-CH_3$ | H | $-(CH_2)_3-$ | $CF_2Cl$ | Fp. 182-183°C |
| 1.644 | 2-Cl | $6-CH_3$ | H | $-(CH_2)_3-$ | $CHF_2$ | Fp. 173-174°C |
| 1.645 | 2-Cl | $6-CH_3$ | H | $-(CH_2)_4-$ | Cl | |
| 1.646 | 2-Cl | $6-CH_3$ | H | $-(CH_2)_4-$ | $CF_3$ | Fp. 170-172°C |
| 1.647 | 2-Cl | $6-CH_3$ | H | $-(CH_2)_4-$ | $CF_2Cl$ | Fp. 185-186°C |
| 1.648 | 2-Cl | $6-CH_3$ | H | $-(CH_2)_4-$ | $CHF_2$ | Fp. 167-168°C |
| 1.649 | 2-Cl | $6-CH_3$ | H | $-(CH_2)_5-$ | $CF_3$ | |
| 1.650 | 2-Cl | $6-CH_3$ | H | $-(CH_2)_6-$ | $CF_3$ | |
| 1.651 | 2-Br | H | H | $-(CH_2)_3-$ | Cl | |
| 1.652 | 2-Br | H | H | $-(CH_2)_3-$ | $CF_3$ | |
| 1.653 | 2-Br | H | H | $-(CH_2)_4-$ | Cl | |

Tabelle 1 (Fortsetzung)

| Verb.No | $R^1$ | $R^2$ | $R^3$ | $-R^5-R^6-$ | $R^4$ | Phys. Daten |
|---|---|---|---|---|---|---|
| 1.654 | 2-Br | H | H | $-(CH_2)_4-$ | $CF_3$ | |
| 1.655 | 2-Br | H | H | $-(CH_2)_4-$ | $CF_2Cl$ | |
| 1.656 | $2-CF_3$ | H | H | $-(CH_2)_3-$ | Cl | |
| 1.657 | $2-CF_3$ | H | H | $-(CH_2)_3-$ | $CF_3$ | |
| 1.658 | $2-CF_3$ | H | H | $-(CH_2)_3-$ | $CF_2Cl$ | |
| 1.659 | $2-CF_3$ | 6-Cl | H | $-(CH_2)_3-$ | $CF_3$ | |
| 1.660 | $2-CF_3$ | 6-Cl | H | $-(CH_2)_4-$ | $CF_3$ | |
| 1.661 | $2-OCH_3$ | H | H | $-(CH_2)_3-$ | Cl | |
| 1.662 | $2-OCH_3$ | H | H | $-(CH_2)_3-$ | $CF_3$ | |
| 1.663 | $2-OCH_3$ | H | H | $-(CH_2)_4-$ | Cl | |
| 1.664 | $2-OCH_3$ | H | H | $-(CH_2)_4-$ | $CF_3$ | |
| 1.665 | $2-OCH_3$ | 5-Cl | H | $-(CH_2)_3-$ | Cl | |
| 1.666 | $2-OCH_3$ | 5-Cl | H | $-(CH_2)_3-$ | $CF_3$ | |
| 1.667 | $2-OCH_3$ | 5-Cl | H | $-(CH_2)_4-$ | Cl | |
| 1.668 | $2-OCH_3$ | 5-Cl | H | $-(CH_2)_4-$ | $CF_3$ | |
| 1.669 | $2-OCHF_2$ | H | H | $-(CH_2)_3-$ | Cl | |
| 1.670 | $2-OCHF_2$ | H | H | $-(CH_2)_3-$ | $CF_3$ | |
| 1.671 | $2-OCHF_2$ | H | H | $-(CH_2)_4-$ | Cl | |
| 1.672 | $2-OCHF_2$ | H | H | $-(CH_2)_4-$ | $CF_3$ | Fp. 134-135°C |
| 1.673 | $2-OCHF_2$ | 5-Cl | H | $-(CH_2)_3-$ | Cl | |
| 1.674 | $2-OCHF_2$ | 5-Cl | H | $-(CH_2)_3-$ | $CF_3$ | |
| 1.675 | $2-OCHF_2$ | 5-Cl | H | $-(CH_2)_4-$ | Cl | |
| 1.676 | $2-OCHF_2$ | 5-Cl | H | $-(CH_2)_4-$ | $CF_3$ | |
| 1.677 | $2-OCF_3$ | H | H | $-(CH_2)_3-$ | Cl | |
| 1.678 | $2-OCF_3$ | H | H | $-(CH_2)_3-$ | $CF_3$ | |
| 1.679 | $2-OCF_3$ | H | H | $-(CH_2)_4-$ | Cl | |
| 1.680 | $2-OCF_3$ | H | H | $-(CH_2)_4-$ | $CF_3$ | |
| 1.681 | $2-OCF_3$ | 5-Cl | H | $-(CH_2)_3-$ | Cl | |
| 1.682 | $2-OCF_3$ | 5-Cl | H | $-(CH_2)_3-$ | $CF_3$ | |
| 1.683 | $2-OCF_3$ | 5-Cl | H | $-(CH_2)_4-$ | $CF_3$ | |
| 1.684 | $2-CH_3$ | 5-Cl | H | $-(CH_2)_3-$ | Cl | |
| 1.685 | $2-CH_3$ | 5-Cl | H | $-(CH_2)_3-$ | $CF_3$ | |
| 1.686 | $2-CH_3$ | 5-Cl | H | $-(CH_2)_4-$ | Cl | |
| 1.687 | $2-CH_3$ | 5-Cl | H | $-(CH_2)_4-$ | $CF_3$ | |
| 1.688 | 2-CN | H | H | $-(CH_2)_3-$ | $CF_3$ | |
| 1.689 | 2-CN | H | H | $-(CH_2)_4-$ | $CF_3$ | |
| 1.690 | $2-COOCH_3$ | H | H | $-(CH_2)_3-$ | $CF_3$ | |
| 1.691 | $2-COOCH_3$ | H | H | $-(CH_2)_4-$ | $CF_3$ | |
| 1.692 | $2-CH_3$ | $6-CH_3$ | H | $-(CH_2)_4-$ | Cl | |
| 1.693 | $2-CH_3$ | $6-CH_3$ | H | $-(CH_2)_4-$ | $CF_3$ | |
| 1.694 | $2-CH_3$ | $6-CH_3$ | H | $-(CH_2)_3-$ | $CF_3$ | |
| 1.695 | $2-CH_3$ | $6-C_2H_5$ | H | $-(CH_2)_3-$ | $CF_3$ | |
| 1.696 | $2-CH_3$ | $6-C_2H_5$ | H | $-(CH_2)_4-$ | $CF_3$ | |
| 1.697 | $2-NO_2$ | H | H | $-(CH_2)_3-$ | $CH_3$ | |
| 1.698 | $2-NO_2$ | H | H | $-(CH_2)_4-$ | $CH_3$ | |
| 1.699 | $2-NO_2$ | $6-CH_3$ | H | $-(CH_2)_3-$ | $CH_3$ | |
| 1.700 | $2-NO_2$ | $6-CH_3$ | H | $-(CH_2)_4-$ | $CH_3$ | |

Tabelle 1 (Fortsetzung)

| Verb.No | $R^1$ | $R^2$ | $R^3$ | $R^4$ | $R^5$ | $R^6$ | Phys. Daten |
|---|---|---|---|---|---|---|---|
| 1.701 | 2-$SCH_3$ | H | H | $CH_3$ | $CF_3$ | $CH_3$ | |
| 1.702 | 2-$SOCH_3$ | H | H | $CH_3$ | $CF_3$ | $CH_3$ | |
| 1.703 | 2-$SCHF_2$ | H | H | $CH_3$ | $CF_3$ | $CH_3$ | |
| 1.704 | 2-$SOCHF_2$ | H | H | $CH_3$ | $CF_3$ | $CH_3$ | |
| 1.705 | 2-$CHF_2$ | H | H | $C_2H_5$ | $CF_3$ | $CH_3$ | |
| 1.706 | 2-$CH_2F$ | H | H | $CH_3$ | $CF_3$ | $CH_3$ | |
| 1.707 | 2-$CF_2CH_3$ | H | H | $CH_3$ | $CF_3$ | $CH_3$ | |
| 1.708 | 2-$CH_2CH_2CF_3$ | H | H | $CH_3$ | $CF_3$ | $CH_3$ | |
| 1.709 | 2-$CHClCHClCF_3$ | H | H | $CH_3$ | $CF_3$ | $CH_3$ | |
| 1.710 | 2-$OCF_2CHF_2$ | H | H | $CH_3$ | $CF_3$ | $CH_3$ | |
| 1.711 | 2-$OCF_2CHF_2$ | 5-Cl | H | $CH_3$ | $CF_3$ | $CH_3$ | |
| 1.712 | 2-$SCH_3$ | 5-Cl | H | $CH_3$ | $CF_3$ | $CH_3$ | |
| 1.713 | 2-$SO_2CH_3$ | 5-Cl | H | $CH_3$ | $CF_3$ | $CH_3$ | |
| 1.714 | 2-$SCHF_2$ | 5-Cl | H | $CH_3$ | $CF_3$ | $CH_3$ | |
| 1.715 | 2-$SO_2CHF_2$ | 5-Cl | H | $CH_3$ | $CF_3$ | $CH_3$ | |
| 1.716 | 2-$CF_2CF_3$ | H | H | $C_2H_5$ | $CF_3$ | $CH_3$ | |
| 1.717 | 2-$CF_2CF_3$ | H | H | $CH_3$ | $CF_3$ | Cl | |
| 1.718 | 2-$OCH_2CH_2Cl$ | H | H | $CH_3$ | $CF_3$ | $CH_3$ | |
| 1.719 | 2-$OCH_2CH_2Cl$ | 5-Cl | H | $C_2H_5$ | $CF_3$ | $CH_3$ | |
| 1.720 | 2-$COOCH_3$ | 6-Cl | H | $CH_3$ | $CF_3$ | $CH_3$ | |
| 1.721 | 2-$COOCH_3$ | 6-Cl | H | $C_2H_5$ | $CF_3$ | $CH_3$ | |
| 1.722 | 2-$COOC_2H_5$ | 6-Cl | H | $CH_3$ | $CF_3$ | $SCH_3$ | |
| 1.723 | 2-$COOCH_3$ | 6-Cl | H | $CH_3$ | Cl | $SCH_3$ | |
| 1.724 | 2-$COOCH_3$ | 6-Cl | H | $CH_3$ | Br | $SCH_3$ | |
| 1.725 | 2-$COOCH_3$ | 6-$CH_3$ | H | $CH_3$ | $CF_3$ | $CH_3$ | |
| 1.726 | 2-$COOCH_3$ | 6-$CH_3$ | H | $CF_3$ | Cl | $CH_3$ | |
| 1.727 | 2-$COOC_3H_7$(n) | 6-$CH_3$ | H | $CH_3$ | $CF_3$ | $CH_3$ | |
| 1.728 | 2-$NO_2$ | H | H | $CH_3$ | Br | Cl | |
| 1.729 | 2-$NO_2$ | 6-$CH_3$ | H | $CF_3$ | F | $CH_3$ | |
| 1.730 | 2-$NO_2$ | 6-$CH_3$ | H | $CF_3$ | Br | $CH_3$ | |
| 1.731 | 2-$NO_2$ | 6-$CH_3$ | H | $CH_3$ | F | $CH_3$ | |
| 1.732 | 2-$NO_2$ | 6-$CH_3$ | H | $CH_3$ | Br | $CH_3$ | |
| 1.733 | 2-$NO_2$ | 6-$CH_3$ | H | $CH_3$ | $CF_3$ | Cyclopentyl | |
| 1.734 | 2-$NO_2$ | 6-$CH_3$ | H | $CH_3$ | $CF_3$ | Cyclopropyl | |
| 1.735 | 2-$NO_2$ | H | H | $CH_3$ | $CF_3$ | Cyclohexyl | |
| 1.736 | 2-$NO_2$ | H | H | $C_2H_5$ | $CF_3$ | Cyclopentyl | |
| 1.737 | 2-Cl | 5-Cl | H | $CH_3$ | $CF_3$ | Cyclopentyl | |
| 1.738 | 2-$NO_2$ | H | H | Cyclopentyl | $CF_3$ | $CH_3$ | |
| 1.739 | 2-$NO_2$ | 6-$CH_3$ | H | Cyclopentyl | $CF_3$ | $CH_3$ | |

Tabelle 1 (Fortsetzung)

| Verb.No | $R^1$ | $R^2$ | $R^3$ | $R^4$ | $R^5$ | $R^6$ | Phys. Daten |
|---|---|---|---|---|---|---|---|
| 1.740 | 2-Cl | 5-Cl | H | Cyclopentyl | $CF_3$ | $CH_3$ | |
| 1.741 | 2-Cl | 5-Cl | H | Cyclohexyl | $CF_3$ | $CH_3$ | |
| 1.742 | 2-Cl | 5-Cl | H | Cyclohexyl | Cl | $CH_3$ | |
| 1.743 | 2-$NO_2$ | H | H | Cyclohexyl | $CF_3$ | $CH_3$ | |
| 1.744 | 2-$NO_2$ | 6-$CH_3$ | H | Cyclohexyl | $CF_3$ | $CH_3$ | |
| 1.745 | 2-$NO_2$ | H | H | $SCHF_2$ | $CF_3$ | $CH_3$ | |
| 1.746 | 2-$NO_2$ | H | H | $SCF_3$ | $CF_3$ | $CH_3$ | |
| 1.747 | 2-$NO_2$ | 6-$CH_3$ | H | $SCHF_2$ | $CF_3$ | $CH_3$ | |
| 1.748 | 2-$NO_2$ | 6-$CH_3$ | H | SCHFCl | $CF_3$ | $CH_3$ | |
| 1.749 | 2-Cl | 5-Cl | H | $SCHF_2$ | $CF_3$ | $CH_3$ | |
| 1.750 | 2-Cl | 6-$CH_3$ | H | $SCHF_2$ | $CF_3$ | $CH_3$ | |
| 1.751 | 2-$NO_2$ | 6-$CH_3$ | H | Phenyl | $CF_3$ | $CH_3$ | |
| 1.752 | 2-$NO_2$ | 6-$CH_3$ | H | 4-Cl-Phenyl | $CF_3$ | $CH_3$ | |
| 1.753 | 2-$NO_2$ | 6-$CH_3$ | H | 2-Furyl | $CF_3$ | $CH_3$ | |
| 1.754 | 2-$NO_2$ | H | H | 2-Furyl | $CF_3$ | $CH_3$ | |
| 1.755 | 2-Cl | H | H | 2-Furyl | $CF_3$ | $CH_3$ | |

| Verb.No | $R^1$ | $R^2$ | $R^3$ | $-R^5-R^6$ | $R^4$ | Phys. Daten |
|---|---|---|---|---|---|---|
| 1.756 | 2-$NO_2$ | H | H | $-CH_2CH_2COCH_2-$ | $CF_3$ | |
| 1.757 | 2-$NO_2$ | 6-$CH_3$ | H | $-CH_2CH_2COCH_2-$ | $CF_3$ | |
| 1.758 | 2-$NO_2$ | H | H | $-CH_2CH_2CHClCH_2-$ | $CF_3$ | |
| 1.759 | 2-$NO_2$ | 6-$CH_3$ | H | $-CH_2CH_2CHClCH_2-$ | $CF_3$ | |
| 1.760 | 2-Cl | 5-Cl | H | $-CH_2CH_2CHClCH_2-$ | $CF_3$ | |
| 1.761 | 2-Cl | 5-Cl | H | $-CH_2CH_2COCH_2-$ | $CF_3$ | |
| 1.762 | 2-Cl | 6-Cl | H | $-CH_2CH_2COCH_2-$ | $CF_3$ | |
| 1.763 | 2-$OCF_3$ | H | H | $-CH_2CH_2COCH_2-$ | $CF_3$ | |
| 1.764 | 2-Cl | 6-$CH_3$ | H | $-CH_2CH_2COCH_2-$ | $CF_3$ | |
| 1.765 | 2-$SCH_3$ | H | H | $-CH_2CH_2CH_2CH_2-$ | $CF_3$ | |
| 1.766 | 2-$SCH_3$ | H | H | $-CH_2-CH_2CH_2-$ | $CF_3$ | |
| 1.767 | 2-$SC_2H_5$ | H | H | $-CH_2-CH_2-CH_2-CH_2-$ | $CF_3$ | |
| 1.768 | 2-$SOC_2H_5$ | H | H | $-CH_2-CH_2-CH_2-CH_2-$ | $CF_3$ | |
| 1.769 | 2-$SCHF_2$ | H | H | $-CH_2-CH_2-CH_2-CH_2-$ | $CF_3$ | |
| 1.770 | 2-$SOCHF_2$ | H | H | $-CH_2-CH_2-CH_2-CH_2-$ | $CF_3$ | |
| 1.771 | 2-F | 6-Cl | H | $-CH_2-CH_2-CH_2-CH_2-$ | $CF_3$ | Fp. 192-193°C |
| 1.772 | 2-F | 6-Cl | H | $-CH_2-CH_2-CH_2-$ | $CF_3$ | Fp. 176-177°C |
| 1.773 | 2-$SOCH_3$ | 5-Cl | H | $-CH_2-CH_2-CH_2-CH_2-$ | $CF_3$ | Fp. 164-165°C |

## H 2. Zwischenverbindungen der Formel II

### H.2.1. 2-(2-Methyl-6-nitrophenylamino)-5,6,7,8-tetrahydro-4-trifluormethyl-chinazolin

Eine Mischung aus 10,0 g N-(2-Methyl-6-nitrophenyl)-guanidin und 10,0 g 2-Trifluoracetylcyclohexanon werden in 60 ml Tetrahydrofuran 90 min bei Raumtemperatur gerührt und danach 3 Stunden zum Sieden erhitzt. Nach Abdestillieren des Lösungsmittels wird der Rückstand an Kieselgel mit Essigester/Hexan (1:2) gereinigt und das so erhaltene Produkt aus Hexan umkristallisiert.

Man isoliert 11,0 g der Titelverbindung der Formel

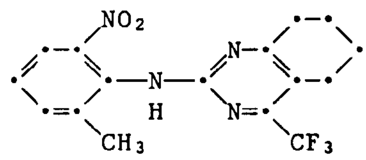

als Kristalle vom Smp. 103-104°C (Verb. No. 2.563).

Analog sind die Verbindungen der Tabelle 2 erhältlich:

Tabelle 2: Verbindungen der Formel

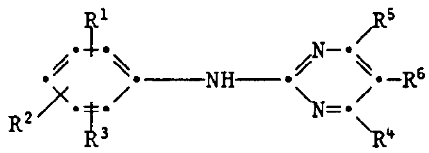

| Verb.No | $R^1$ | $R^2$ | $R^3$ | $R^4$ | $R^5$ | $R^6$ | Phys. Daten |
|---|---|---|---|---|---|---|---|
| 2.001 | 2-Cl | H | H | $CH_3$ | Cl | $CH_3$ | |
| 2.002 | 2-Cl | H | H | $CH_3$ | $CF_3$ | $CH_3$ | |
| 2.003 | 2-Cl | H | H | $C_2H_5$ | $CF_3$ | $CH_3$ | |
| 2.004 | 2-Cl | H | H | $OCH_3$ | $CF_3$ | $CH_3$ | |
| 2.005 | 2-Cl | H | H | $CH_3$ | $CF_3$ | $C_2H_5$ | |
| 2.006 | 2-F | H | H | $CH_3$ | $CF_3$ | $CH_3$ | |
| 2.007 | 2-F | H | H | $CH_3$ | Cl | $CH_3$ | |
| 2.008 | 2-F | H | H | $C_2H_5$ | $CF_3$ | $CH_3$ | |
| 2.009 | 2-F | H | H | $SCH_3$ | $CF_3$ | $CH_3$ | |
| 2.010 | 2-F | H | H | $OCH_3$ | $CH_3$ | CN | |
| 2.011 | 2-Br | H | H | $CH_3$ | Cl | $CH_3$ | |
| 2.012 | 2-Br | H | H | $CH_3$ | $CF_3$ | $CH_3$ | |
| 2.013 | 2-Br | H | H | $C_2H_5$ | $CF_3$ | $CH_3$ | |
| 2.014 | 2-Br | H | H | $C_2H_5$ | $CF_2Cl$ | $CH_3$ | |
| 2.015 | 2-Br | H | H | $C_2H_5$ | CHFCl | $CH_3$ | |
| 2.016 | 2-Br | H | H | $C_2H_5$ | $C_2F_5$ | $CH_3$ | |
| 2.017 | 2-Br | H | H | $C_2H_5$ | $CHCl_2$ | $CH_3$ | |
| 2.018 | 2-Br | H | H | $CH_3$ | $CF_2CF_3$ | $CH_3$ | |
| 2.019 | 2-Br | H | H | $OCH_3$ | $CF_3$ | $CH_3$ | |
| 2.020 | 2-Br | H | H | $SCH_3$ | $CF_3$ | $CH_3$ | |
| 2.021 | 2-Br | H | H | $SCH_3$ | $CH_3$ | $CH_3$ | |
| 2.022 | 2-Br | H | H | $SO_2CH_3$ | $CH_3$ | $CH_3$ | |
| 2.023 | 2-Br | H | H | Cyclopropyl | $CF_3$ | $CH_3$ | |
| 2.024 | 2-Br | H | H | $C_3H_7(i)$ | $CF_3$ | $CH_3$ | |
| 2.025 | 2-Br | H | H | $CH_3$ | Cl | $CH_2CH_2Cl$ | |
| 2.026 | 2-Br | H | H | $CH_3$ | Cl | $CH_2CH_2OCH_3$ | |
| 2.027 | 2-Br | H | H | $OCH_3$ | $CH_3$ | $CH_2CH_2OCH_3$ | |
| 2.028 | 2-J | H | H | $CH_3$ | Cl | $CH_3$ | |
| 2.029 | 2-J | H | H | $CH_3$ | $CF_3$ | $CH_3$ | |
| 2.030 | 2-J | H | H | $C_2H_5$ | $CF_3$ | $CH_3$ | |
| 2.031 | 2-J | H | H | $OCH_3$ | $CH_3$ | $CH_3$ | |
| 2.032 | 2-J | H | H | $CH_3$ | Cl | $C_2H_5$ | |
| 2.033 | 2-J | H | H | Cyclopropyl | $CF_3$ | $CH_3$ | |
| 2.034 | 2-$CF_3$ | H | H | $CH_3$ | Cl | $CH_3$ | |
| 2.035 | 2-$CF_3$ | H | H | $OCH_3$ | $CH_3$ | $CH_3$ | |
| 2.036 | 2-$CF_3$ | H | H | $SCH_3$ | $CH_3$ | $CH_3$ | |
| 2.037 | 2-$CF_3$ | H | H | $SO_2CH_3$ | $CH_3$ | $CH_3$ | |
| 2.038 | 2-$CF_3$ | H | H | $CH_3$ | $CF_3$ | $CH_3$ | |
| 2.039 | 2-$CF_3$ | H | H | $C_2H_5$ | $CF_3$ | $CH_3$ | |
| 2.040 | 2-$CF_3$ | H | H | $C_2H_5$ | $CF_2Cl$ | $CH_3$ | |
| 2.041 | 2-$CF_3$ | H | H | $C_2H_5$ | $CHCl_2$ | $CH_3$ | |

Tabelle 2 (Fortsetzung)

| Verb.No | $R^1$ | $R^2$ | $R^3$ | $R^4$ | $R^5$ | $R^6$ | Phys. Daten |
|---|---|---|---|---|---|---|---|
| 2.042 | 2-$CF_3$ | H | H | $C_2H_5$ | $CF_2CF_3$ | $CH_3$ | |
| 2.043 | 2-$CF_3$ | H | H | $OCH_3$ | $CH_3$ | CN | |
| 2.044 | 2-$CF_3$ | H | H | $OCH_3$ | $CH_3$ | $NO_2$ | |
| 2.045 | 2-$CF_3$ | H | H | $OCH_3$ | $CH_3$ | Cl | |
| 2.046 | 2-$NO_2$ | H | H | $CH_3$ | Cl | $CH_3$ | |
| 2.047 | 2-$NO_2$ | H | H | $CH_3$ | $CF_3$ | $CH_3$ | |
| 2.048 | 2-$NO_2$ | H | H | $OCH_3$ | $CF_3$ | $CH_3$ | |
| 2.049 | 2-$NO_2$ | H | H | $SCH_3$ | $CH_3$ | $CH_3$ | |
| 2.050 | 2-$NO_2$ | H | H | $SOCH_3$ | $CH_3$ | $CH_3$ | |
| 2.051 | 2-$NO_2$ | H | H | $SO_2CH_3$ | $CH_3$ | $CH_3$ | |
| 2.052 | 2-$NO_2$ | H | H | $SCH_3$ | $CF_3$ | $CH_3$ | |
| 2.053 | 2-$NO_2$ | H | H | $CH_3$ | $CF_3$ | Phenyl | |
| 2.054 | 2-$NO_2$ | H | H | $OCH_3$ | $CF_3$ | 4-Cl-Phenyl | |
| 2.055 | 2-$NO_2$ | H | H | Cl | $CF_3$ | 4-Cl-Phenyl | |
| 2.056 | 2-$NO_2$ | H | H | $CH_3$ | Cl | $CH_2CH_2Cl$ | Fp. 145-146°C |
| 2.057 | 2-$NO_2$ | H | H | $OCH_3$ | $CH_3$ | $CH_2CH_2OCH_3$ | |
| 2.058 | 2-$NO_2$ | H | H | $C_2H_5$ | $CF_3$ | $CH_3$ | Fp. 109-110°C |
| 2.059 | 2-$NO_2$ | H | H | $C_2H_5$ | $CF_2Cl$ | $CH_3$ | |
| 2.060 | 2-$NO_2$ | H | H | $C_2H_5$ | $CHCl_2$ | $CH_3$ | |
| 2.061 | 2-$NO_2$ | H | H | $C_2H_5$ | CHFCl | $CH_3$ | |
| 2.062 | 2-$NO_2$ | H | H | $C_2H_5$ | $C_2F_5$ | $CH_3$ | |
| 2.063 | 2-$NO_2$ | H | H | $C_2H_5$ | $C_3F_7$(n) | $CH_3$ | |
| 2.064 | 2-$NO_2$ | H | H | $C_2H_5$ | $CCl_2CF_3$ | $CH_3$ | |
| 2.065 | 2-$NO_2$ | H | H | $C_3H_7$(n) | $CF_3$ | $CH_3$ | |
| 2.066 | 2-$NO_2$ | H | H | $C_3H_7$(n) | $CF_3$ | $C_2H_5$ | |
| 2.067 | 2-$NO_2$ | H | H | $C_3H_7$(i) | $CF_3$ | $CH_3$ | |
| 2.068 | 2-$NO_2$ | H | H | Cyclopropyl | $CF_3$ | $CH_3$ | |
| 2.069 | 2-$NO_2$ | H | H | $CH_3$ | $OCHF_2$ | $CH_3$ | |
| 2.070 | 2-$NO_2$ | H | H | $OCH_3$ | $CH_3$ | $NO_2$ | |
| 2.071 | 2-$NO_2$ | H | H | $OCH_3$ | $CH_3$ | Cl | |
| 2.072 | 2-$NO_2$ | H | H | $OCH_3$ | $CH_3$ | CN | |
| 2.073 | 2-$NO_2$ | H | H | $CH_3$ | Cl | CN | |
| 2.074 | 2-$NO_2$ | H | H | $CH_3$ | Cl | $NO_2$ | |
| 2.075 | 2-$NO_2$ | H | H | $CH_3$ | Cl | Cl | |
| 2.076 | 2-$NO_2$ | H | H | $SCH_3$ | $CH_3$ | Cl | Fp. 104-105°C |
| 2.077 | 2-$NO_2$ | H | H | $SO_2CH_3$ | $CH_3$ | Cl | |
| 2.078 | 2-$NO_2$ | H | H | $OCH_3$ | $CH_3$ | $COOCH_3$ | |
| 2.079 | 2-$NO_2$ | H | H | $CH_3$ | $CH_3$ | $COOCH_3$ | |
| 2.080 | 2-$NO_2$ | H | H | $CH_3$ | $CF_2Cl$ | $CH_3$ | |
| 2.081 | 2-$OCH_3$ | H | H | $CH_3$ | Cl | $CH_3$ | |
| 2.082 | 2-$OCH_3$ | H | H | $CH_3$ | $CF_3$ | $CH_3$ | |
| 2.083 | 2-$OCH_3$ | H | H | $C_2H_5$ | $CF_3$ | $CH_3$ | |
| 2.084 | 2-$OCH_3$ | H | H | $OCH_3$ | $CH_3$ | $CH_3$ | |
| 2.085 | 2-$OCH_3$ | H | H | $OCH_3$ | $CF_3$ | $CH_3$ | |
| 2.086 | 2-$OCF_3$ | H | H | $CH_3$ | Cl | $CH_3$ | |
| 2.087 | 2-$OCF_3$ | H | H | $CH_3$ | $CF_3$ | $CH_3$ | |
| 2.088 | 2-$OCF_3$ | H | H | $OCH_3$ | $CH_3$ | $CH_3$ | |

Tabelle 2 (Fortsetzung)

| Verb.No | $R^1$ | $R^2$ | $R^3$ | $R^4$ | $R^5$ | $R^6$ | Phys. Daten |
|---|---|---|---|---|---|---|---|
| 2.089 | 2-$OCF_3$ | H | H | $SCH_3$ | $CH_3$ | $CH_3$ | |
| 2.090 | 2-$OCF_3$ | H | H | $SO_2CH_3$ | $CH_3$ | $CH_3$ | |
| 2.091 | 2-$OCF_3$ | H | H | $C_2H_5$ | $CF_3$ | $CH_3$ | |
| 2.092 | 2-$OCF_3$ | H | H | $C_2H_5$ | $CF_2Cl$ | $CH_3$ | |
| 2.093 | 2-$OCF_3$ | H | H | $SCH_3$ | $CF_3$ | $CH_3$ | |
| 2.094 | 2-$OCF_3$ | H | H | $OCH_3$ | $CF_3$ | $CH_3$ | |
| 2.095 | 2-$OCF_3$ | H | H | $OC_2H_5$ | $CF_3$ | $CH_3$ | |
| 2.096 | 2-$OCHF_2$ | H | H | $CH_3$ | Cl | $CH_3$ | |
| 2.097 | 2-$OCHF_2$ | H | H | $CH_3$ | Cl | $C_2H_5$ | |
| 2.098 | 2-$OCHF_2$ | H | H | $OCH_3$ | $CH_3$ | $C_2H_5$ | |
| 2.099 | 2-$OCHF_2$ | H | H | $SCH_3$ | $CH_3$ | $CH_3$ | |
| 2.100 | 2-$OCHF_2$ | H | H | $SO_2CH_3$ | $CH_3$ | $CH_3$ | |
| 2.101 | 2-$OCHF_2$ | H | H | $CH_3$ | $CF_3$ | $CH_3$ | |
| 2.102 | 2-$OCHF_2$ | H | H | $C_2H_5$ | $CF_3$ | $CH_3$ | |
| 2.103 | 2-$OCHF_2$ | H | H | $C_2H_5$ | $CF_2Cl$ | $CH_3$ | |
| 2.104 | 2-$OCHF_2$ | H | H | $CH_3$ | Cl | $CH_2CH_2Cl$ | |
| 2.105 | 2-$OCHF_2$ | H | H | $OCH_3$ | $CH_3$ | $CH_2CH_2OCH_3$ | |
| 2.106 | 2-$OCHF_2$ | H | H | $OC_2H_5$ | $CH_3$ | $CH_2CH_2OC_2H_5$ | |
| 2.107 | 2-$OCHF_2$ | H | H | Cyclopropyl | $CF_3$ | $CH_3$ | |
| 2.108 | 2-$OCHF_2$ | H | H | $C_3H_7$(i) | $CF_3$ | $CH_3$ | |
| 2.109 | 2-$OC_2H_5$ | H | H | $CH_3$ | Cl | $CH_3$ | |
| 2.110 | 2-$OC_2H_5$ | H | H | $CH_3$ | $CF_3$ | $CH_3$ | |
| 2.111 | 2-$OC_2H_5$ | H | H | $OCH_3$ | $CH_3$ | $CH_3$ | |
| 2.112 | 2-$OC_2H_5$ | H | H | $OCH_3$ | $CF_3$ | $CH_3$ | |
| 2.113 | 2-CN | H | H | $CH_3$ | $CF_3$ | $CH_3$ | |
| 2.114 | 2-CN | H | H | $C_2H_5$ | $CF_3$ | $CH_3$ | |
| 2.115 | 2-CN | H | H | $OCH_3$ | $CH_3$ | $CH_3$ | |
| 2.116 | 2-CN | H | H | $OCH_3$ | $CF_3$ | $CH_3$ | |
| 2.117 | 2-CN | H | H | $SCH_3$ | $CF_3$ | $CH_3$ | |
| 2.118 | 2-CN | H | H | Cyclopropyl | $CF_3$ | $CH_3$ | |
| 2.119 | 2-CN | H | H | $OCH_3$ | $CH_3$ | Cl | |
| 2.120 | 2-$COOCH_3$ | H | H | $CH_3$ | $CF_3$ | $CH_3$ | |
| 2.121 | 2-$COOCH_3$ | H | H | $OCH_3$ | $CF_3$ | $CH_3$ | |
| 2.122 | 2-$COOCH_3$ | H | H | $C_2H_5$ | $CF_3$ | $CH_3$ | |
| 2.123 | 2-$COOCH_3$ | H | H | $CH_3$ | $CF_3$ | Phenyl | |
| 2.124 | 2-$CON(CH_3)_2$ | H | H | $CH_3$ | Cl | $CH_3$ | |
| 2.125 | 2-$CON(CH_3)_2$ | H | H | $CH_3$ | $CF_3$ | $CH_3$ | |
| 2.126 | 2-$CON(CH_3)_2$ | H | H | $OCH_3$ | $CF_3$ | $CH_3$ | |
| 2.127 | 2-$CON(CH_3)_2$ | H | H | $OCH_3$ | $CF_3$ | $C_2H_5$ | |
| 2.128 | 2-$CH_3$ | H | H | $CH_3$ | Cl | $CH_3$ | |
| 2.129 | 2-$CH_3$ | H | H | $CH_3$ | $CF_3$ | $CH_3$ | |
| 2.130 | 2-$CH_3$ | H | H | $C_2H_5$ | $CF_3$ | $CH_3$ | |
| 2.131 | 2-$CH_3$ | H | H | $CH_3$ | $CF_2Cl$ | $CH_3$ | |
| 2.132 | 2-$CH_3$ | H | H | $OCH_3$ | $CF_3$ | $CH_3$ | |
| 2.133 | 2-$C_2H_5$ | H | H | $CH_3$ | Cl | $CH_3$ | |
| 2.134 | 2-$C_2H_5$ | H | H | $CH_3$ | $CF_3$ | $CH_3$ | |
| 2.135 | 2-$C_2H_5$ | H | H | $OCH_3$ | $CF_3$ | $CH_3$ | |

Tabelle 2 (Fortsetzung)

| Verb.No | $R^1$ | $R^2$ | $R^3$ | $R^4$ | $R^5$ | $R^6$ | Phys. Daten |
|---|---|---|---|---|---|---|---|
| 2.136 | 2-$C_2H_5$ | H | H | $SCH_3$ | $CF_3$ | $CH_3$ | |
| 2.137 | 3-Cl | H | H | $CH_3$ | $CF_3$ | $CH_3$ | |
| 2.138 | 3-Br | H | H | $CH_3$ | $CF_3$ | $CH_3$ | |
| 2.139 | 3-$CF_3$ | H | H | $C_2H_5$ | $CF_3$ | $CH_3$ | |
| 2.140 | 3-Cl | 5-Cl | H | $CH_3$ | Cl | $CH_3$ | |
| 2.141 | 3-Cl | 5-Cl | H | $CH_3$ | $CF_3$ | $CH_3$ | |
| 2.142 | 3-Cl | 5-Cl | H | $C_2H_5$ | $CF_3$ | $CH_3$ | |
| 2.143 | 3-Cl | 5-Cl | H | $OCH_3$ | $CF_3$ | $CH_3$ | |
| 2.144 | 2-Cl | 5-Cl | H | $CH_3$ | Cl | $CH_3$ | |
| 2.145 | 2-Cl | 5-Cl | H | $CH_3$ | $CF_3$ | $CH_3$ | |
| 2.146 | 2-Cl | 5-Cl | H | $OCH_3$ | $CF_3$ | $CH_3$ | |
| 2.147 | 2-Cl | 5-Cl | H | $SCH_3$ | $CH_3$ | $CH_3$ | |
| 2.148 | 2-Cl | 5-Cl | H | $SOCH_3$ | $CH_3$ | $CH_3$ | |
| 2.149 | 2-Cl | 5-Cl | H | $SO_2CH_3$ | $CH_3$ | $CH_3$ | |
| 2.150 | 2-Cl | 5-Cl | H | $SC_2H_5$ | $CF_3$ | $CH_3$ | |
| 2.151 | 2-Cl | 5-Cl | H | $SC_3H_7$(i) | $CF_3$ | $CH_3$ | |
| 2.152 | 2-Cl | 5-Cl | H | $OC_2H_5$ | $CF_3$ | $CH_3$ | |
| 2.153 | 2-Cl | 5-Cl | H | $SOC_2H_5$ | $CF_3$ | $CH_3$ | |
| 2.154 | 2-Cl | 5-Cl | H | $OC_3H_7$(i) | $CF_3$ | $CH_3$ | |
| 2.155 | 2-Cl | 5-Cl | H | $CH_3$ | Cl | Phenyl | |
| 2.156 | 2-Cl | 5-Cl | H | $OCH_3$ | $CH_3$ | Phenyl | |
| 2.157 | 2-Cl | 5-Cl | H | $CH_3$ | Cl | $CH_2CH_2Cl$ | |
| 2.158 | 2-Cl | 5-Cl | H | $OCH_3$ | $CH_3$ | $CH_2CH_2OCH_3$ | |
| 2.159 | 2-Cl | 5-Cl | H | $C_2H_5$ | $CF_3$ | $CH_3$ | |
| 2.160 | 2-Cl | 5-Cl | H | $CH_3$ | $CF_2Cl$ | $CH_3$ | |
| 2.161 | 2-Cl | 5-Cl | H | $CH_3$ | $C_2H_5$ | $CH_3$ | |
| 2.162 | 2-Cl | 5-Cl | H | $CH_3$ | $C_3F_7$(n) | $CH_3$ | |
| 2.163 | 2-Cl | 5-Cl | H | $C_2H_5$ | $CF_2Cl$ | $CH_3$ | |
| 2.164 | 2-Cl | 5-Cl | H | $C_2H_5$ | $CHF_2$ | $CH_3$ | |
| 2.165 | 2-Cl | 5-Cl | H | $CH_3$ | $CCl_2CF_3$ | $CH_3$ | |
| 2.166 | 2-Cl | 5-Cl | H | $C_3H_7$(n) | $CF_3$ | $CH_3$ | |
| 2.167 | 2-Cl | 5-Cl | H | $C_3H_7$(i) | $CF_3$ | $CH_3$ | |
| 2.168 | 2-Cl | 5-Cl | H | Cyclopropyl | $CF_3$ | $CH_3$ | |
| 2.169 | 2-Cl | 5-Cl | H | $CH_3$ | $CF_3$ | $C_2H_5$ | |
| 2.170 | 2-Cl | 5-Cl | H | $CH_3$ | $CF_3$ | $C_3H_7$(i) | |
| 2.171 | 2-Cl | 5-Cl | H | $CH_3$ | $OCHF_2$ | $CH_3$ | |
| 2.172 | 2-Cl | 5-Cl | H | $OCH_3$ | $CH_3$ | $NO_2$ | |
| 2.173 | 2-Cl | 5-Cl | H | $OCH_3$ | $CH_3$ | Cl | |
| 2.174 | 2-Cl | 5-Cl | H | $OCH_3$ | $CH_3$ | CN | |
| 2.175 | 2-Cl | 5-Cl | H | $OC_2H_5$ | $CH_3$ | CN | |
| 2.176 | 2-Cl | 5-Cl | H | $CH_3$ | Cl | CN | |
| 2.177 | 2-Cl | 5-Cl | H | $CH_3$ | Cl | $NO_2$ | |
| 2.178 | 2-Cl | 5-Cl | H | $CH_3$ | Cl | Cl | |
| 2.179 | 2-Cl | 5-Cl | H | $C_2H_5$ | Cl | Cl | |
| 2.180 | 2-Cl | 5-Cl | H | $C_2H_5$ | Cl | Br | |
| 2.181 | 2-Cl | 5-Cl | H | $OCH_3$ | $CH_3$ | $COOCH_3$ | |
| 2.182 | 2-Cl | 5-Cl | H | $CH_3$ | $CH_3$ | $COOC_2H_5$ | |

Tabelle 2 (Fortsetzung)

| Verb.No | $R^1$ | $R^2$ | $R^3$ | $R^4$ | $R^5$ | $R^6$ | Phys. Daten |
|---|---|---|---|---|---|---|---|
| 2.183 | 2-Cl | 6-Cl | H | $CH_3$ | Cl | $CH_3$ | |
| 2.184 | 2-Cl | 6-Cl | H | $CH_3$ | $CF_3$ | $CH_3$ | |
| 2.185 | 2-Cl | 6-Cl | H | $CH_3$ | $CF_3$ | $C_2H_5$ | |
| 2.186 | 2-Cl | 6-Cl | H | $OCH_3$ | $CF_3$ | $CH_3$ | |
| 2.187 | 2-Cl | 6-Cl | H | $SCH_3$ | $CF_3$ | $CH_3$ | |
| 2.188 | 2-Cl | 6-Cl | H | $CF_3$ | Cl | $CH_3$ | Fp. 128-130°C |
| 2.189 | 2-Cl | 6-Cl | H | $SC_4H_9$(n) | $CF_3$ | $CH_3$ | |
| 2.190 | 2-Cl | 6-Cl | H | $SC_2H_5$ | $CH_3$ | $CH_3$ | |
| 2.191 | 2-Cl | 6-Cl | H | $SOC_2H_5$ | $CH_3$ | $CH_3$ | |
| 2.192 | 2-Cl | 6-Cl | H | $SO_2C_2H_5$ | $CH_3$ | $CH_3$ | |
| 2.193 | 2-Cl | 6-Cl | H | $C_2H_5$ | $CF_3$ | $CH_3$ | |
| 2.194 | 2-Cl | 6-Cl | H | $C_3H_7$(n) | $CF_3$ | $CH_3$ | |
| 2.195 | 2-Cl | 6-Cl | H | $C_4H_9$(n) | $CF_3$ | $CH_3$ | |
| 2.196 | 2-Cl | 6-Cl | H | Cyclopropyl | $CF_3$ | $CH_3$ | |
| 2.197 | 2-Cl | 6-Cl | H | $C_3H_7$(i) | $CF_3$ | $CH_3$ | Fp. 81-82°C |
| 2.198 | 2-Cl | 6-Cl | H | $CH_3$ | $CF_2Cl$ | $CH_3$ | |
| 2.199 | 2-Cl | 6-Cl | H | $CH_3$ | $CHF_2$ | $CH_3$ | |
| 2.200 | 2-Cl | 6-Cl | H | $CH_3$ | $CHCl_2$ | $CH_3$ | |
| 2.201 | 2-Cl | 6-Cl | H | $CH_3$ | CHFCl | $CH_3$ | |
| 2.202 | 2-Cl | 6-Cl | H | $CH_3$ | $CCl_2CF_3$ | $CH_3$ | |
| 2.203 | 2-Cl | 6-Cl | H | $CH_3$ | $OCHF_2$ | $CH_3$ | |
| 2.204 | 2-Cl | 6-Cl | H | $OCH_3$ | $CH_3$ | $NO_2$ | |
| 2.205 | 2-Cl | 6-Cl | H | $OCH_3$ | $CF_3$ | $NO_2$ | |
| 2.206 | 2-Cl | 6-Cl | H | $OCH_3$ | $CF_3$ | Cl | |
| 2.207 | 2-Cl | 6-Cl | H | $OCH_3$ | $CH_3$ | CN | |
| 2.208 | 2-Cl | 6-Cl | H | $CH_3$ | Cl | CN | |
| 2.209 | 2-Cl | 6-Cl | H | $CH_3$ | Cl | $NO_2$ | |
| 2.210 | 2-Cl | 6-Cl | H | $CH_3$ | Cl | Cl | |
| 2.211 | 2-Cl | 6-Cl | H | $CH_3$ | Cl | Br | |
| 2.212 | 2-Cl | 6-Cl | H | $OCH_3$ | $CH_3$ | $COOCH_3$ | |
| 2.213 | 2-Cl | 3-Cl | H | $CH_3$ | Cl | $CH_3$ | |
| 2.214 | 2-Cl | 3-Cl | H | $CH_3$ | $CF_3$ | $CH_3$ | |
| 2.215 | 2-Cl | 3-Cl | H | $C_2H_5$ | $CF_3$ | $CH_3$ | |
| 2.216 | 2-Cl | 3-Cl | H | $C_2H_5$ | $CF_2Cl$ | $CH_3$ | |
| 2.217 | 2-$NO_2$ | 3-$CH_3$ | H | $CH_3$ | Cl | $CH_3$ | |
| 2.218 | 2-$NO_2$ | 3-$CH_3$ | H | $CH_3$ | $CF_3$ | $CH_3$ | |
| 2.219 | 2-$NO_2$ | 3-$CH_3$ | H | $C_2H_5$ | $CF_3$ | $CH_3$ | |
| 2.220 | 2-$NO_2$ | 3-$CH_3$ | H | $C_2H_5$ | $CF_2Cl$ | $CH_3$ | |
| 2.221 | 2-$NO_2$ | 3-Cl | H | $CH_3$ | Cl | $CH_3$ | |
| 2.222 | 2-$NO_2$ | 3-Cl | H | $CH_3$ | $CF_3$ | $CH_3$ | |
| 2.223 | 2-$NO_2$ | 3-Cl | H | $OCH_3$ | $CH_3$ | $CH_3$ | |
| 2.224 | 2-$NO_2$ | 3-Cl | H | $C_2H_5$ | $CF_3$ | $CH_3$ | |
| 2.225 | 2-$NO_2$ | 3-Cl | H | $C_2H_5$ | $CF_2Cl$ | $CH_3$ | |
| 2.226 | 2-$NO_2$ | 5-Cl | H | $CH_3$ | Cl | $CH_3$ | |
| 2.227 | 2-$NO_2$ | 5-Cl | H | $CH_3$ | $CF_3$ | $CH_3$ | |
| 2.228 | 2-$NO_2$ | 5-Cl | H | $C_2H_5$ | $CF_3$ | $CH_3$ | |
| 2.229 | 2-$NO_2$ | 5-Cl | H | $CH_3$ | $CF_2Cl$ | $CH_3$ | |

Tabelle 2 (Fortsetzung)

| Verb.No | $R^1$ | $R^2$ | $R^3$ | $R^4$ | $R^5$ | $R^6$ | Phys. Daten |
|---|---|---|---|---|---|---|---|
| 2.230 | 2-$NO_2$ | 5-Cl | H | $SCH_3$ | $CH_3$ | $CH_3$ | |
| 2.231 | 2-$NO_2$ | 5-Cl | H | $SOCH_3$ | $CH_3$ | $CH_3$ | |
| 2.232 | 2-$NO_2$ | 5-Cl | H | $SO_2CH_3$ | $CH_3$ | $CH_3$ | |
| 2.233 | 2-$NO_2$ | 5-Cl | H | CN | $CH_3$ | $CH_3$ | |
| 2.234 | 2-$NO_2$ | 5-Cl | H | $OCH_3$ | $CF_3$ | $CH_3$ | |
| 2.235 | 2-$NO_2$ | 5-Cl | H | $SCH_3$ | $CF_3$ | $CH_3$ | |
| 2.236 | 2-$NO_2$ | 5-Cl | H | $OC_3H_7$(i) | $CF_3$ | $CH_3$ | |
| 2.237 | 2-$NO_2$ | 5-Cl | H | $SC_2H_5$ | $CF_3$ | $CH_3$ | |
| 2.238 | 2-$NO_2$ | 5-Cl | H | $C_3H_7$(i) | $CF_3$ | $CH_3$ | |
| 2.239 | 2-$NO_2$ | 6-Cl | H | $CH_3$ | Cl | $CH_3$ | |
| 2.240 | 2-$NO_2$ | 6-Cl | H | $CH_3$ | $CF_3$ | $CH_3$ | |
| 2.241 | 2-$NO_2$ | 6-Cl | H | $OCH_3$ | $CF_3$ | $CH_3$ | |
| 2.242 | 2-$NO_2$ | 6-Cl | H | $SCH_3$ | $CF_3$ | $CH_3$ | |
| 2.243 | 2-$NO_2$ | 6-Cl | H | $SOCH_3$ | $CF_3$ | $CH_3$ | |
| 2.244 | 2-$NO_2$ | 6-Cl | H | $SO_2CH_3$ | $CF_3$ | $CH_3$ | |
| 2.245 | 2-$NO_2$ | 6-Cl | H | $C_2H_5$ | $CF_3$ | $CH_3$ | |
| 2.246 | 2-$NO_2$ | 6-Cl | H | Cyclopropyl | $CF_3$ | $CH_3$ | |
| 2.247 | 2-$NO_2$ | 6-Cl | H | $C_3H_7$(i) | $CF_3$ | $CH_3$ | |
| 2.248 | 2-$NO_2$ | 6-Cl | H | $CH_3$ | $CF_2Cl$ | $CH_3$ | |
| 2.249 | 2-$NO_2$ | 5-$CH_3$ | H | $CH_3$ | Cl | $CH_3$ | |
| 2.250 | 2-$NO_2$ | 5-$CH_3$ | H | $CH_3$ | $CF_3$ | $CH_3$ | |
| 2.251 | 2-$NO_2$ | 5-$CH_3$ | H | $OCH_3$ | $CF_3$ | $CH_3$ | |
| 2.252 | 2-$NO_2$ | 5-$CH_3$ | H | $CF_3$ | Cl | $CH_3$ | |
| 2.253 | 2-$NO_2$ | 5-$CH_3$ | H | $SCH_3$ | $CF_3$ | $CH_3$ | |
| 2.254 | 2-$NO_2$ | 5-$CH_3$ | H | $C_2H_5$ | $CF_3$ | $CH_3$ | |
| 2.255 | 2-$NO_2$ | 5-$CH_3$ | H | $C_3H_7$(n) | $CF_3$ | $CH_3$ | |
| 2.256 | 2-$NO_2$ | 5-$CH_3$ | H | $C_3H_7$(i) | $CF_3$ | $CH_3$ | |
| 2.257 | 2-$NO_2$ | 5-$CH_3$ | H | Cyclopropyl | $CF_3$ | $CH_3$ | |
| 2.258 | 2-$NO_2$ | 5-$CH_3$ | H | $CH_3$ | $CF_2Cl$ | $CH_3$ | |
| 2.259 | 2-$NO_2$ | 5-$CH_3$ | H | $CH_3$ | CHFCl | $CH_3$ | |
| 2.260 | 2-$NO_2$ | 5-$CH_3$ | H | $OCH_3$ | $CH_3$ | $NO_2$ | |
| 2.261 | 2-$NO_2$ | 5-$CH_3$ | H | $OCH_3$ | $CH_3$ | CN | |
| 2.262 | 2-$NO_2$ | 5-$CH_3$ | H | $OCH_3$ | $CH_3$ | Cl | |
| 2.263 | 2-$NO_2$ | 5-$CH_3$ | H | $OCH_3$ | $CH_3$ | $COOCH_3$ | |
| 2.264 | 2-$NO_2$ | 5-$CH_3$ | H | $OC_3H_7$(n) | $CF_3$ | $CH_3$ | |
| 2.265 | 2-$NO_2$ | 5-$CH_3$ | H | $SC_2H_5$ | $CF_3$ | $CH_3$ | |
| 2.266 | 2-$NO_2$ | 6-$CH_3$ | H | $CH_3$ | Cl | $CH_3$ | |
| 2.267 | 2-$NO_2$ | 6-$CH_3$ | H | $OCH_3$ | $CH_3$ | $CH_3$ | |
| 2.268 | 2-$NO_2$ | 6-$CH_3$ | H | $SCH_3$ | $CH_3$ | $CH_3$ | |
| 2.269 | 2-$NO_2$ | 6-$CH_3$ | H | $SOCH_3$ | $CH_3$ | $CH_3$ | |
| 2.270 | 2-$NO_2$ | 6-$CH_3$ | H | $SO_2CH_3$ | $CH_3$ | $CH_3$ | |
| 2.271 | 2-$NO_2$ | 6-$CH_3$ | H | $CF_3$ | Cl | $CH_3$ | |
| 2.272 | 2-$NO_2$ | 6-$CH_3$ | H | $OCH_3$ | $CF_3$ | $CH_3$ | |
| 2.273 | 2-$NO_2$ | 6-$CH_3$ | H | $OC_2H_5$ | $CF_3$ | $CH_3$ | |
| 2.274 | 2-$NO_2$ | 6-$CH_3$ | H | $SCH_3$ | $CF_3$ | $CH_3$ | |
| 2.275 | 2-$NO_2$ | 6-$CH_3$ | H | $SOCH_3$ | $CF_3$ | $CH_3$ | |
| 2.276 | 2-$NO_2$ | 6-$CH_3$ | H | $SO_2CH_3$ | $CF_3$ | $CH_3$ | |

Tabelle 2 (Fortsetzung)

| Verb.No | $R^1$ | $R^2$ | $R^3$ | $R^4$ | $R^5$ | $R^6$ | Phys. Daten |
|---|---|---|---|---|---|---|---|
| 2.277 | 2-$NO_2$ | 6-$CH_3$ | H | $SC_3H_7$(i) | $CF_3$ | $CH_3$ | |
| 2.278 | 2-$NO_2$ | 6-$CH_3$ | H | $CF_3$ | Cl | $C_2H_5$ | |
| 2.279 | 2-$NO_2$ | 6-$CH_3$ | H | $OCH_3$ | $CF_3$ | $C_2H_5$ | |
| 2.280 | 2-$NO_2$ | 6-$CH_3$ | H | $SCH_3$ | $CF_3$ | $C_2H_5$ | |
| 2.281 | 2-$NO_2$ | 6-$CH_3$ | H | $CF_3$ | Cl | $C_3H_7$(i) | |
| 2.282 | 2-$NO_2$ | 6-$CH_3$ | H | $OCH_3$ | $CF_3$ | $C_3H_7$(i) | |
| 2.283 | 2-$NO_2$ | 6-$CH_3$ | H | $SC_2H_5$ | $CF_3$ | $C_3H_7$(i) | |
| 2.284 | 2-$NO_2$ | 6-$CH_3$ | H | $CH_3$ | $CF_3$ | $CH_3$ | Fp. 134-135°C |
| 2.285 | 2-$NO_2$ | 6-$CH_3$ | H | $C_2H_5$ | $CF_3$ | $CH_3$ | Fp. 112-113°C |
| 2.286 | 2-$NO_2$ | 6-$CH_3$ | H | $C_3H_7$(n) | $CF_3$ | $CH_3$ | |
| 2.287 | 2-$NO_2$ | 6-$CH_3$ | H | $C_3H_7$(i) | $CF_3$ | $CH_3$ | |
| 2.288 | 2-$NO_2$ | 6-$CH_3$ | H | Cyclopropyl | $CF_3$ | $CH_3$ | |
| 2.289 | 2-$NO_2$ | 6-$CH_3$ | H | $C_4H_9$(n) | $CF_3$ | $CH_3$ | |
| 2.290 | 2-$NO_2$ | 6-$CH_3$ | H | $CH_3$ | $CF_2Cl$ | $CH_3$ | |
| 2.291 | 2-$NO_2$ | 6-$CH_3$ | H | $CH_3$ | $CHF_2$ | $CH_3$ | |
| 2.292 | 2-$NO_2$ | 6-$CH_3$ | H | $CH_3$ | $CHCl_2$ | $CH_3$ | |
| 2.293 | 2-$NO_2$ | 6-$CH_3$ | H | $CH_3$ | $CHFCl$ | $CH_3$ | |
| 2.294 | 2-$NO_2$ | 6-$CH_3$ | H | $CH_3$ | $C_2F_5$ | $CH_3$ | |
| 2.295 | 2-$NO_2$ | 6-$CH_3$ | H | $CH_3$ | $C_3F_7$(n) | $CH_3$ | |
| 2.296 | 2-$NO_2$ | 6-$CH_3$ | H | $C_2H_5$ | $CF_2Cl$ | $CH_3$ | |
| 2.297 | 2-$NO_2$ | 6-$CH_3$ | H | $C_3H_7$(i) | $CF_2Cl$ | $CH_3$ | |
| 2.298 | 2-$NO_2$ | 6-$CH_3$ | H | Cyclopropyl | $CF_2Cl$ | $CH_3$ | |
| 2.299 | 2-$NO_2$ | 6-$CH_3$ | H | $C_2H_5$ | $C_2F_5$ | $CH_3$ | |
| 2.300 | 2-$NO_2$ | 6-$CH_3$ | H | $C_2H_5$ | $CHCl_2$ | $CH_3$ | |
| 2.301 | 2-$NO_2$ | 6-$CH_3$ | H | $CH_3$ | $CCl_2CF_3$ | $CH_3$ | |
| 2.302 | 2-$NO_2$ | 6-$CH_3$ | H | $CH_3$ | $OCHF_2$ | $CH_3$ | |
| 2.303 | 2-$NO_2$ | 6-$CH_3$ | H | $CH_3$ | Cl | $CH_2CH_2Cl$ | |
| 2.304 | 2-$NO_2$ | 6-$CH_3$ | H | $OCH_3$ | $CH_3$ | $CH_2CH_2OCH_3$ | |
| 2.305 | 2-$NO_2$ | 6-$CH_3$ | H | $SCH_3$ | $CH_3$ | $CH_2CH_2SCH_3$ | |
| 2.306 | 2-$NO_2$ | 6-$CH_3$ | H | CN | $CH_3$ | $CH_3$ | |
| 2.307 | 2-$NO_2$ | 6-$CH_3$ | H | $CH_3$ | $CF_3$ | Phenyl | |
| 2.308 | 2-$NO_2$ | 6-$CH_3$ | H | $CH_3$ | Cl | Phenyl | |
| 2.309 | 2-$NO_2$ | 6-$CH_3$ | H | $OCH_3$ | $CH_3$ | $NO_2$ | |
| 2.310 | 2-$NO_2$ | 6-$CH_3$ | H | $OCH_3$ | $CH_3$ | Cl | |
| 2.311 | 2-$NO_2$ | 6-$CH_3$ | H | $OCH_3$ | $CH_3$ | CN | |
| 2.312 | 2-$NO_2$ | 6-$CH_3$ | H | $CH_3$ | Cl | CN | |
| 2.313 | 2-$NO_2$ | 6-$CH_3$ | H | $CH_3$ | Cl | $NO_2$ | |
| 2.314 | 2-$NO_2$ | 6-$CH_3$ | H | $CH_3$ | Cl | Cl | |
| 2.315 | 2-$NO_2$ | 6-$CH_3$ | H | $SCH_3$ | $CH_3$ | Cl | |
| 2.316 | 2-$NO_2$ | 6-$CH_3$ | H | $CH_3$ | Cl | $OCH_3$ | |
| 2.317 | 2-$NO_2$ | 6-$CH_3$ | H | $OCH_3$ | $CH_3$ | $COOC_2H_5$ | |
| 2.318 | 2-$NO_2$ | 6-$CH_3$ | H | $CH_3$ | $CH_3$ | $COOCH_3$ | |
| 2.319 | 2-$NO_2$ | 5-F | H | $CH_3$ | Cl | $CH_3$ | |
| 2.320 | 2-$NO_2$ | 5-F | H | $CH_3$ | $CF_3$ | $CH_3$ | |
| 2.321 | 2-$NO_2$ | 5-F | H | $C_2H_5$ | $CF_3$ | $CH_3$ | |
| 2.322 | 2-$NO_2$ | 6-F | H | $CH_3$ | Cl | $CH_3$ | |
| 2.323 | 2-$NO_2$ | 6-F | H | $OCH_3$ | $CH_3$ | $CH_3$ | |

Tabelle 2 (Fortsetzung)

| Verb.No | $R^1$ | $R^2$ | $R^3$ | $R^4$ | $R^5$ | $R^6$ | Phys. Daten |
|---|---|---|---|---|---|---|---|
| 2.324 | 2-$NO_2$ | 6-F | H | $CH_3$ | $CF_3$ | $CH_3$ | |
| 2.325 | 2-$NO_2$ | 6-F | H | $CH_3$ | $CF_2Cl$ | $CH_3$ | |
| 2.326 | 2-$NO_2$ | 6-Br | H | $CH_3$ | Cl | $CH_3$ | |
| 2.327 | 2-$NO_2$ | 6-Br | H | $CH_3$ | $CF_3$ | $CH_3$ | |
| 2.328 | 2-$NO_2$ | 6-Br | H | $C_2H_5$ | $CF_3$ | $CH_3$ | |
| 2.329 | 2-$NO_2$ | 6-Br | H | Cyclopropyl | $CF_3$ | $CH_3$ | |
| 2.330 | 2-$NO_2$ | 6-Br | H | $CH_3$ | CHFCl | $CH_3$ | |
| 2.331 | 2-$NO_2$ | 6-Br | H | $CH_3$ | $CF_2Cl$ | $CH_3$ | |
| 2.332 | 2-$NO_2$ | 6-Br | H | $CH_3$ | $CHCl_2$ | $CH_3$ | |
| 2.333 | 2-F | 5-F | H | $CH_3$ | Cl | $CH_3$ | |
| 2.334 | 2-F | 5-F | H | $CH_3$ | $CF_3$ | $CH_3$ | |
| 2.335 | 2-F | 5-F | H | $C_2H_5$ | $CF_3$ | $CH_3$ | |
| 2.336 | 2-F | 5-F | H | $C_2H_5$ | $C_2F_5$ | $CH_3$ | |
| 2.337 | 2-F | 5-F | H | $C_2H_5$ | $CF_2Cl$ | $CH_3$ | |
| 2.338 | 2-F | 6-F | H | $CH_3$ | Cl | $CH_3$ | |
| 2.339 | 2-F | 6-F | H | $CH_3$ | $CF_3$ | $CH_3$ | |
| 2.340 | 2-F | 6-F | H | $C_2H_5$ | $CF_3$ | $CH_3$ | Fp. 75° |
| 2.341 | 2-F | 6-F | H | $OCH_3$ | $CF_3$ | $CH_3$ | |
| 2.342 | 2-F | 6-F | H | $SCH_3$ | $CF_3$ | $CH_3$ | |
| 2.343 | 2-F | 6-Cl | H | $CH_3$ | Cl | $CH_3$ | |
| 2.344 | 2-F | 6-Cl | H | $CH_3$ | $CF_3$ | $CH_3$ | |
| 2.345 | 2-F | 6-Cl | H | $CH_3$ | $C_2F_5$ | $CH_3$ | |
| 2.346 | 2-F | 6-Cl | H | $C_2H_5$ | $CF_3$ | $CH_3$ | |
| 2.347 | 2-F | 6-Cl | H | $C_2H_5$ | $CF_2Cl$ | $CH_3$ | |
| 2.348 | 2-$CF_3$ | 6-Cl | H | $CH_3$ | Cl | $CH_3$ | |
| 2.349 | 2-$CF_3$ | 6-Cl | H | $CH_3$ | $CF_3$ | $CH_3$ | |
| 2.350 | 2-$CF_3$ | 6-Cl | H | $C_2H_5$ | $CF_3$ | $CH_3$ | |
| 2.351 | 2-$CF_3$ | 6-Cl | H | $C_2H_5$ | $CF_2Cl$ | $CH_3$ | |
| 2.352 | 2-$CF_3$ | 6-Cl | H | $CH_3$ | $C_2F_5$ | $CH_3$ | |
| 2.353 | 2-$CF_3$ | 6-$CH_3$ | H | $CH_3$ | Cl | $CH_3$ | |
| 2.354 | 2-$CF_3$ | 6-$CH_3$ | H | $CH_3$ | $CF_3$ | $CH_3$ | |
| 2.355 | 2-$CF_3$ | 6-$CH_3$ | H | $C_2H_5$ | $CF_3$ | $CH_3$ | |
| 2.356 | 2-$CF_3$ | 6-$CH_3$ | H | Cyclopropyl | $CF_3$ | $CH_3$ | |
| 2.357 | 2-$OCHF_2$ | 5-Cl | H | $CH_3$ | Cl | $CH_3$ | |
| 2.358 | 2-$OCHF_2$ | 5-Cl | H | $CH_3$ | $CF_3$ | $CH_3$ | |
| 2.359 | 2-$OCHF_2$ | 5-Cl | H | $C_2H_5$ | $CF_3$ | $CH_3$ | |
| 2.360 | 2-$OCHF_2$ | 5-Cl | H | $CH_3$ | $CF_2Cl$ | $CH_3$ | |
| 2.361 | 2-$OCHF_2$ | 5-Cl | H | $CH_3$ | $CHCl_2$ | $CH_3$ | |
| 2.362 | 2-$OCHF_2$ | 5-Cl | H | $CF_3$ | Cl | $CH_3$ | |
| 2.363 | 2-$OCHF_2$ | 5-Cl | H | $OCH_3$ | $CF_3$ | $CH_3$ | |
| 2.364 | 2-$OCHF_2$ | 5-Cl | H | $SC_2H_5$ | $CF_3$ | $CH_3$ | |
| 2.365 | 2-$OCHF_2$ | 5-Cl | H | $CF_3$ | Cl | $C_2H_5$ | |
| 2.366 | 2-$OCHF_2$ | 5-Cl | H | $OCH_3$ | $CF_3$ | $C_2H_5$ | |
| 2.367 | 2-$OCHF_2$ | 5-Cl | H | $OC_2H_5$ | $CF_3$ | $C_2H_5$ | |
| 2.368 | 2-$OCHF_2$ | 5-Cl | H | $SCH_3$ | $CF_3$ | $C_2H_5$ | |
| 2.369 | 2-$OCF_3$ | 5-Cl | H | $CH_3$ | Cl | $CH_3$ | |
| 2.370 | 2-$OCF_3$ | 5-Cl | H | $CH_3$ | $CF_3$ | $CH_3$ | |

Tabelle 2 (Fortsetzung)

| Verb.No | $R^1$ | $R^2$ | $R^3$ | $R^4$ | $R^5$ | $R^6$ | Phys. Daten |
|---|---|---|---|---|---|---|---|
| 2.371 | 2-$OCF_3$ | 5-Cl | H | $C_2H_5$ | $CF_3$ | $CH_3$ | |
| 2.372 | 2-$OCF_3$ | 5-Cl | H | $CH_3$ | $C_2H_5$ | $CH_3$ | |
| 2.373 | 2-$OCF_3$ | 5-Cl | H | $CH_3$ | $CF_2Cl$ | $CH_3$ | |
| 2.374 | 2-$C_2H_5$ | 6-$CH_3$ | H | $CH_3$ | Cl | $CH_3$ | |
| 2.375 | 2-$C_2H_5$ | 6-$CH_3$ | H | $CH_3$ | $CF_3$ | $CH_3$ | |
| 2.376 | 2-$C_2H_5$ | 6-$CH_3$ | H | $OCH_3$ | $CF_3$ | $CH_3$ | |
| 2.377 | 2-$C_2H_5$ | 6-$CH_3$ | H | $SCH_3$ | $CF_3$ | $CH_3$ | |
| 2.378 | 2-$C_2H_5$ | 6-$CH_3$ | H | $OC_3H_7$(i) | $CF_3$ | $CH_3$ | |
| 2.379 | 2-$CF_3$ | 6-$NO_2$ | H | $CH_3$ | Cl | $CH_3$ | |
| 2.380 | 2-$CF_3$ | 6-$NO_2$ | H | $CH_3$ | $CF_3$ | $CH_3$ | |
| 2.381 | 2-$NO_2$ | 6-$NO_2$ | H | $CH_3$ | $CF_3$ | $CH_3$ | |
| 2.382 | 2-$NO_2$ | 6-$NO_2$ | H | $OCH_3$ | $CF_3$ | $CH_3$ | |
| 2.383 | 2-Cl | 6-Cl | 3-$CH_3$ | $CH_3$ | Cl | $CH_3$ | |
| 2.384 | 2-Cl | 6-Cl | 3-$CH_3$ | $CH_3$ | $CF_3$ | $CH_3$ | |
| 2.385 | 2-Cl | 6-Cl | 3-$CH_3$ | $CH_3$ | $CF_3$ | $C_2H_5$ | |
| 2.386 | 2-Cl | 6-Cl | 3-Cl | $CH_3$ | Cl | $CH_3$ | |
| 2.387 | 2-Cl | 6-Cl | 3-Cl | $CH_3$ | $CF_3$ | $CH_3$ | |
| 2.388 | 2-Cl | 6-Cl | 3-Cl | $C_2H_5$ | $CF_3$ | $CH_3$ | |
| 2.389 | 2-$CH_3$ | 3-Cl | H | $CH_3$ | Cl | $CH_3$ | |
| 2.390 | 2-$CH_3$ | 3-Cl | H | $CH_3$ | $CF_3$ | $CH_3$ | |
| 2.391 | 2-$CH_3$ | 3-Cl | H | $C_2H_5$ | $CF_3$ | $CH_3$ | |
| 2.392 | 2-$CH_3$ | 5-Cl | H | $CH_3$ | Cl | $CH_3$ | |
| 2.393 | 2-$CH_3$ | 5-Cl | H | $CH_3$ | $CF_3$ | $CH_3$ | |
| 2.394 | 2-$CH_3$ | 5-Cl | H | $C_2H_5$ | $CF_3$ | $CH_3$ | |
| 2.395 | 2-$CH_3$ | 5-Cl | H | $C_2H_5$ | $CF_2Cl$ | $CH_3$ | |
| 2.396 | 2-$CH_3$ | 5-Cl | H | $OCH_3$ | $CF_3$ | $CH_3$ | |
| 2.397 | 2-$CH_3$ | 6-Cl | H | $CH_3$ | Cl | $CH_3$ | |
| 2.398 | 2-$CH_3$ | 6-Cl | H | $CH_3$ | $CF_3$ | $CH_3$ | |
| 2.399 | 2-$CH_3$ | 6-Cl | H | $C_2H_5$ | $CF_3$ | $CH_3$ | |
| 2.400 | 2-$CH_3$ | 6-Cl | H | $C_2H_5$ | $CHCl_2$ | $CH_3$ | |
| 2.401 | 2-$CH_3$ | 6-Cl | H | $C_2H_5$ | $CF_2Cl$ | $CH_3$ | |
| 2.402 | 2-$CH_3$ | 6-Cl | H | $OC_2H_5$ | $CF_3$ | $CH_3$ | |
| 2.403 | 2-$CH_3$ | 6-Cl | H | $SCH_3$ | $CF_3$ | $CH_3$ | |
| 2.404 | 2-$CH_3$ | 6-Cl | H | $OC_4H_9$(n) | $CF_3$ | $CH_3$ | |
| 2.405 | 2-$CH_3$ | 6-Cl | H | $CH_3$ | Cl | $SCH_3$ | |
| 2.406 | 2-$CH_3$ | 6-$CH_3$ | H | $CH_3$ | Cl | $CH_3$ | |
| 2.407 | 2-$CH_3$ | 6-$CH_3$ | H | $CH_3$ | $CF_3$ | $CH_3$ | |
| 2.408 | 2-$CH_3$ | 6-$CH_3$ | H | $C_2H_5$ | $CF_3$ | $CH_3$ | |
| 2.409 | 2-$CH_3$ | 6-$CH_3$ | H | Cyclopropyl | $CF_3$ | $CH_3$ | |
| 2.410 | 2-$CH_3$ | 6-$CH_3$ | H | $C_2H_5$ | $CF_2Cl$ | $CH_3$ | |
| 2.411 | 2-Cl | 3-$CH_3$ | H | $CH_3$ | Cl | $CH_3$ | |
| 2.412 | 2-Cl | 3-$CH_3$ | H | $CH_3$ | $CF_3$ | $CH_3$ | |
| 2.413 | 2-Cl | 3-$CH_3$ | H | $C_2H_5$ | $CF_3$ | $CH_3$ | |
| 2.414 | 2-Cl | 5-$CH_3$ | H | $CH_3$ | Cl | $CH_3$ | |
| 2.415 | 2-Cl | 5-$CH_3$ | H | $CH_3$ | $CF_3$ | $CH_3$ | |
| 2.416 | 2-Cl | 5-$CH_3$ | H | $C_2H_5$ | $CF_3$ | $CH_3$ | |
| 2.417 | 2-Cl | 5-$CH_3$ | H | $C_2H_5$ | $CF_2Cl$ | $CH_3$ | |

Tabelle 2 (Fortsetzung)

| Verb.No | $R^1$ | $R^2$ | $R^3$ | $R^4$ | $R^5$ | $R^6$ | Phys. Daten |
|---|---|---|---|---|---|---|---|
| 2.418 | 2-$NO_2$ | H | H | $CH_2Cl$ | Cl | $CH_3$ | |
| 2.419 | 2-$NO_2$ | H | H | $CH_2OCH_3$ | Cl | $CH_3$ | |
| 2.420 | 2-$NO_2$ | H | H | $CH_3$ | Cl | $SCH_3$ | |
| 2.421 | 2-$NO_2$ | H | H | $CH_3$ | Cl | $SC_2H_5$ | |
| 2.422 | 2-$NO_2$ | H | H | $CH_3$ | Cl | $OCH_3$ | |
| 2.423 | 2-$NO_2$ | H | H | $CH_3$ | Cl | $OC_2H_5$ | |
| 2.424 | 2-$NO_2$ | 6-$CH_3$ | H | $CH_2OCH_3$ | Cl | $CH_3$ | |
| 2.425 | 2-$NO_2$ | 6-$CH_3$ | H | $CH_3$ | Cl | $SCH_3$ | |
| 2.426 | 2-$NO_2$ | 6-$CH_3$ | H | $CH_3$ | Cl | $OC_2H_5$ | |
| 2.427 | 2-$NO_2$ | 6-$CH_3$ | H | $CH_3$ | Cl | $SC_2H_5$ | |
| 2.428 | 2-$NO_2$ | 6-$CH_3$ | H | $CH_3$ | Cl | $SC_3H_7$(i) | |
| 2.429 | 2-Cl | 5-Cl | H | $CH_3$ | Cl | $OCH_3$ | |
| 2.430 | 2-Cl | 5-Cl | H | $CH_3$ | Cl | $SCH_3$ | |
| 2.431 | 2-Cl | 5-Cl | H | $CH_3$ | Cl | $OC_2H_5$ | |
| 2.432 | 2-Cl | 6-Cl | H | $CH_2OCH_3$ | $CF_3$ | $CH_3$ | |
| 2.433 | 2-Cl | 6-Cl | H | $CH_2OCH_3$ | Cl | $CH_3$ | |
| 2.434 | 2-Cl | 6-Cl | H | $CH_3$ | Cl | $OCH_3$ | |
| 2.435 | 2-Cl | 6-Cl | H | $CH_3$ | Cl | $SCH_3$ | |
| 2.436 | 2-Cl | 6-Cl | H | $CH_3$ | Cl | $OC_2H_5$ | |
| 2.437 | 2-Cl | 6-Cl | H | $CH_3$ | Cl | $SC_2H_5$ | |
| 2.438 | 2-$NO_2$ | H | H | $CH_2COOCH_3$ | $CF_3$ | $CH_3$ | |
| 2.439 | 2-$NO_2$ | H | H | $CH_2\overset{O}{\overset{\|}{C}}CH_3$ | $CF_3$ | $CH_3$ | |
| 2.440 | 2-$OCH_3$ | 5-Cl | H | $CH_3$ | Cl | $CH_3$ | |
| 2.441 | 2-$OCH_3$ | 5-Cl | H | $CH_3$ | $CF_3$ | $CH_3$ | |
| 2.442 | 2-$OCH_3$ | 5-Cl | H | $C_2H_5$ | $CF_3$ | $CH_3$ | |
| 2.443 | 2-$OCH_3$ | 5-Cl | H | $CH_3$ | $CF_2Cl$ | $CH_3$ | |
| 2.444 | 2-$OCH_3$ | 5-Cl | H | $CH_3$ | $C_2F_5$ | $CH_3$ | |
| 2.445 | 2-$OCH_3$ | 5-Cl | H | Cyclopropyl | $CF_3$ | $CH_3$ | |

| Verb.No | $R^1$ | $R^2$ | $R^3$ | $-R^5 - R^6-$ | $R^4$ | Phys. Daten |
|---|---|---|---|---|---|---|
| 2.446 | 2-$NO_2$ | H | H | $-CH_2-CH_2-$ | $CF_3$ | |
| 2.447 | 2-$NO_2$ | H | H | $-CHCH_3-CH_2-$ | $CF_3$ | |
| 2.448 | 2-$NO_2$ | H | H | $-CH_2-CH_2-$ | $CF_2Cl$ | |
| 2.449 | 2-$NO_2$ | H | H | $-(CH_2)_3-$ | $CF_3$ | Fp. 148-150°C |
| 2.450 | 2-$NO_2$ | H | H | $-(CH_2)_3-$ | Cl | |
| 2.451 | 2-$NO_2$ | H | H | $-(CH_2)_3-$ | $OCH_3$ | |
| 2.452 | 2-$NO_2$ | H | H | $-(CH_2)_3-$ | $SCH_3$ | |
| 2.453 | 2-$NO_2$ | H | H | $-(CH_2)_3-$ | $SOCH_3$ | |
| 2.454 | 2-$NO_2$ | H | H | $-(CH_2)_3-$ | $SO_2CH_3$ | |
| 2.455 | 2-$NO_2$ | H | H | $-CHCH_3-CH_2-CH_2-$ | $CF_3$ | |
| 2.456 | 2-$NO_2$ | H | H | $-CHCH_3-CH_2-CH_2-$ | Cl | |
| 2.457 | 2-$NO_2$ | H | H | $-CHCH_3-CH_2-CH_2-$ | $CHCl_2$ | |
| 2.458 | 2-$NO_2$ | H | H | $-CHCH_3-CH_2-CH_2-$ | $CHFCl$ | |
| 2.459 | 2-$NO_2$ | H | H | $-CHCH_3-CH_2-CH_2-$ | $CF_2Cl$ | |
| 2.460 | 2-$NO_2$ | H | H | $-C(CH_3)_2-CH_2-CH_2-$ | $CF_3$ | |
| 2.461 | 2-$NO_2$ | H | H | $-C(CH_3)_2-CH_2CH_2$ | Cl | |

Tabelle 2 (Fortsetzung)

| Verb.No | $R^1$ | $R^2$ | $R^3$ | $-R^5-R^6-$ | $R^4$ | Phys. Daten |
|---|---|---|---|---|---|---|
| 2.462 | $2-NO_2$ | H | H | $-C(CH_3)_2-CH_2-CH_2$ | $CF_2CF_3$ | |
| 2.463 | $2-NO_2$ | H | H | $-CH=CCH_3-CH_2-$ | $CF_3$ | |
| 2.464 | $2-NO_2$ | H | H | $-CH=CCH_3-CH_2-$ | Cl | |
| 2.465 | $2-NO_2$ | H | H | $-CH=CCH_3-CH_2-$ | $CHF_2$ | |
| 2.466 | $2-NO_2$ | H | H | $-CH_2-CHCH_3-CH_2-$ | Cl | |
| 2.467 | $2-NO_2$ | H | H | $-CH_2-CHCH_3-CH_2-$ | $CF_3$ | |
| 2.468 | $2-NO_2$ | H | H | $-CH_2-CH_2-CHCH_3-$ | Cl | |
| 2.469 | $2-NO_2$ | H | H | $-CH_2-CH_2-CHCH_3-$ | $CF_3$ | |
| 2.470 | $2-NO_2$ | H | H | $-CH(COOCH_3)-CH_2-CH_2-$ | Cl | |
| 2.471 | $2-NO_2$ | H | H | $-CH(COOC_2H_5)-CH_2-CH_2-$ | Cl | |
| 2.472 | $2-NO_2$ | H | H | $-CH_2-CH_2-S-$ | Cl | |
| 2.473 | $2-NO_2$ | H | H | $-CH_2-CH_2-S-$ | $CF_3$ | |
| 2.474 | $2-NO_2$ | H | H | $-CH_2-CH_2-S-$ | $CF_2Cl$ | |
| 2.475 | $2-NO_2$ | H | H | $-CH_2-CH_2-S-$ | $CHCl_2$ | |
| 2.476 | $2-NO_2$ | H | H | $-CH_2-C(CH_3)_2-CH_2-$ | Cl | |
| 2.477 | $2-NO_2$ | H | H | $-CH_2-C(CH_3)_2-CH_2-$ | $CF_3$ | |
| 2.478 | $2-NO_2$ | H | H | $-CH_2-C(CH_3)_2-CH_2-$ | $CF_2CF_3$ | |
| 2.479 | $2-NO_2$ | H | H | $-CH_2-CH_2-C(CH_3)_2-$ | Cl | |
| 2.480 | $2-NO_2$ | H | H | $-CH_2-CH_2-C(CH_3)_2-$ | $CF_3$ | |
| 2.481 | $2-NO_2$ | H | H | $-CH_2-CH_2-C(CH_3)_2-$ | $CF_2CF_3$ | |
| 2.482 | $2-NO_2$ | H | H | $-CHCH_3-CHCH_3-CH_2-$ | Cl | |
| 2.483 | $2-NO_2$ | H | H | $-CHCH_3-CHCH_3-CH_3-$ | $CF_3$ | |
| 2.484 | $2-NO_2$ | H | H | $-CHCH_3-CHCH_3-CH_2-$ | $CF_2Cl$ | |
| 2.485 | $2-NO_2$ | H | H | $-CH(C_2H_5)-CH_2-CH_2-$ | Cl | |
| 2.486 | $2-NO_2$ | H | H | $-CH(C_2H_5)-CH_2-CH_2-$ | $CF_3$ | |
| 2.487 | $2-NO_2$ | H | H | $-C(CH_3)(C_2H_5)-CH_2-CH_2$ | Cl | |
| 2.488 | $2-NO_2$ | H | H | $-C(CH_3)(C_2H_5)-CH_2CH_2$ | $CF_3$ | |
| 2.489 | $2-NO_2$ | H | H | $-CHCH_3-(CH_2)_3-$ | Cl | |
| 2.490 | $2-NO_2$ | H | H | $-CHCH_3-(CH_2)_3-$ | $CF_3$ | Fp. 88-89°C |
| 2.491 | $2-NO_2$ | H | H | $-CHCH_3-(CH_2)_3-$ | $CF_2CF_3$ | |
| 2.492 | $2-NO_2$ | H | H | $-CHCH_3-(CH_2)_3-$ | $CF_2Cl$ | |
| 2.493 | $2-NO_2$ | H | H | $-CHCH_3-(CH_2)_3-$ | $CHF_2$ | |
| 2.494 | $2-NO_2$ | H | H | $-(CH_2)_4-$ | Cl | Fp. 153-154°C |
| 2.495 | $2-NO_2$ | H | H | $-(CH_2)_4-$ | $OCH_3$ | Fp. 109-112°C |
| 2.496 | $2-NO_2$ | H | H | $-(CH_2)_4-$ | $CF_3$ | Fp. 129-130°C |
| 2.497 | $2-NO_2$ | H | H | $-(CH_2)_4-$ | $CHF_2$ | Fp. 127-128°C |
| 2.498 | $2-NO_2$ | H | H | $-(CH_2)_4-$ | $CHCl_2$ | |
| 2.499 | $2-NO_2$ | H | H | $-(CH_2)_4-$ | CHFCl | |
| 2.500 | $2-NO_2$ | H | H | $-(CH_2)_4-$ | $CF_2CF_3$ | |
| 2.501 | $2-NO_2$ | H | H | $-(CH_2)_4-$ | $C_3F_7(n)$ | |
| 2.502 | $2-NO_2$ | H | H | $-(CH_2)_4-$ | $CCl_2CF_3$ | |
| 2.503 | $2-NO_2$ | H | H | $-(CH_2)_4-$ | $SCH_3$ | |
| 2.504 | $2-NO_2$ | H | H | $-(CH_2)_4-$ | $SOCH_3$ | |
| 2.505 | $2-NO_2$ | H | H | $-(CH_2)_4-$ | $SO_2CH_3$ | |
| 2.506 | $2-NO_2$ | H | H | $-C(CH_3)_2-(CH_2)_3-$ | Cl | |
| 2.507 | $2-NO_2$ | H | H | $-C(CH_3)_2-(CH_2)_3-$ | $CF_3$ | |
| 2.508 | $2-NO_2$ | H | H | $-C(CH_3)_2-(CH_2)_3-$ | $CF_2Cl$ | |
| 2.509 | $2-NO_2$ | H | H | $-C(CH_3)_2-(CH_2)_3-$ | $CHF_2$ | |

Tabelle 2 (Fortsetzung)

| Verb.No | $R^1$ | $R^2$ | $R^3$ | $-R^5-R^6-$ | $R^4$ | Phys. Daten |
|---|---|---|---|---|---|---|
| 2.510 | $2-NO_2$ | H | H | $-C(CH_3)_2-(CH_2)_3-$ | $CF_2CF_3$ | |
| 2.511 | $2-NO_2$ | H | H | $-CH_2-CHCH_3-(CH_2)_2-$ | Cl | |
| 2.512 | $2-NO_2$ | H | H | $-CH_2-CHCH_3-(CH_2)_2-$ | $CF_3$ | Fp. 119-120°C |
| 2.513 | $2-NO_2$ | H | H | $-(CH_2)_3-CHCH_3-$ | Cl | |
| 2.514 | $2-NO_2$ | H | H | $-(CH_2)_3-CHCH_3-$ | $CF_3$ | |
| 2.515 | $2-NO_2$ | H | H | $-(CH_2)_2-CHCH_3-CH_2-$ | Cl | |
| 2.516 | $2-NO_2$ | H | H | $-(CH_2)_2-CHCH_3-CH_2-$ | $OCH_3$ | |
| 2.517 | $2-NO_2$ | H | H | $-(CH_2)_2-CHCH_3-CH_2-$ | $CF_3$ | Fp. 184-185°C |
| 2.518 | $2-NO_2$ | H | H | $-(CH_2)_2-CHCH_3-CH_2-$ | $CF_2Cl$ | |
| 2.519 | $2-NO_2$ | H | H | $-(CH_2)_2-CHCH_3-CH_2-$ | $CHCl_2$ | |
| 2.520 | $2-NO_2$ | H | H | $-(CH_2)_2-C(CH_3)_2-CH_2-$ | Cl | |
| 2.521 | $2-NO_2$ | H | H | $-(CH_2)_2-C(CH_3)_2-CH_3-$ | $CF_3$ | |
| 2.522 | $2-NO_2$ | H | H | $-CH=CCH_3-CH_2-C(CH_3)_2-$ | Cl | |
| 2.523 | $2-NO_2$ | H | H | $-CH=CCH_3-CH_2-C(CH_3)_2-$ | $CF_3$ | |
| 2.524 | $2-NO_2$ | H | H | $-CH_2-CH(OCH_3)-(CH_2)_2$ | Cl | |
| 2.525 | $2-NO_2$ | H | H | $-CH_2-CH(OCH_3)-(CH_2)_2$ | $CF_3$ | |
| 2.526 | $2-NO_2$ | H | H | $-(CH_2)_2-S-CH_2-$ | Cl | |
| 2.527 | $2-NO_2$ | H | H | $-(CH_2)_2-S-CH_2-$ | $CF_3$ | |
| 2.528 | $2-NO_2$ | H | H | $-(CH_2)_2-O-CH_2-$ | Cl | |
| 2.529 | $2-NO_2$ | H | H | $-(CH_2)_2-NCH_3-CH_2-$ | $CF_3$ | Fp. 194-195°C |
| 2.530 | $2-NO_2$ | H | H | $-O-(CH_2)_2-$ | $CF_3$ | |
| 2.531 | $2-NO_2$ | H | H | $-O-(CH_2)_3-$ | $CF_3$ | |
| 2.532 | $2-NO_2$ | H | H | $-CH(COOC_2H_5)-(CH_2)_3$ | Cl | |
| 2.533 | $2-NO_2$ | H | H | $-CH(COOC_2H_5)-(CH_2)_3$ | $CF_3$ | |
| 2.534 | $2-NO_2$ | H | H | $-CHCH_3-CH_2-C(CH_3)_2-CH_2-$ | Cl | |
| 2.535 | $2-NO_2$ | H | H | $-CHCH_3-CH_2-C(CH_3)_2-CH_2-$ | $CF_3$ | |
| 2.536 | $2-NO_2$ | H | H | $-CHCH_3-CH_2-C(CH_3)_2-CH_2-$ | $CF_2CF_3$ | |
| 2.537 | $2-NO_2$ | H | H | $-(CH_2)_5-$ | Cl | |
| 2.538 | $2-NO_2$ | H | H | $-(CH_2)_5-$ | $CF_3$ | Fp. 148-149°C |
| 2.539 | $2-NO_2$ | H | H | $-CHCH_3-(CH_2)_4-$ | Cl | |
| 2.540 | $2-NO_2$ | H | H | $-CHCH_3-(CH_2)_4-$ | $CF_3$ | |
| 2.541 | $2-NO_2$ | H | H | $-C(CH_3)_2-(CH_2)_4-$ | Cl | |
| 2.542 | $2-NO_2$ | H | H | $-C(CH_3)_2-(CH_2)_4-$ | $CF_3$ | |
| 2.543 | $2-NO_2$ | H | H | $-(CH_2)_6-$ | Cl | |
| 2.544 | $2-NO_2$ | H | H | $-(CH_2)_6-$ | $CF_3$ | Fp. 150-151°C |
| 2.545 | $2-NO_2$ | $6-CH_3$ | H | $-(CH_2)_3-$ | $CF_3$ | Fp. 135-136°C |
| 2.546 | $2-NO_2$ | $6-CH_3$ | H | $-(CH_2)_3-$ | Cl | |
| 2.547 | $2-NO_2$ | $6-CH_3$ | H | $-(CH_2)_3-$ | $OCH_3$ | |
| 2.548 | $2-NO_2$ | $6-CH_3$ | H | $-(CH_2)_3-$ | $CF_2Cl$ | Fp. 116-118°C |
| 2.549 | $2-NO_2$ | $6-CH_3$ | H | $-(CH_2)_3-$ | $CF_2CF_3$ | |
| 2.550 | $2-NO_2$ | $6-CH_3$ | H | $-(CH_2)_3-$ | $CHCl_2$ | |
| 2.551 | $2-NO_2$ | $6-CH_3$ | H | $-CHCH_3-CH_2-CH_2-$ | Cl | |
| 2.552 | $2-NO_2$ | $6-CH_3$ | H | $-CHCH_3-CH_2-CH_2$ | $CF_3$ | |
| 2.553 | $2-NO_2$ | $6-CH_3$ | H | $-CHCH_3-CH_2-CH_2$ | $CF_2Cl$ | |
| 2.554 | $2-NO_2$ | $6-CH_3$ | H | $-C(CH_3)_2-CH_2-CH_2-$ | Cl | |
| 2.555 | $2-NO_2$ | $6-CH_3$ | H | $-C(CH_3)_2-CH_2-CH_2-$ | $CF_3$ | |
| 2.556 | $2-NO_2$ | $6-CH_3$ | H | $-C(CH_3)_2-CH_2-CH_2-$ | $CF_2CF_3$ | |
| 2.557 | $2-NO_2$ | $6-CH_3$ | H | $-C(CH_3)_2-CH_2-CH_2-$ | $CF_2Cl$ | |

Tabelle 2 (Fortsetzung)

| Verb.No | $R^1$ | $R^2$ | $R^3$ | $-R^5-R^6-$ | $R^4$ | Phys. Daten |
|---|---|---|---|---|---|---|
| 2.558 | 2-$NO_2$ | 6-$CH_3$ | H | $-C(CH_3)_2-CH_2-CH_2-$ | $CHCl_2$ | |
| 2.559 | 2-$NO_2$ | 6-$CH_3$ | H | $-CH_2-CH_2-S-$ | Cl | |
| 2.560 | 2-$NO_2$ | 6-$CH_3$ | H | $-CH_2-CH_2-S-$ | $CF_3$ | |
| 2.561 | 2-$NO_2$ | 6-$CH_3$ | H | $-(CH_2)_4-$ | Cl | |
| 2.562 | 2-$NO_2$ | 6-$CH_3$ | H | $-(CH_2)_4-$ | $OCH_3$ | |
| 2.563 | 2-$NO_2$ | 6-$CH_3$ | H | $-(CH_2)_4-$ | $CF_3$ | Fp. 103-104°C |
| 2.564 | 2-$NO_2$ | 6-$CH_3$ | H | $-(CH_2)_4-$ | $CF_2Cl$ | Fp. 85-86°C |
| 2.565 | 2-$NO_2$ | 6-$CH_3$ | H | $-(CH_2)_4-$ | $CHF_2$ | Fp. 124-126°C |
| 2.566 | 2-$NO_2$ | 6-$CH_3$ | H | $-(CH_2)_4-$ | CHFCl | |
| 2.567 | 2-$NO_2$ | 6-$CH_3$ | H | $-(CH_2)_4-$ | $CHCl_2$ | |
| 2.568 | 2-$NO_2$ | 6-$CH_3$ | H | $-(CH_2)_4-$ | $CF_2CF_3$ | |
| 2.569 | 2-$NO_2$ | 6-$CH_3$ | H | $-(CH_2)_4-$ | $C_3F_7(n)$ | |
| 2.570 | 2-$NO_2$ | 6-$CH_3$ | H | $-(CH_2)_4-$ | $CCl_2CF_3$ | |
| 2.571 | 2-$NO_2$ | 6-$CH_3$ | H | $-(CH_2)_4-$ | $SCH_3$ | |
| 2.572 | 2-$NO_2$ | 6-$CH_3$ | H | $-(CH_2)_4$ | $SOCH_3$ | |
| 2.573 | 2-$NO_2$ | 6-$CH_3$ | H | $-(CH_2)_4$ | $SO_2CH_3$ | |
| 2.574 | 2-$NO_2$ | 6-$CH_3$ | H | $-C(CH_3)_2-(CH_2)_3-$ | Cl | |
| 2.575 | 2-$NO_2$ | 6-$CH_3$ | H | $-C(CH_3)_2-(CH_2)_3-$ | $CF_3$ | |
| 2.576 | 2-$NO_2$ | 6-$CH_3$ | H | $-CH_2-CHCH_3-(CH_2)_2-$ | Cl | |
| 2.577 | 2-$NO_2$ | 6-$CH_3$ | H | $-CH_2-CHCH_3-(CH_2)_2-$ | $CF_3$ | Fp. 154-155°C |
| 2.578 | 2-$NO_2$ | 6-$CH_3$ | H | $-CH_2-CHCH_3-(CH_2)_2-$ | $CF_2Cl$ | |
| 2.579 | 2-$NO_2$ | 6-$CH_3$ | H | $-CH_2-CHCH_3-(CH_2)_2-$ | $CHCl_2$ | |
| 2.580 | 2-$NO_2$ | 6-$CH_3$ | H | $-(CH_2)_2-NCH_3-CH_2-$ | $CF_3$ | |
| 2.581 | 2-$NO_2$ | 6-$CH_3$ | H | $-(CH_2)_5-$ | Cl | |
| 2.582 | 2-$NO_2$ | 6-$CH_3$ | H | $-(CH_2)_5-$ | $CF_3$ | Oel |
| 2.583 | 2-$NO_2$ | 6-$CH_3$ | H | $-CH_2-CH_2-CH_2-O-$ | Cl | |
| 2.584 | 2-$NO_2$ | 6-$CH_3$ | H | $-CH_2-CH_2-O-$ | $CF_3$ | |
| 2.585 | 2-Cl | H | H | $-(CH_2)_3-$ | $CF_3$ | |
| 2.586 | 2-Cl | H | H | $-(CH_2)_4-$ | $CF_3$ | |
| 2.587 | 2-Cl | 5-Cl | H | $-(CH_2)_3-$ | Cl | |
| 2.588 | 2-Cl | 5-Cl | H | $-(CH_2)_3-$ | $CF_3$ | Fp. 127-128°C |
| 2.589 | 2-Cl | 5-Cl | H | $-(CH_2)_3-$ | $OCH_3$ | |
| 2.590 | 2-Cl | 5-Cl | H | $-(CH_2)_3-$ | $CF_2Cl$ | |
| 2.591 | 2-Cl | 5-Cl | H | $-(CH_2)_3-$ | $CF_2CF_3$ | |
| 2.592 | 2-Cl | 5-Cl | H | $-(CH_2)_3-$ | $CHCl_2$ | |
| 2.593 | 2-Cl | 5-Cl | H | $-C(CH_3)_2-CH_2-CH_2-$ | Cl | |
| 2.594 | 2-Cl | 5-Cl | H | $-C(CH_3)_2-CH_2-CH_2-$ | $OCH_3$ | |
| 2.595 | 2-Cl | 5-Cl | H | $-C(CH_3)_2-CH_2-CH_2-$ | $CF_3$ | |
| 2.596 | 2-Cl | 5-Cl | H | $-C(CH_3)_2-CH_2-CH_2-$ | $CF_2Cl$ | |
| 2.597 | 2-Cl | 5-Cl | H | $-C(CH_3)_2-CH_2-CH_2-$ | $CHCl_2$ | |
| 2.598 | 2-Cl | 5-Cl | H | $-C(CH_3)_2-CH_2-CH_2-$ | $CF_2CF_3$ | |
| 2.599 | 2-Cl | 5-Cl | H | $-CH_2-CH_2-S-$ | Cl | |
| 2.600 | 2-Cl | 5-Cl | H | $-CH_2-CH_2-S-$ | $CF_3$ | |
| 2.601 | 2-Cl | 5-Cl | H | $-(CH_2)_4-$ | Cl | Fp. 119-120°C |
| 2.602 | 2-Cl | 5-Cl | H | $-(CH_2)_4-$ | $OCH_3$ | Fp. 101-103°C |
| 2.603 | 2-Cl | 5-Cl | H | $-(CH_2)_4-$ | $SCH_3$ | |
| 2.604 | 2-Cl | 5-Cl | H | $-(CH_2)_4-$ | $SOCH_3$ | |
| 2.605 | 2-Cl | 5-Cl | H | $-(CH_2)_4-$ | $SO_2CH_3$ | |

Tabelle 2 (Fortsetzung)

| Verb.No | $R^1$ | $R^2$ | $R^3$ | $-R^5-R^6-$ | $R^4$ | Phys. Daten |
|---|---|---|---|---|---|---|
| 2.606 | 2-Cl | 5-Cl | H | $-(CH_2)_4-$ | $CF_3$ | Fp. 123-124°C |
| 2.607 | 2-Cl | 5-Cl | H | $-(CH_2)_4-$ | $CF_2Cl$ | |
| 2.608 | 2-Cl | 5-Cl | H | $-(CH_2)_4-$ | $CHF_2$ | |
| 2.609 | 2-Cl | 5-Cl | H | $-(CH_2)_4-$ | $CHCl_2$ | |
| 2.610 | 2-Cl | 5-Cl | H | $-(CH_2)_4-$ | CHFCl | |
| 2.611 | 2-Cl | 5-Cl | H | $-(CH_2)_4-$ | $CF_2CF_3$ | |
| 2.612 | 2-Cl | 5-Cl | H | $-(CH_2)_4-$ | $C_3F_7n$ | |
| 2.613 | 2-Cl | 5-Cl | H | $-C(CH_3)_2-(CH_2)_3-$ | Cl | |
| 2.614 | 2-Cl | 5-Cl | H | $-C(CH_3)_2-(CH_2)_3-$ | $OCH_3$ | |
| 2.615 | 2-Cl | 5-Cl | H | $-C(CH_3)_2-(CH_2)_3-$ | $CF_3$ | |
| 2.616 | 2-Cl | 5-Cl | H | $-C(CH_3)_2-(CH_2)_3-$ | $CF_2Cl$ | |
| 2.617 | 2-Cl | 5-Cl | H | $-(CH_2)_5-$ | Cl | |
| 2.618 | 2-Cl | 5-Cl | H | $-(CH_2)_5-$ | $OCH_3$ | |
| 2.619 | 2-Cl | 5-Cl | H | $-(CH_2)_5-$ | $CF_3$ | Fp. 104-105°C |
| 2.620 | 2-Cl | 5-Cl | H | $-(CH_2)_5-$ | $CF_2Cl$ | |
| 2.621 | 2-Cl | 5-Cl | H | $-(CH_2)_6-$ | Cl | |
| 2.622 | 2-Cl | 5-Cl | H | $-(CH_2)_6-$ | $OCH_3$ | |
| 2.623 | 2-Cl | 5-Cl | H | $-(CH_2)_6-$ | $CF_3$ | Fp. 91-92°C |
| 2.624 | 2-Cl | 5-Cl | H | $-(CH_2)_6-$ | $CF_2Cl$ | |
| 2.625 | 2-Cl | 6-Cl | H | $-(CH_2)_3-$ | Cl | |
| 2.626 | 2-Cl | 6-Cl | H | $-(CH_2)_3-$ | $OCH_3$ | |
| 2.627 | 2-Cl | 6-Cl | H | $-(CH_3)_2-$ | $CF_3$ | Fp. 169-170°C |
| 2.628 | 2-Cl | 6-Cl | H | $-(CH_2)_3-$ | $CF_2Cl$ | |
| 2.629 | 2-Cl | 6-Cl | H | $-(CH_2)_3-$ | $CHF_2$ | Fp. 145-146°C |
| 2.630 | 2-Cl | 6-Cl | H | $-(CH_2)_3-$ | $CF_2CF_3$ | |
| 2.631 | 2-Cl | 6-Cl | H | $-(CH_2)_4-$ | Cl | |
| 2.632 | 2-Cl | 6-Cl | H | $-(CH_2)_4-$ | $OCH_3$ | |
| 2.633 | 2-Cl | 6-Cl | H | $-(CH_2)_4-$ | $CF_3$ | Fp. 165-166°C |
| 2.634 | 2-Cl | 6-Cl | H | $-(CH_2)_4-$ | $CF_2Cl$ | |
| 2.635 | 2-Cl | 6-Cl | H | $-(CH_2)_4-$ | $CHCl_2$ | |
| 2.636 | 2-Cl | 6-Cl | H | $-(CH_2)_4-$ | $CHF_2$ | |
| 2.637 | 2-Cl | 6-Cl | H | $-CH_2-CH_2-CHCH_3-CH_2-$ | $CF_3$ | |
| 2.638 | 2-Cl | 6-Cl | H | $-CH_2-CH_2-CHCH_3-CH_2-$ | $CF_2Cl$ | |
| 2.639 | 2-Cl | 6-Cl | H | $-CH_2-CH_2-CHCH_3-CH_2-$ | $CHF_3$ | |
| 2.640 | 2-Cl | 6-Cl | H | $-(CH_2)_5-$ | $CF_3$ | |
| 2.641 | 2-Cl | $6-CH_3$ | H | $-(CH_2)_3-$ | Cl | |
| 2.642 | 2-Cl | $6-CH_3$ | H | $-(CH_2)_3-$ | $CF_3$ | Fp. 138-141°C |
| 2.643 | 2-Cl | $6-CH_3$ | H | $-(CH_2)_3-$ | $CF_2Cl$ | Fp. 135-137°C |
| 2.644 | 2-Cl | $6-CH_3$ | H | $-(CH_2)_3-$ | $CHF_2$ | Fp. 129-130°C |
| 2.645 | 2-Cl | $6-CH_3$ | H | $-(CH_2)_4-$ | Cl | |
| 2.646 | 2-Cl | $6-CH_3$ | H | $-(CH_2)_4-$ | $CF_3$ | Fp. 131-132°C |
| 2.647 | 2-Cl | $6-CH_3$ | H | $-(CH_2)_4-$ | $CF_2Cl$ | Fp. 101-103°C |
| 2.648 | 2-Cl | $6-CH_3$ | H | $-(CH_2)_4-$ | $CHF_2$ | Fp. 134-136°C |
| 2.649 | 2-Cl | $6-CH_3$ | H | $-(CH_2)_5-$ | $CF_3$ | |
| 2.650 | 2-Cl | $6-CH_3$ | H | $-(CH_2)_6-$ | $CF_3$ | |
| 2.651 | 2-Br | H | H | $-(CH_2)_3-$ | Cl | |
| 2.652 | 2-Br | H | H | $-(CH_2)_3-$ | $CF_3$ | |
| 2.653 | 2-Br | H | H | $-(CH_2)_4-$ | Cl | |

Tabelle 2 (Fortsetzung)

| Verb.No | $R^1$ | $R^2$ | $R^3$ | $-R^5-R^6-$ | $R^4$ | Phys. Daten |
|---|---|---|---|---|---|---|
| 2.654 | 2-Br | H | H | $-(CH_2)_4-$ | $CF_3$ | |
| 2.655 | 2-Br | H | H | $-(CH_2)_4-$ | $CF_2Cl$ | |
| 2.656 | $2-CF_3$ | H | H | $-(CH_2)_3-$ | Cl | |
| 2.657 | $2-CF_3$ | H | H | $-(CH_2)_3-$ | $CF_3$ | |
| 2.658 | $2-CF_3$ | H | H | $-(CH_2)_3-$ | $CF_2Cl$ | |
| 2.659 | $2-CF_3$ | 6-Cl | H | $-(CH_2)_3-$ | $CF_3$ | |
| 2.660 | $2-CF_3$ | 6-Cl | H | $-(CH_2)_4-$ | $CF_3$ | |
| 2.661 | $2-OCH_3$ | H | H | $-(CH_2)_3-$ | Cl | |
| 2.662 | $2-OCH_3$ | H | H | $-(CH_2)_3-$ | $CF_3$ | |
| 2.663 | $2-OCH_3$ | H | H | $-(CH_2)_4-$ | Cl | |
| 2.664 | $2-OCH_3$ | H | H | $-(CH_2)_4-$ | $CF_3$ | |
| 2.665 | $2-OCH_3$ | 5-Cl | H | $-(CH_2)_3-$ | Cl | |
| 2.666 | $2-OCH_3$ | 5-Cl | H | $-(CH_2)_3-$ | $CF_3$ | |
| 2.667 | $2-OCH_3$ | 5-Cl | H | $-(CH_2)_4-$ | Cl | |
| 2.668 | $2-OCH_3$ | 5-Cl | H | $-(CH_2)_4-$ | $CF_3$ | |
| 2.669 | $2-OCHF_2$ | H | H | $-(CH_2)_3-$ | Cl | |
| 2.670 | $2-OCHF_2$ | H | H | $-(CH_2)_3-$ | $CF_3$ | |
| 2.671 | $2-OCHF_2$ | H | H | $-(CH_2)_4-$ | Cl | |
| 2.672 | $2-OCHF_2$ | H | H | $-(CH_2)_4-$ | $CF_3$ | Fp. 114-115°C |
| 2.673 | $2-OCHF_2$ | 5-Cl | H | $-(CH_2)_3-$ | Cl | |
| 2.674 | $2-OCHF_2$ | 5-Cl | H | $-(CH_2)_3-$ | $CF_3$ | |
| 2.675 | $2-OCHF_2$ | 5-Cl | H | $-(CH_2)_4-$ | Cl | |
| 2.676 | $2-OCHF_2$ | 5-Cl | H | $-(CH_2)_4-$ | $CF_3$ | |
| 2.677 | $2-OCF_3$ | H | H | $-(CH_2)_3-$ | Cl | |
| 2.678 | $2-OCF_3$ | H | H | $-(CH_2)_3-$ | $CF_3$ | |
| 2.679 | $2-OCF_3$ | H | H | $-(CH_2)_4-$ | Cl | |
| 2.680 | $2-OCF_3$ | H | H | $-(CH_2)_4-$ | $CF_3$ | |
| 2.681 | $2-OCF_3$ | 5-Cl | H | $-(CH_2)_3-$ | Cl | |
| 2.682 | $2-OCF_3$ | 5-Cl | H | $-(CH_2)_3-$ | $CF_3$ | |
| 2.683 | $2-OCF_3$ | 5-Cl | H | $-(CH_2)_4-$ | $CF_3$ | |
| 2.684 | $2-CH_3$ | 5-Cl | H | $-(CH_2)_3-$ | Cl | |
| 2.685 | $2-CH_3$ | 5-Cl | H | $-(CH_2)_3-$ | $CF_3$ | |
| 2.686 | $2-CH_3$ | 5-Cl | H | $-(CH_2)_4-$ | Cl | |
| 2.687 | $2-CH_3$ | 5-Cl | H | $-(CH_2)_4-$ | $CF_3$ | |
| 2.688 | 2-CN | H | H | $-(CH_2)_3-$ | $CF_3$ | |
| 2.689 | 2-CN | H | H | $-(CH_2)_4-$ | $CF_3$ | |
| 2.690 | $2-COOCH_3$ | H | H | $-(CH_2)_3-$ | $CF_3$ | |
| 2.691 | $2-COOCH_3$ | H | H | $-(CH_2)_4-$ | $CF_3$ | |
| 2.692 | $2-CH_3$ | $6-CH_3$ | H | $-(CH_2)_4-$ | Cl | |
| 2.693 | $2-CH_3$ | $6-CH_3$ | H | $-(CH_2)_4-$ | $CF_3$ | |
| 2.694 | $2-CH_3$ | $6-CH_3$ | H | $-(CH_2)_3-$ | $CF_3$ | Fp. 119°C |
| 2.695 | $2-CH_3$ | $6-C_2H_5$ | H | $-(CH_2)_3-$ | $CF_3$ | |
| 2.696 | $2-CH_3$ | $6-C_2H_5$ | H | $-(CH_2)_4-$ | $CF_3$ | |
| 2.697 | $2-NO_2$ | H | H | $-(CH_2)_3-$ | $CH_3$ | |
| 2.698 | $2-NO_2$ | H | H | $-(CH_2)_4-$ | $CH_3$ | |
| 2.699 | $2-NO_2$ | $6-CH_3$ | H | $-(CH_2)_3-$ | $CH_3$ | |
| 2.700 | $2-NO_2$ | $6-CH_3$ | H | $-(CH_2)_4-$ | $CH_3$ | |

Tabelle 2 (Fortsetzung)

| Verb.No | $R^1$ | $R^2$ | $R^3$ | $R^4$ | $R^5$ | $R^6$ | Phys. Daten |
|---|---|---|---|---|---|---|---|
| 2.701 | 2-$SCH_3$ | H | H | $CH_3$ | $CF_3$ | $CH_3$ | |
| 2.702 | 2-$SOCH_3$ | H | H | $CH_3$ | $CF_3$ | $CH_3$ | |
| 2.703 | 2-$SCHF_2$ | H | H | $CH_3$ | $CF_3$ | $CH_3$ | |
| 2.704 | 2-$SOCHF_2$ | H | H | $CH_3$ | $CF_3$ | $CH_3$ | |
| 2.705 | 2-$CHF_2$ | H | H | $C_2H_5$ | $CF_3$ | $CH_3$ | |
| 2.706 | 2-$CH_2F$ | H | H | $CH_3$ | $CF_3$ | $CH_3$ | |
| 2.707 | 2-$CF_2CH_3$ | H | H | $CH_3$ | $CF_3$ | $CH_3$ | |
| 2.708 | 2-$CH_2CH_2CF_3$ | H | H | $CH_3$ | $CF_3$ | $CH_3$ | |
| 2.709 | 2-$CHClCHClCF_3$ | H | H | $CH_3$ | $CF_3$ | $CH_3$ | |
| 2.710 | 2-$OCF_2CHF_2$ | H | H | $CH_3$ | $CF_3$ | $CH_3$ | |
| 2.711 | 2-$OCF_2CHF_2$ | 5-Cl | H | $CH_3$ | $CF_3$ | $CH_3$ | |
| 2.712 | 2-$SCH_3$ | 5-Cl | H | $CH_3$ | $CF_3$ | $CH_3$ | |
| 2.713 | 2-$SO_2CH_3$ | 5-Cl | H | $CH_3$ | $CF_3$ | $CH_3$ | |
| 2.714 | 2-$SCHF_2$ | 5-Cl | H | $CH_3$ | $CF_3$ | $CH_3$ | |
| 2.715 | 2-$SO_2CHF_2$ | 5-Cl | H | $CH_3$ | $CF_3$ | $CH_3$ | |
| 2.716 | 2-$CF_2CF_3$ | H | H | $C_2H_5$ | $CF_3$ | $CH_3$ | |
| 2.717 | 2-$CF_2CF_3$ | H | H | $CH_3$ | $CF_3$ | Cl | |
| 2.718 | 2-$OCH_2CH_2Cl$ | H | H | $CH_3$ | $CF_3$ | $CH_3$ | |
| 2.719 | 2-$OCH_2CH_2Cl$ | 5-Cl | H | $C_2H_5$ | $CF_3$ | $CH_3$ | |
| 2.720 | 2-$COOCH_3$ | 6-Cl | H | $CH_3$ | $CF_3$ | $CH_3$ | |
| 2.721 | 2-$COOCH_3$ | 6-Cl | H | $C_2H_5$ | $CF_3$ | $CH_3$ | |
| 2.722 | 2-$COOC_2H_5$ | 6-Cl | H | $CH_3$ | $CF_3$ | $SCH_3$ | |
| 2.723 | 2-$COOCH_3$ | 6-Cl | H | $CH_3$ | Cl | $SCH_3$ | |
| 2.724 | 2-$COOCH_3$ | 6-Cl | H | $CH_3$ | Br | $SCH_3$ | |
| 2.725 | 2-$COOCH_3$ | 6-$CH_3$ | H | $CH_3$ | $CF_3$ | $CH_3$ | |
| 2.726 | 2-$COOCH_3$ | 6-$CH_3$ | H | $CF_3$ | Cl | $CH_3$ | |
| 2.727 | 2-$COOC_3H_7$(n) | 6-$CH_3$ | H | $CH_3$ | $CF_3$ | $CH_3$ | |
| 2.728 | 2-$NO_2$ | H | H | $CH_3$ | Br | Cl | |
| 2.729 | 2-$NO_2$ | 6-$CH_3$ | H | $CF_3$ | F | $CH_3$ | |
| 2.730 | 2-$NO_2$ | 6-$CH_3$ | H | $CF_3$ | Br | $CH_3$ | |
| 2.731 | 2-$NO_2$ | 6-$CH_3$ | H | $CH_3$ | F | $CH_3$ | |
| 2.732 | 2-$NO_2$ | 6-$CH_3$ | H | $CH_3$ | Br | $CH_3$ | |
| 2.733 | 2-$NO_2$ | 6-$CH_3$ | H | $CH_3$ | $CF_3$ | Cyclopentyl | |
| 2.734 | 2-$NO_2$ | 6-$CH_3$ | H | $CH_3$ | $CF_3$ | Cyclopropyl | |
| 2.735 | 2-$NO_2$ | H | H | $CH_3$ | $CF_3$ | Cyclohexyl | |
| 2.736 | 2-$NO_2$ | H | H | $C_2H_5$ | $CF_3$ | Cyclopentyl | |
| 2.737 | 2-Cl | 5-Cl | H | $CH_3$ | $CF_3$ | Cyclopentyl | |
| 2.738 | 2-$NO_2$ | H | H | Cyclopentyl | $CF_3$ | $CH_3$ | |
| 2.739 | 2-$NO_2$ | 6-$CH_3$ | H | Cyclopentyl | $CF_3$ | $CH_3$ | |

Tabelle 2 (Fortsetzung)

| Verb.No | $R^1$ | $R^2$ | $R^3$ | $R^4$ | $R^5$ | $R^6$ | Phys. Daten |
|---|---|---|---|---|---|---|---|
| 2.740 | 2-Cl | 5-Cl | H | Cyclopentyl | $CF_3$ | $CH_3$ | |
| 2.741 | 2-Cl | 5-Cl | H | Cyclohexyl | $CF_3$ | $CH_3$ | |
| 2.742 | 2-Cl | 5-Cl | H | Cyclohexyl | Cl | $CH_3$ | |
| 2.743 | $2-NO_2$ | H | H | Cyclohexyl | $CF_3$ | $CH_3$ | |
| 2.744 | $2-NO_2$ | $6-CH_3$ | H | Cyclohexyl | $CF_3$ | $CH_3$ | |
| 2.745 | $2-NO_2$ | H | H | $SCHF_2$ | $CF_3$ | $CH_3$ | |
| 2.746 | $2-NO_2$ | H | H | $SCF_3$ | $CF_3$ | $CH_3$ | |
| 2.747 | $2-NO_2$ | $6-CH_3$ | H | $SCHF_2$ | $CF_3$ | $CH_3$ | |
| 2.748 | $2-NO_2$ | $6-CH_3$ | H | SCHFCl | $CF_3$ | $CH_3$ | |
| 2.749 | 2-Cl | 5-Cl | H | $SCHF_2$ | $CF_3$ | $CH_3$ | |
| 2.750 | 2-Cl | $6-CH_3$ | H | $SCHF_2$ | $CF_3$ | $CH_3$ | |
| 2.751 | $2-NO_2$ | $6-CH_3$ | H | Phenyl | $CF_3$ | $CH_3$ | |
| 2.752 | $2-NO_2$ | $6-CH_3$ | H | 4-Cl-Phenyl | $CF_3$ | $CH_3$ | |
| 2.753 | $2-NO_2$ | $6-CH_3$ | H | 2-Furyl | $CF_3$ | $CH_3$ | |
| 2.754 | $2-NO_2$ | H | H | 2-Furyl | $CF_3$ | $CH_3$ | |
| 2.755 | 2-Cl | H | H | 2-Furyl | $CF_3$ | $CH_3$ | |

| Verb.No | $R^1$ | $R^2$ | $R^3$ | $-R^5 - R^6$ | $R^4$ | Phys. Daten |
|---|---|---|---|---|---|---|
| 2.756 | $2-NO_2$ | H | H | $-CH_2CH_2COCH_2-$ | $CF_3$ | |
| 2.757 | $2-NO_2$ | $6-CH_3$ | H | $-CH_2CH_2COCH_2-$ | $CF_3$ | |
| 2.758 | $2-NO_2$ | H | H | $-CH_2CH_2CHClCH_2-$ | $CF_3$ | |
| 2.759 | $2-NO_2$ | $6-CH_3$ | H | $-CH_2CH_2CHClCH_2-$ | $CF_3$ | |
| 2.760 | 2-Cl | 5-Cl | H | $-CH_2CH_2CHClCH_2-$ | $CF_3$ | |
| 2.761 | 2-Cl | 5-Cl | H | $-CH_2CH_2COCH_2-$ | $CF_3$ | |
| 2.762 | 2-Cl | 6-Cl | H | $-CH_2CH_2COCH_2-$ | $CF_3$ | |
| 2.763 | $2-OCF_3$ | H | H | $-CH_2CH_2COCH_2-$ | $CF_3$ | |
| 2.764 | 2-Cl | $6-CH_3$ | H | $-CH_2CH_2COCH_2-$ | $CF_3$ | |
| 2.765 | $2-SCH_3$ | H | H | $-CH_2CH_2CH_2CH_2-$ | $CF_3$ | |
| 2.766 | $2-SCH_3$ | H | H | $-CH_2-CH_2CH_2-$ | $CF_3$ | |
| 2.767 | $2-SC_2H_5$ | H | H | $-CH_2-CH_2-CH_2-CH_2-$ | $CF_3$ | |
| 2.768 | $2-SOC_2H_5$ | H | H | $-CH_2-CH_2-CH_2-CH_2-$ | $CF_3$ | |
| 2.769 | $2-SCHF_2$ | H | H | $-CH_2-CH_2-CH_2-CH_2-$ | $CF_3$ | |
| 2.770 | $2-SOCHF_2$ | H | H | $-CH_2-CH_2-CH_2-CH_2-$ | $CF_3$ | |
| 2.771 | 2-F | 6-Cl | H | $-CH_2-CH_2-CH_2-CH_2-$ | $CF_3$ | Fp. 112-113°C |
| 2.772 | 2-F | 6-Cl | H | $-CH_2-CH_2-CH_2-$ | $CF_3$ | Fp. 147-148°C |
| 2.773 | $2-SOCH_3$ | 5-Cl | H | $-CH_2-CH_2-CH_2-CH_2-$ | $CF_3$ | Fp. 194-195°C |

## Biologische Beispiele

### Beispiel B1: Pre-emergente Herbizid-Wirkung

Im Gewächshaus wird unmittelbar nach der Einsaat der Versuchspflanzen in Saatschalen die Erdoberfläche mit einer wässrigen Spritzbrühe entsprechend einer Aufwandmenge von 4 kg Wirksubstanz/Hektar behandelt. Die Saatschalen werden im Gewächshaus bei 22 - 25°C und 50 - 70 % relativer Luftfeuchtigkeit gehalten.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

Ergebnisse dieses Versuchs sind in Tabelle 3 zusammengefasst:

Tabelle 3

| Verb. No. | Setaria italica | Stellaria media |
|---|---|---|
| 1.058 | 2 | 2 |
| 1.284 | 1 | 2 |
| 1.340 | 2 | 2 |
| 1.449 | 1 | 2 |
| 1.496 | 1 | 1 |
| 1.512 | 2 | 4 |
| 1.545 | 1 | 1 |
| 1.548 | 2 | 1 |
| 1.577 | 2 | 1 |
| 1.627 | 1 | 2 |
| 1.642 | 2 | 1 |
| 1.672 | 2 | 2 |
| 1.772 | 1 | 1 |

Beispiel B2: Selektive Herbizidwirkung im Vorauflauf

Unmittelbar nach der Einsaat der Samen in Blumentöpfe von 12-15 cm Durchmesser wird die Oberfläche mit einer wässrigen Spritzbrühe, entsprechend einer Aufwandmenge von 1000 oder 500 [g] AS/[ha], behandelt.

Die Töpfe werden im Gewächshaus bei einer Temperatur von 22-25°C und 50-70 % relativer Luftfeuchtigkeit belassen.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen unter anderem die folgenden Verbindungen eine gute bis sehr gute Kulturpflanzenverträglichkeit in Gerste, Mais, Soja, Baumwolle oder Raps bei zugleich guter Herbizidwirkung unter anderem gegen Solanum nigrum, Galium aparine und Veronica Sp.: 1.284, 1.545, 1.563 und 1.627.

Beispiel B3 Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat durch eine Spritzung der Prüfsubstanzen auf die Gefässe. Die verwendete Dosis entspricht einer Wirkstoffmenge von 4 kg AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt. Die Verbindungen der Tabelle 1 schädigen dabei die Unkräuter, nicht aber den Reis.

Beispiel B4: Wuchshemmung bei Gräsern mit Klee

Eine Mischung von Gräsern (z.B. Poa, Festuca, Lolium, Bromus, Cynosurus) und Klee (Trifolium pratense/repens) wird in 15 cm-Kunststofftöpfen mit steriler Landerde angesät und im Gewächshaus bei einer Tagestemperatur von 21°C und einer Nachttemperatur von 17°C angezogen. Die Beleuchtungsdauer ist 13,5 Stunden/Tag bei einer Lichtintensität von mind. 7000 Lux. Nach dem Auflauf werden die Pflanzen wöchentlich auf ca. 6 cm höhe zurückgeschnitten. Ca. 42 Tage nach der Saat und 1 Tag nach dem letzten Schnitt erfolgt die Applikation mit 0,3 bis 3 kg Wirkstoff/ha, in der Regel 25 %ig formuliert und in wässriger Spritzbrühe. Die Wasseraufwandmenge ist ca. 500 l/ha.

Ca. 3 Wochen nach der Behandlung findet die Auswertung statt. Hierbei wird die Höhe des Neuzuwachses gemessen. Die geprüften Verbindungen der Tabelle 1 bewirken eine Reduktion des Neuzuwachses im Vergleich zur unbehandelten Kontrolle.

Beispiel B5: Wuchshemmung bei Getreide

Die Pflanzen (z.B. Sommergerste der Sorte Iban) werden in 15 cm-Kunststofftöpfen mit sterilisierter Landerde angesät und in der Klimakammer bei einer Tagestemperatur von 10-15°C und einer Nachttemperatur von 5-10°C angezogen. Die Beleuchtungsdauer ist 13,5 Stunden pro Tag.

Ca. 34 Tage nach der Saat und dem Ausdünnen auf 4 Pflanzen/Topf erfolgt die Applikation mit 0,3 bis 3 kg Wirkstoff/ha, in der Regel 25 %ig formuliert und in wässriger Spritzbrühe. Die Wasseraufwandmenge ist ca. 500 l/ha. Nach der Applikation werden die Pflanzen im Gewächshaus bei einer Tagestemperatur von mindestens 10°C aufgestellt. Die Beleuchtungsdauer ist mindestens 13,5 Stunden/Tag.

Ca. 28 Tage nach der Behandlung findet die Auswertung statt. Hierbei wird die Höhe des Neuzuwachses dargestellt. Die geprüften Verbindungen der Tabelle 1 bewirken eine Reduzierung des Neuzuwachses im Vergleich zur unbehandelten Kontrolle.

Beispiel B6: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerata und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabelle 1 besprüht. Die Wirkstoffmenge beträgt umgerechnet bis zu 500 g Aktivsubstanz pro Hektar. 21 Tage nach Applikation wird das Wachstum der Gräser beurteilt.

Die geprüften Verbindungen der Tabelle 1 bewirken eine Reduzierung des Neuzuwachses im Vergleich zur unbehandelten Kontrolle.

Formulierungsbeispiele

Beispiel F1: Formulierungsbeispiele für Wirkstoffe der Formel I' (% = Gewichtsprozent)

| a) Emulsionskonzentrate | a) | b) | c) |
|---|---|---|---|
| Wirkstoff gemäss Tabelle 1 | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenol-polyäthylenglykoläther (30 mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) |
|---|---|---|---|
| Wirkstoff gem. Tabelle 1 | 80 % | 10 % | 5 % |
| Aethylenglykol-monomethyläther | 20 % | - | - |
| Polyäthylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

| c) Granulate | a) | b) |
|---|---|---|
| Wirkstoff gemäss Tabelle 1 | 5 % | 10 % |
| Kaolin | 94 % | - |
| Hochdisperse Kieselsäure | 1 % | |
| Attapulgit | - | 90 % |

Eine Wirkstofflösung wird auf den Träger aufgesprüht und das Lösungsmittel anschliessend im Vakuum abgedampft.

| d) Stäubemittel | a) | b) |
|---|---|---|
| Wirkstoff gemäss Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertiges Stäubemittel.

| e) Spritzpulver | a) | b) |
|---|---|---|
| Wirkstoff gemäss Tabelle 1 | 20 % | 60 % |
| Na-Ligninsulfonat | 5 % | 5 % |
| Na-Laurylsulfat | - | 6 % |
| Octylphenolpolyäthylenglykoläther (7-8 Mol Ae) | - | 2 % |
| Hochdisperse Kieselsäure | 5 % | 27 % |
| Kaolin | 70 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

f) Extruder Granulat

| | |
|---|---|
| Wirkstoff gemäss Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

g) Umhüllungs-Granulat

| | |
|---|---|
| Wirkstoff gemäss Tabelle 1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

h) Suspensions-Konzentrat

| | |
|---|---|
| Wirkstoff gemäss Tabelle 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | ad 100 % |

Der Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Patentansprüche**

**Patentansprüche für folgende Vertragsstaaten : AT, BE, CH, DE, FR, GB, GR, IT, LI, LU, NL, SE**

1. Harnstoffe der Formel I

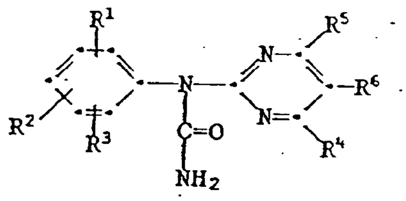

(I),

worin

| | |
|---|---|
| $R^1$, $R^2$ und $R^3$ | unabhängig voneinander Wasserstoff; Nitro; Cyano; Halogen; $C_1$-$C_4$-Alkyl; $C_1$-$C_4$-Alkyl-$S(O)_n$; $C_1$-$C_4$-Alkoxy; $C_1$-$C_4$-Halogenalkyl; $C_1$-$C_4$-Halogenalkoxy; $C_1$-$C_4$-Halogenalkyl-$S(O)_n$; $C_1$-$C_4$-Alkoxycarbonyl; $C_1$-$C_4$-Alkylcarbonyl; Aminocarbonyl; Mono-$C_1$-$C_4$-alkylaminocarbonyl; oder Di-$C_1$-$C_4$-Alkylaminocarbonyl; |
| $R^4$ und $R^5$ | unabhängig voneinander Wasserstoff; $C_1$-$C_4$-Alkyl; $C_1$-$C_4$-Alkoxy; $C_1$-$C_4$-Alkyl-$S(O)_n$; $C_1$-$C_4$-Halogenalkyl; $C_1$-$C_4$-Halogenalkoxy; $C_1$-$C_4$-Halogenalkyl-$S(O)_n$; unsubstituiertes oder bis zu dreifach gleich oder verschieden durch $C_1$-$C_4$-Alkyl, Halogen, $C_1$-$C_4$-Alkoxy, $C_1$-$C_4$-Halogenalkyl, Nitro oder Cyano substituiertes Phenyl; Furanyl; Thiophenyl; $C_3$-$C_6$-Cycloalkyl; $C_1$-$C_4$-Alkoxycarbonyl; $C_1$-$C_4$-Alkoxy-$C_1$-$C_4$-alkyl; $C_1$-$C_4$-Alkoxycarbonyl-$C_1$-$C_4$-alkyl; $C_1$-$C_4$-Alkylcarbonyl-$C_1$-$C_4$-alkyl; $C_3$-$C_4$-Alkenyloxycarbonyl-$C_1$-$C_4$-alkyl; $C_3$-$C_4$-Alkinyloxycarbonyl-$C_1$-$C_4$-alkyl; Halogen; oder Cyano; |
| $R^6$ | $C_1$-$C_4$-Alkyl; Halogen; Cyano; $C_3$-$C_6$-Cycloalkyl; $C_1$-$C_4$-Halogenalkyl; $C_1$-$C_4$-Alkoxy; Nitro; $C_1$-$C_4$-Alkyl-$S(O)_n$; $C_1$-$C_4$-Halogenalkoxy; $C_1$-$C_4$-Halogenalkyl-$S(O)_n$ ; oder unsubstituiertes oder bis zu dreifach gleich oder verschieden durch Halogen, $C_1$-$C_4$-Alkyl, $C_1$-$C_4$-Halogenalkyl, $C_1$-$C_4$-Alkyl-$S(O)_n$, $C_1$-$C_4$-Alkoxy, $C_1$-$C_4$-Halogenalkoxy, Nitro oder Cyano substituiertes Phenyl; oder $C_1$-$C_4$-Alkylthio-$C_1$-$C_4$-alkyl; |
| n | 0, 1 oder 2; oder |
| $R^5$ und $R^6$ | gemeinsam mit den beiden Kohlenstoffatomen an welche sie gebunden sind einen anellierten, teilweise ungesättigten 4- bis 8-gliedrigen Ring, der bis zu dreifach gleich oder verschieden durch $C_1$-$C_4$-Alkyl, Halogen, $C_1$-$C_4$-Alkoxycarbonyl substituiert sein kann und/oder durch O, S oder N-($C_1$-$C_4$)-Alkyl unterbrochen ist und/oder eine Doppelbindung und/oder eine Carbonylgruppe enthalten kann; |

bedeutet, unter Einschluss ihrer Salze mit Säuren, Basen und Komplexbildnern.

2. Harnstoffe gemäss Anspruch 1 der Formel Ih

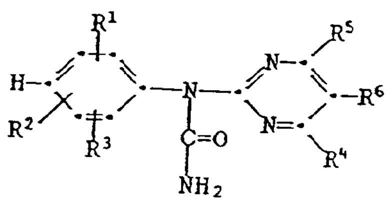

(Ih),

worin

| | |
|---|---|
| $R^1$ | Wasserstoff; Nitro; Cyano; Halogen; $C_1$-$C_4$-Alkyl; $C_1$-$C_4$-Alkyl-$S(O)_n$; $C_1$-$C_4$-Alkoxy; $C_1$-$C_4$-Halogenalkyl; $C_1$-$C_4$-Halogenalkoxy; $C_1$-$C_4$-Halogenalkyl-$S(O)_n$; $C_1$-$C_4$-Alkoxycarbonyl; $C_1$-$C_4$-Alkylcarbonyl; Aminocarbonyl; Mono-$C_1$-$C_4$-alkylaminocarbonyl; oder Di-$C_1$-$C_4$-Alkylaminocarbonyl; |
| $R^2$ | Wasserstoff; Nitro; Halogen; $C_1$-$C_4$-Alkyl; oder $C_1$-$C_4$-Halogenalkyl; und |
| $R^3$ | Wasserstoff; Halogen; oder $C_1$-$C_4$-Alkyl bedeutet und |
| $R^4$ bis $R^6$ | und n wie in Anspruch 1 definiert sind. |

3. Harnstoffe gemäss Anspruch 1 der Formel Ih

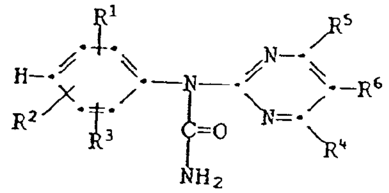

(Ih),

worin

| | |
|---|---|
| $R^1$ | Wasserstoff; Nitro; Cyano; Halogen; $C_1$-$C_3$-Alkyl; $C_1$-$C_2$-Alkyl-S(O)$_n$; $C_1$-$C_2$-Halogenalkyl-S(O)$_n$; $C_1$-$C_3$-Alkoxy; $C_1$-$C_3$-Halogenalkyl; $C_1$-$C_2$-Halogenalkoxy; $C_1$-$C_3$-Alkoxycarbonyl; oder Dimethylaminocarbonyl; |
| $R^2$ | Wasserstoff; Nitro; Fluor; Chlor; Brom;-oder $C_1$-$C_3$-Alkyl; |
| $R^3$ | Wasserstoff; Chlor; oder $C_1$-$C_3$-Alkyl; |
| $R^4$ | Wasserstoff; $C_1$-$C_4$-Alkyl; $C_1$-$C_3$-Alkoxy; $C_1$-$C_4$-Alkyl-S(O)$_n$-; $C_1$-$C_3$-Halogenalkyl; $C_1$-$C_2$-Halogenalkoxy; Phenyl; Chlorphenyl; Furanyl; $C_3$-$C_6$-Cycloalkyl; $C_1$-$C_4$-Alkoxycarbonyl-$C_1$-$C_2$-alkyl; $C_1$-$C_2$-Alkoxy-$C_1$-$C_2$-alkyl; $C_1$-$C_2$-Halogenalkylthio; Chlor; Cyano; oder Acetonyl; , |
| $R^5$ | Wasserstoff; $C_1$-$C_3$-Alkyl; $C_1$-$C_3$-Alkoxy; Halogen; $C_1$-$C_3$-Halogenalkyl; oder $C_1$-$C_3$-Halogenalkoxy; |
| $R^6$ | $C_1$-$C_3$-Alkyl; $C_1$-$C_3$-Alkoxy; $C_1$-$C_3$-Halogenalkyl; $C_1$-$C_3$-Halogenalkoxy; Chlor; Brom; $C_1$-$C_3$-Alkylthio; Cyano; Nitro; Phenyl; Chlorphenyl: $C_1$-$C_2$-Alkylthio-$C_1$-$C_2$-alkyl; oder $C_3$-$C_6$-Cycloalkyl; |
| n | 0, 1 oder 2; |
| $R^5$ und $R^6$ | gemeinsam eine gegebenenfalls bis zu dreifach durch $C_1$-$C_3$-Alkyl oder gegebenenfalls einfach durch $C_1$-$C_3$-Alkoxycarbonyl oder Chlor substituierte $-CH_2CH_2-$, $-CH_2-CH_2-CH_2-$, $-CH_2-CH_2-CH_2-CH_2-$, $-CH_2-CH_2-CO-CH_2-$, $-CH=CH-CH_2-$, $-CH=CH-CH_2-CH_2-$, $-O-CH_2-CH_2-CH_2-$, $-CH_2-CH_2-S-$, $-CH_2-CH_2-S-CH_2-$, $-CH_2-CH_2-O-CH_2-$ oder $-CH_2-CH_2-N(C_1-C_3-Alkyl)-CH_2$-Brücke |

bedeutet.

**4.** Harnstoffe gemäss Anspruch 1 der Formel Ih

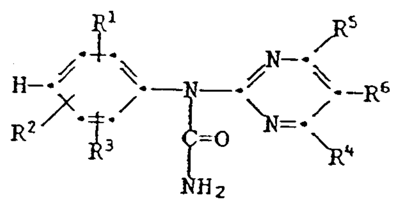

(Ih),

worin

| | |
|---|---|
| $R^1$ | Nitro; Cyano; Halogen; Methyl; Ethyl; $C_1$-$C_3$-Halogenalkyl; $C_1$-$C_2$-Halogenalkyl-S(O)$_n$; Methoxy; Ethoxy; $C_1$-$C_3$-Halogenalkyl; Halogenmethoxy; $C_1$-$C_3$-Alkoxycarbonyl; öder Dimethylaminocarbonyl; |
| n | 0, 1 oder 2; |
| $R^2$ | Wasserstoff; Fluor; Chlor; Brom; Nitro; oder Methyl; |
| $R^3$ | Wasserstoff; Chlor; oder Methyl; |
| $R^4$ | $C_1$-$C_4$-Alkyl; $C_1$-$C_3$-Alkoxy; $C_1$-$C_4$-Alkylthio; Methylsulfinyl; Ethylsulfinyl; Methylsulfonyl; Ethylsulfonyl; $C_3$-$C_6$-Cycloalkyl; Chlor; Cyano; $C_1$-$C_3$-Halogenalkyl; Methoxymethyl; Methoxycarbonylmethyl; Acetonyl; Phenyl; 4-Chlorphenyl; oder 2-Furanyl; |
| $R^5$ | $C_1$-$C_2$-Alkyl; Chlor; $C_1$-$C_3$-Haloalkyl; oder Halogenmethoxy; |
| $R^6$ | $C_1$-$C_3$-Alkyl; Methoxy; Ethoxy; $C_1$-$C_2$-Haloalkyl; Chlor; Brom; $C_1$-$C_3$-Alkylthio; Cyano; Nitro; Phenyl; 4-Chlorphenyl; 2-Methylthioethyl; oder $C_3$-$C_6$-Cycloalkyl; oder |
| $R^5$ und $R^6$ | gemeinsam $-(CH_2)_3-$, $CHCH_3-CH_2-CH_2-$, $-(CH_3)_2-CH_2-CH_2-$, $-CH_2-CH(CH_3)-CH_2-$, $-CH_2-CH_2-CH(CH_3)-$, $-CH_2-C(CH_3)_2-CH_2-$, $-CH_2-CH_2-$, $-(CH_2)_4-$, $-CH(CH_3)-(CH_2)_3-$, $-CH_2-CH(CH_3)-(CH_2)_2-$, $-(CH_2)_3-CH(CH_3)-$, $-(CH_2)_2-CH(CH_3)-CH_2-$, $-(CH_2)_5-$, $-(CH_2)_6-$ oder $(-CH_2)_3-O-$ |

bedeutet.

5. Harnstoffe gemäss einem der Ansprüche 1 bis 4 der Formel Ia oder Ib

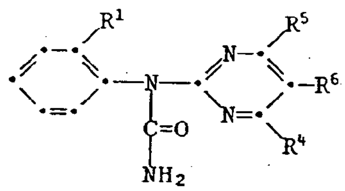

(Ia)

oder

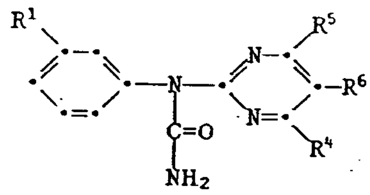

(Ib).

6. Harnstoffe gemäss einem der Ansprüche 1 bis 4 der Formel Ic, Id, Ie oder If

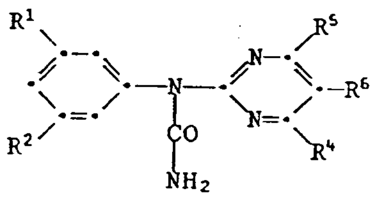

(Ic)

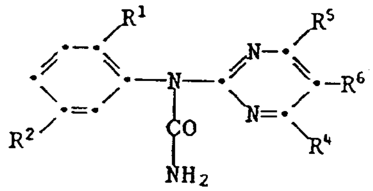

(Id)

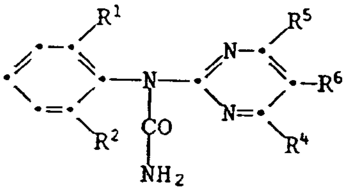

(Ie)

oder

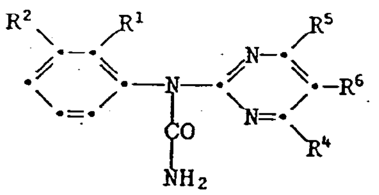

(If).

7. Harnstoffe gemäss einem der Ansprüche 1 bis 4 der Formel Ig

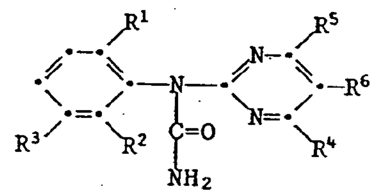

(Ig).

8. Verfahren zur Herstellung von Harnstoffen der Formel I gemäss einem der Ansprüche 1 bis 7, dadurch gekennzeichnet, dass man

a) ein Anilin der Formel II mit Phosgen zu einem Carbaminchlorid der Formel III umsetzt und dieses in einer zweiten Stufe mit $NH_3$ zu

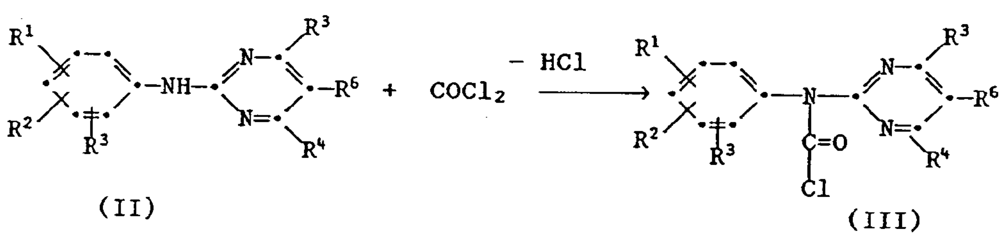


(II) (III)

$$III + NH_3 \xrightarrow{-\ HCl} I$$

einem Harnstoff der Formel I umsetzt oder

b) ein Anilin der Formel II mit Halogensulfonylisocyanat XVIII zu einem Halogensulfonylharnstoff der Formel IV umsetzt

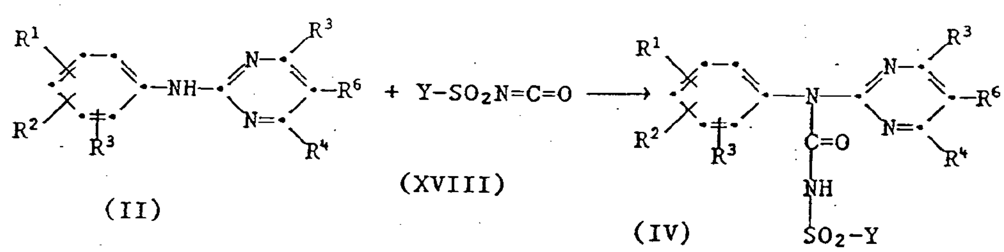

(II) (XVIII) (IV)

$$IV + 2H_2O \xrightarrow[-\ SO_4H_2]{-\ HY} I$$

und diesen in einer zweiten Stufe oder direkt zu einer Verbindung der Formel I hydrolisiert, wobei Y für

eine unter den Reaktionsbedingungen abspaltbare Gruppe wie Halogen, vorzugsweise Chlor, steht.

**9.** Verfahren zur Herstellung von Harnstoffen der Formel I'

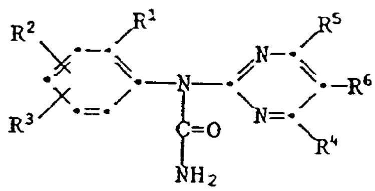

(I'),

worin $R^1$ in der ortho-Stellung des Phenylringes gebunden ist und Nitro bedeutet, und die Reste $R^2$ bis $R^6$ wie in einem der Ansprüche 1 bis 7 definiert sind, dadurch gekennzeichnet, dass man einen Sulfonylharnstoff der Formel V unter Einwirkung einer wässrigen Base zu einem Harnstoff der Formel I' umlagert

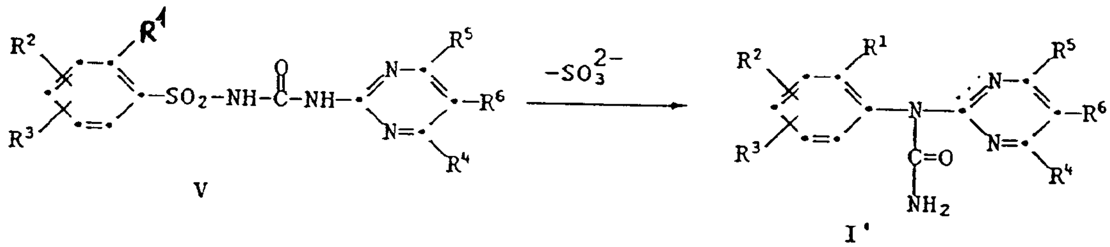


V

I'

**10.** Carbamoylchloride der Formel III

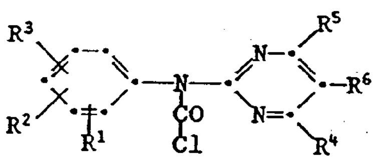

(III),

worin die Reste $R^1$ bis $R^6$ wie in einem der Ansprüche 1 bis 7 definiert sind.

**11.** Verfahren zur Herstellung von Carbamoylchloriden der Formel III gemäss Anspruch 10, dadurch gekennzeichnet, dass man ein Anilin der Formel II mit Phosgen zu III umsetzt

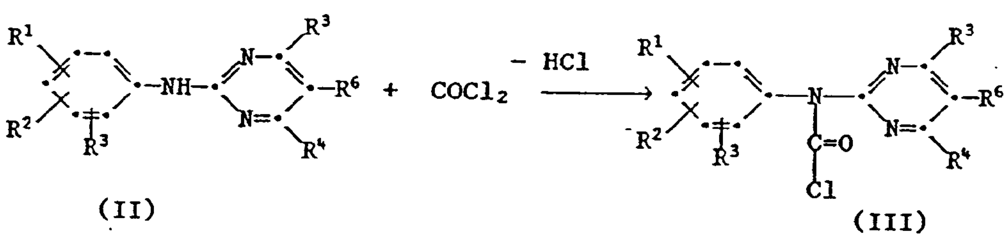


(II)

(III)

**12.** Harnstoffe der Formel IV

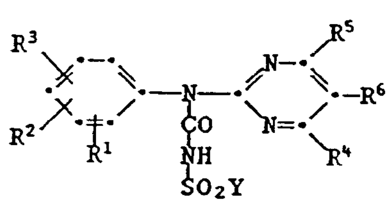

(IV),

worin die Reste $R^1$ bis $R^6$ wie in einem der Ansprüche 1 bis 7 definiert sind und Y für Halogen steht.

**13.** Verfahren zur Herstellung von Harnstoffen gemäss Anspruch 12, dadurch gekennzeichnet, dass man ein Anilin der Formel II, worin $R^1$ bis $R^6$ wie in Anspruch 12 definiert sind, mit einem Halogensulfonylisocyanat XVIII, zu einem Halogensulfonylharnstoff der Formel IV umsetzt

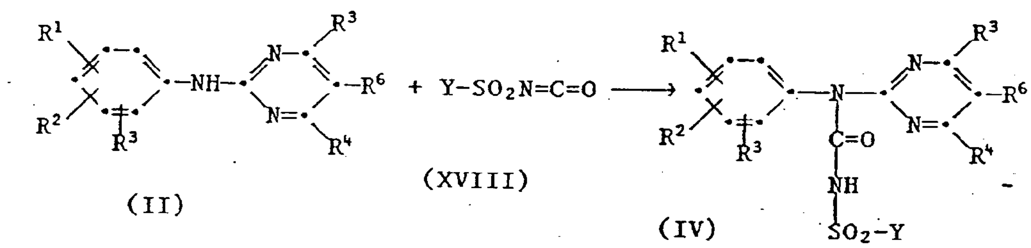

(II) (XVIII) (IV)

**14.** Isocyanate der Formel XIII

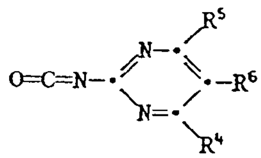

XIII,

worin die Reste $R^4$ bis $R^6$ wie in Anspruch 12 definiert sind.

**15.** Herbizides oder pflanzenwuchsregulatorisches Mittel, enthaltend als Wirksubstanz eine Verbindung der Formel I, gemäss einem der Ansprüche 1 bis 7, neben weiteren Hilfs-und/oder Trägerstoffen.

**16.** Verfahren zur Bekämpfung unerwünschten Pflanzenwuchses, dadurch gekennzeichnet, dass man ein herbizid wirksame Menge einer Verbindung, gemäss einem der Ansprüche 1 bis 7, oder eines Mittels gemäss Anspruch 15 auf die zu bekämpfende Pflanze oder deren Lebensraum einwirken lässt.

**17.** Verfahren gemäss Anspruch 16 zur pre- oder postemergenten Bekämpfung unerwünschten Pflanzenwuchses in Nutzpflanzenkulturen.

**18.** Verfahren zur Beeinflussung des Pflanzenwuchses, dadurch gekennzeichnet, dass man eine pflanzenwuchsregulatorisch wirksame Menge einer Verbindung, gemäss einem der Ansprüche 1 bis 7 oder eines Mittels, gemäss Anspruch 15 auf die Pflanze oder deren Lebensraum einwirken lässt.

**Patentansprüche für folgenden Vertragsstaat : ES**

**1.** Verfahren zur Herstellung von Harnstoffen der Formel I

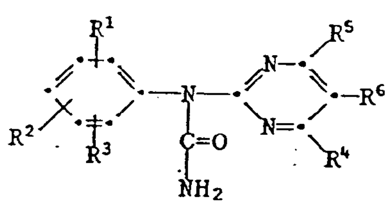

(I),

worin

$R^1$, $R^2$ und $R^3$ unabhängig voneinander Wasserstoff; Nitro; Cyano; Halogen; $C_1$-$C_4$-Alkyl; $C_1$-$C_4$-Alkyl-$S(O)_n$ ; $C_1$-$C_4$-Alkoxy; $C_1$-$C_4$-Halogenalkyl; $C_1$-$C_4$-Halogenalkoxy; $C_1$-$C_4$-Halogen-

alkyl-S(O)$_n$; $C_1$-$C_4$-Alkoxycarbonyl; $C_1$-$C_4$-Alkylcarbonyl; Aminocarbonyl; Mono-$C_1$-$C_4$-alkylaminocarbonyl; oder Di-$C_1$-$C_4$-Alkylaminocarbonyl;

$R^4$ und $R^5$ unabhängig voneinander Wasserstoff; $C_1$-$C_4$-Alkyl; $C_1$-$C_4$-Alkoxy; $C_1$-$C_4$-Alkyl-S(O)$_n$ ; $C_1$-$C_4$-Halogenalkyl; $C_1$-$C_4$-Halogenalkoxy; $C_1$-$C_4$-Halogenalkyl-S(O)$_n$; unsubstituiertes oder bis zu dreifach gleich oder verschieden durch $C_1$-$C_4$-Alkyl, Halogen, $C_1$-$C_4$-Alkoxy, $C_1$-$C_4$-Halogenalkyl, Nitro oder Cyano substituiertes Phenyl; Furanyl; Thiophenyl; $C_3$-$C_6$-Cycloalkyl; $C_1$-$C_4$-Alkoxycarbonyl; $C_1$-$C_4$-Alkoxy-$C_1$-$C_4$-alkyl; $C_1$-$C_4$-Alkoxycarbonyl-$C_1$-$C_4$-alkyl; $C_1$-$C_4$-Alkylcarbonyl-$C_1$-$C_4$-alkyl; $C_3$-$C_4$-Alkenyloxycarbonyl-$C_1$-$C_4$-alkyl; $C_3$-$C_4$-Alkinyloxycarbonyl-$C_1$-$C_4$-alkyl; Halogen; oder Cyano;

$R^6$ $C_1$-$C_4$-Alkyl; Halogen; Cyano; $C_3$-$C_6$-Cycloalkyl; $C_1$-$C_4$-Halogenalkyl; $C_1$-$C_4$-Alkoxy; Nitro; $C_1$-$C_4$-Alkyl-S(O)$_n$; $C_1$-$C_4$-Halogenalkoxy; $C_1$-$C_4$-Halogenalkyl-S(O)$_n$; oder unsubstituiertes oder bis zu dreifach gleich oder verschieden durch Halogen, $C_1$-$C_4$-Alkyl, $C_1$-$C_4$-Halogenalkyl, $C_1$-$C_4$-Alkyl-S(O)$_n$ , $C_1$-$C_4$-Alkoxy, $C_1$-$C_4$-Halogenalkoxy, Nitro oder Cyano substituiertes Phenyl; oder $C_1$-$C_4$-Alkylthio-$C_1$-$C_4$-alkyl;

n 0, 1 oder 2; oder

$R^5$ und $R^6$ gemeinsam mit den beiden Kohlenstoffatomen an welche sie gebunden sind einen anellierten, teilweise ungesättigten 4- bis 8-gliedrigen Ring, der bis zu dreifach gleich oder verschieden durch $C_1$-$C_4$-Alkyl, Halogen, $C_1$-$C_4$-Alkoxycarbonyl substituiert sein kann und/oder durch O, S oder N-($C_1$-$C_4$)-Alkyl unterbrochen ist und/oder

eine Doppelbindung und/oder eine Carbonylgruppe enthalten kann; dadurch gekennzeichnet, dass man

a) ein Anilin der Formel II mit Phosgen zu einem Carbaminchlorid der Formel III umsetzt und dieses in einer zweiten Stufe mit $NH_3$ zu

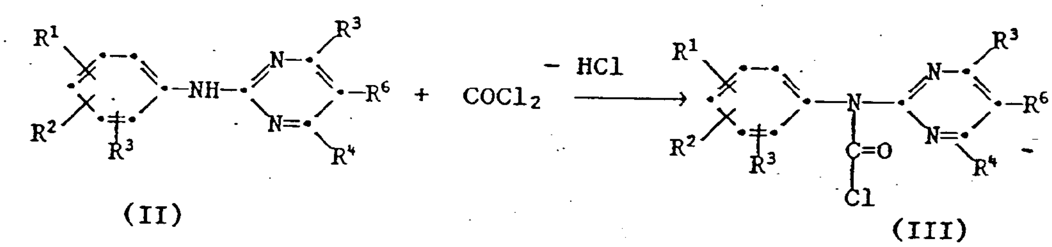


(II) (III)

$$III + NH_3 \xrightarrow{-\ HCl} I$$

einem Harnstoff der Formel I umsetzt oder

b) ein Anilin der Formel II mit Halogensulfonylisocyanat XVIII zu einem Halogensulfonylharnstoff der Formel IV umsetzt

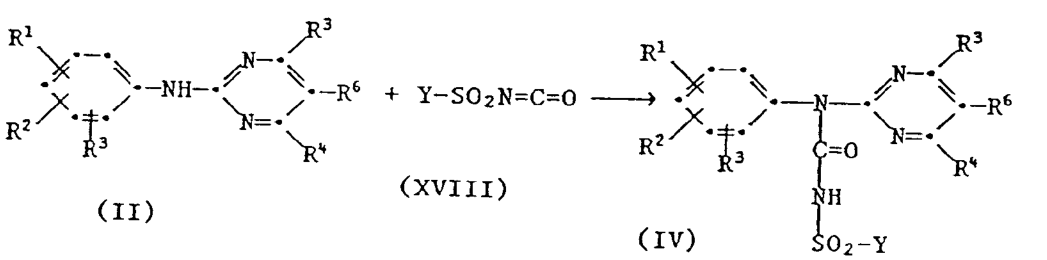

(II) (XVIII) (IV)

$$IV + 2H_2O \xrightarrow[-\ SO_4H_2]{-\ HY} I$$

und diesen in einer zweiten Stufe oder direkt zu einer Verbindung der Formel I hydrolisiert, wobei Y für

eine unter den Reaktionsbedingungen abspaltbare Gruppe wie Halogen, vorzugsweise Chlor, steht.

2. Verfahren gemäss Anspruch 1 zur Herstellung von Harnstoffen der Formel Ih

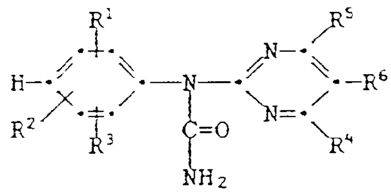

(Ih)

worin

| | |
|---|---|
| $R^1$ | Wasserstoff; Nitro; Cyano; Halogen; $C_1$-$C_3$-Alkyl; $C_1$-$C_2$-Alkyl-$S(O)_n$; $C_1$-$C_2$-Halogenalkyl-$S(O)_n$ ; $C_1$-$C_3$-Alkoxy; $C_1$-$C_3$-Halogenalkyl; $C_1$-$C_2$-Halogenalkoxy; $C_1$-$C_3$-Alkoxycarbonyl; oder Dimethylaminocarbonyl; |
| $R^2$ | Wasserstoff; Nitro; Fluor; Chlor; Brom; oder $C_1$-$C_3$-Alkyl; |
| $R^3$ | Wasserstoff; Chlor; oder $C_1$-$C_3$-Alkyl; |
| $R^4$ | Wasserstoff; $C_1$-$C_4$-Alkyl; $C_1$-$C_3$-Alkoxy; $C_1$-$C_4$-Alkyl-$S(O)_n$-; $C_1$-$C_3$-Halogenalkyl; $C_1$-$C_2$-Halogenalkoxy; Phenyl; Chlorphenyl; Furanyl; $C_3$-$C_6$-Cycloalkyl; $C_1$-$C_4$-Alkoxycarbonyl-$C_1$-$C_2$-alkyl; $C_1$-$C_2$-Alkoxy-$C_1$-$C_2$-alkyl; $C_1$-$C_2$-Halogenalkylthio; Chlor; Cyano; oder Acetonyl; |
| $R^5$ | Wasserstoff; $C_1$-$C_3$-Alkyl; $C_1$-$C_3$-Alkoxy; Halogen; $C_1$-$C_3$-Halogenalkyl; oder $C_1$-$C_3$-Halogenalkoxy; |
| $R^6$ | $C_1$-$C_3$-Alkyl; $C_1$-$C_3$-Alkoxy; $C_1$-$C_3$-Halogenalkyl; $C_1$-$C_3$-Halogenalkoxy; Chlor; Brom; $C_1$-$C_3$-Alkylthio; Cyano; Nitro; Phenyl; Chlorphenyl: $C_1$-$C_2$-Alkylthio-$C_1$-$C_2$-alkyl; oder $C_3$-$C_6$-Cycloalkyl; |
| n | 0, 1 oder 2; |
| $R^5$ und $R^6$ | gemeinsam eine gegebenenfalls bis zu dreifach durch $C_1$-$C_3$-Alkyl oder gegebenenfalls einfach durch $C_1$-$C_3$-Alkoxycarbonyl oder Chlor substituierte $-CH_2-CH_2-$, $-CH_2-CH_2-CH_2-$, $-CH_2-CH_2-CH_2-CH_2-$, $-CH_2-CH_2-CO-CH_2-$, $-CH=CH-CH_2-$, $-CH=CH-CH_2-CH_2-$, $-O-CH_2-CH_2-CH_2-$, $-CH_2-CH_2-S-$, $-CH_2-CH_2-S-CH_2-$, $-CH_2-CH_2-O-CH_2-$ oder $-CH_2-CH_2-N(C_1-C_3$-Alkyl)-$CH_2$-Brücke |

bedeutet.

3. Verfahren zur Herstellung von Harnstoffen der Formel I'

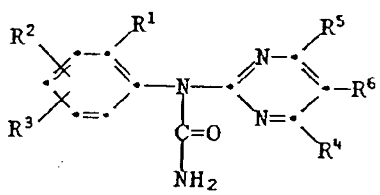

(I'),

worin $R^1$ in der ortho-Stellung des Phenylringes gebunden ist und Nitro bedeutet, und die Reste $R^2$ bis $R^6$ wie in Anspruch 1 oder 2 definiert sind, dadurch gekennzeichnet, dass man einen Sulfonylharnstoff der Formel V unter Einwirkung einer wässrigen Base zu einem Harnstoff der Formel I' umlagert

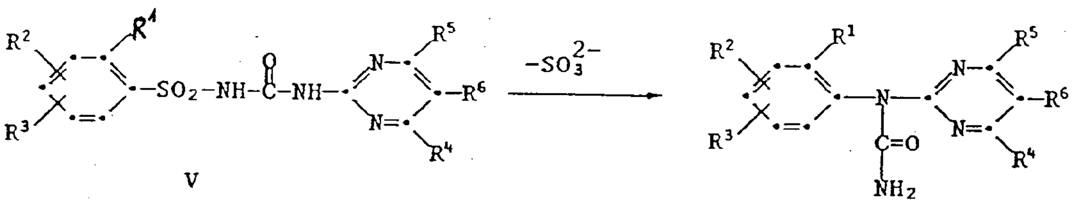


V

I'

4. Verfahren zur Herstellung von Carbamoylchloriden der Formel III

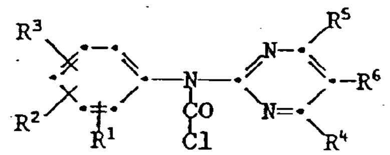

(III),

worin die Reste $R^1$ bis $R^6$ wie in Anspruch 1 oder 2 definiert sind, dadurch gekennzeichnet, dass man ein Anilin der Formel II mit Phosgen zu III umsetzt

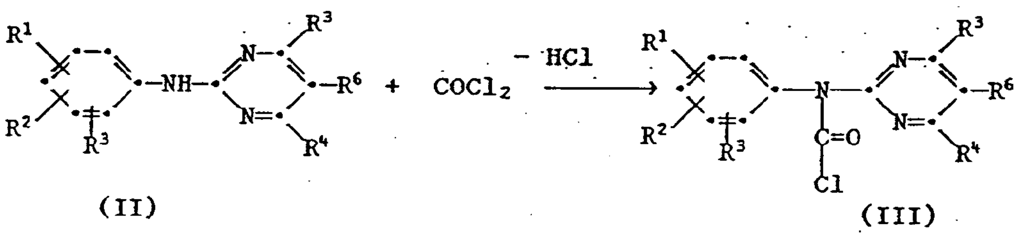


(II) (III)

5. Verfahren zur Herstellung von Harnstoffen der Formel IV

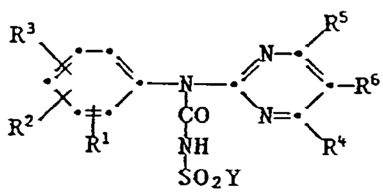

(IV),

worin die Reste $R^1$ bis $R^6$ wie in Anspruch 1 oder 2 definiert sind und Y für Halogen steht, dadurch gekennzeichnet, dass man ein Anilin der Formel II, worin $R^1$ bis $R^6$ wie zuvor definiert sind, mit einem Halogensulfonylisocyanat XVIII, zu einem Halogensulfonylharnstoff der Formel IV umsetzt

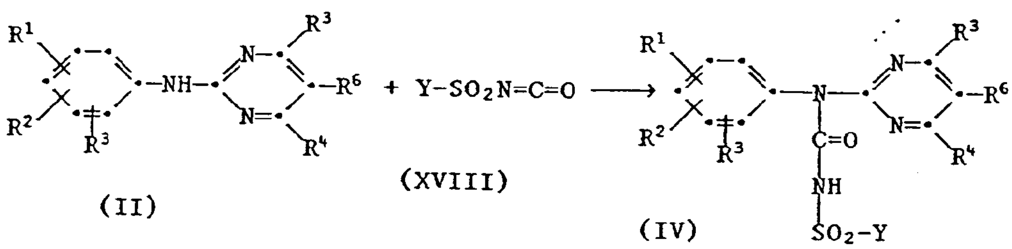


(II) (XVIII) (IV)

6. Verfahren zur Bekämpfung unerwünschten Pflanzenwuchses oder zur Beeinflussung des Pflanzenwuchses, dadurch gekennzeichnet, dass man eine herbizid wirksame oder eine pflanzenwuchsregulatorisch wirksame Menge eines Harnstoffes der Formel I

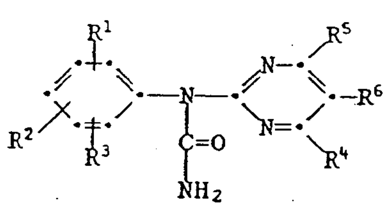

(I),

worin

$R^1$, $R^2$ und $R^3$ unabhängig voneinander Wässerstoff; Nitro; Cyano; Halogen; $C_1$-$C_4$-Alkyl; $C_1$-$C_4$-Alkyl-$S(O)_n$; $C_1$-$C_4$-Alkoxy; $C_1$-$C_4$-Halogenalkyl; $C_1$-$C_4$-Halogenalkoxy; $C_1$-$C_4$-Halogenalkyl-$S(O)_n$ ; $C_1$-$C_4$-Alkoxycarbonyl; $C_1$-$C_4$-Alkylcarbonyl; Aminocarbonyl; Mono-$C_1$-$C_4$-alkylaminocarbonyl; oder Di-$C_1$-$C_4$-Alkylaminocarbonyl;

$R^4$ und $R^5$ unabhängig voneinander Wasserstoff; $C_1$-$C_4$-Alkyl; $C_1$-$C_4$-Alkoxy; $C_1$-$C_4$-Alkyl-$S(O)_n$; $C_1$-$C_4$-Halogenalkyl; $C_1$-$C_4$-Halogenalkoxy; $C_1$-$C_4$-Halogenalkyl-$S(O)_n$ ; unsubstituiertes oder bis zu dreifach gleich oder verschieden durch $C_1$-$C_4$-Alkyl, Halogen, $C_1$-$C_4$-Alkoxy, $C_1$-$C_4$-Halogenalkyl, Nitro oder Cyano substituiertes Phenyl; Furanyl; Thiophenyl; $C_3$-$C_6$-Cycloalkyl; $C_1$-$C_4$-Alkoxycarbonyl; $C_1$-$C_4$-Alkoxy-$C_1$-$C_4$-alkyl; $C_1$-$C_4$-Alkoxycarbonyl-$C_1$-$C_4$-alkyl; $C_1$-$C_4$-Alkylcarbonyl-$C_1$-$C_4$-alkyl; $C_1$-$C_4$-Alkenyloxycarbonyl-$C_1$-$C_4$-alkyl; $C_3$-$C_4$-Alkinyloxycarbonyl-$C_1$-$C_4$-alkyl; Halogen; oder Cyano;

$R^6$ $C_1$-$C_4$-Alkyl; Halogen; Cyano; $C_3$-$C_6$-Cycloalkyl; $C_1$-$C_4$-Halogenalkyl; $C_1$-$C_4$-Alkoxy; Nitro; $C_1$-$C_4$-Alkyl-$S(O)_n$; $C_1$-$C_4$-Halogenalkoxy; $C_1$-$C_4$-Halogenalkyl-$S(O)_n$ ; oder unsubstituiertes oder bis zu dreifach gleich oder verschieden durch Halogen, $C_1$-$C_4$-Alkyl, $C_1$-$C_4$-Halogenalkyl, $C_1$-$C_4$-Alkyl-$S(O)_n$, $C_1$-$C_4$-Alkoxy, $C_1$-$C_4$-Halogenalkoxy, Nitro oder Cyano substituiertes Phenyl; oder $C_1$-$C_4$-Alkylthio-$C_1$-$C_4$-alkyl;

n 0, 1 oder 2; oder

$R^5$ und $R^6$ gemeinsam mit den beiden Kohlenstoffatomen an welche sie gebunden sind einen anellierten, teilweise ungesättigten 4- bis 8-gliedrigen Ring, der bis zu dreifach gleich oder verschieden durch $C_1$-$C_4$-Alkyl, Halogen, $C_1$-$C_4$-Alkoxycarbonyl substituiert sein kann und/oder durch O, S oder N-($C_1$-$C_4$)-Alkyl unterbrochen ist und/oder eine Doppelbindung und/oder eine Carbonylgruppe enthalten kann;

auf die Pflanze oder auch Lebensraum einwirken lässt.

7. Verfahren nach Anspruch 6 zur Bekämpfung unerwünschten Pflanzenwuchses in Nutzpflanzenkulturen.

8. Verfahren nach Anspruch 6 zur Beeinflussung des Wachstums von Kulturpflanzen.

9. Herbizides Mittel, enthaltend eine Verbindung der Formel I

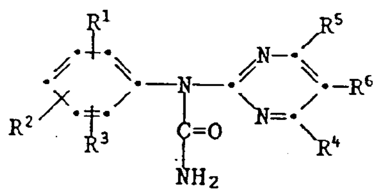

(I),

worin

$R^1$, $R^2$ und $R^3$ unabhängig voneinander Wasserstoff; Nitro; Cyano; Halogen; $C_1$-$C_4$-Alkyl; $C_1$-$C_4$-Alkyl-$S(O)_n$ ; $C_1$-$C_4$-Alkoxy; $C_1$-$C_4$-Halogenalkyl; $C_1$-$C_4$-Halogenalkoxy; $C_1$-$C_4$-Halogenalkyl-$S(O)_n$ ; $C_1$-$C_4$-Alkoxycarbonyl; $C_1$-$C_4$-Alkylcarbonyl; Aminocarbonyl; Mono-$C_1$-$C_4$-alkylaminocarbonyl; oder Di-$C_1$-$C_4$-Alkylaminocarbonyl;

$R^4$ und $R^5$ unabhängig voneinander Wasserstoff; $C_1$-$C_4$-Alkyl; $C_1$-$C_4$-Alkoxy; $C_1$-$C_4$-Alkyl-$S(O)_n$; $C_1$-$C_4$-Halogenalkyl; $C_1$-$C_4$-Halogenalkoxy; $C_1$-$C_4$-Halogenalkyl-$S(O)_n$ ; unsubstituiertes oder bis zu dreifach gleich oder verschieden durch $C_1$-$C_4$-Alkyl, Halogen, $C_1$-$C_4$-Alkoxy, $C_1$-$C_4$-Halogenalkyl, Nitro oder Cyano substituiertes Phenyl; Furanyl; Thiophenyl; $C_3$-$C_6$-Cycloalkyl; $C_1$-$C_4$-Alkoxycarbonyl; $C_1$-$C_4$-Alkoxy-$C_1$-$C_4$-alkyl; $C_1$-$C_4$-Alkoxycarbonyl-$C_1$-$C_4$-alkyl; $C_1$-$C_4$-Alkylcarbonyl-$C_1$-$C_4$-alkyl; $C_3$-$C_4$-Alkenyloxycarbonyl-$C_1$-$C_4$-alkyl; $C_3$-$C_4$-Alkinyloxycarbonyl-$C_1$-$C_4$-alkyl; Halogen; oder Cyano;

$R^6$ $C_1$-$C_4$-Alkyl; Halogen; Cyano; $C_3$-$C_6$-Cycloalkyl; $C_1$-$C_4$-Halogenalkyl; $C_1$-$C_4$-Alkoxy; Nitro; $C_1$-$C_4$-Alkyl-$S(O)_n$; $C_1$-$C_4$-Halogenalkoxy; $C_1$-$C_4$-Halogenalkyl-$S(O)_n$; oder unsubstituiertes oder bis zu dreifach gleich oder verschieden durch Halogen, $C_1$-$C_4$-Alkyl, $C_1$-$C_4$-Halogenalkyl, $C_1$-$C_4$-Alkyl-$S(O)_n$, $C_1$-$C_4$-Alkoxy, $C_1$-$C_4$-Halogenalkoxy, Nitro oder Cyano substituiertes Phenyl; oder $C_1$-$C_4$-Alkylthio-$C_1$-$C_4$-alkyl;

n 0, 1 oder 2; oder

$R^5$ und $R^6$ gemeinsam mit den beiden Kohlenstoffatomen an welche sie gebunden sind einen anellierten, teilweise ungesättigten 4- bis 8-gliedrigen Ring, der bis zu dreifach gleich

oder verschieden durch $C_1$-$C_4$-Alkyl, Halogen, $C_1$-$C_4$-Alkoxycarbonyl substituiert sein kann und/oder durch O, S oder N-($C_1$-$C_4$)-Alkyl unterbrochen ist und/oder eine Doppelbindung und/oder eine Carbonylgruppe enthalten kann;

dadurch gekennzeichnet, dass es zwischen 0,1 bis 80% Wirkstoff der Formel I, 1 bis 99,9% eines festen oder flüssigen Zusatzstoffes und 0 bis 25% eines Tensides enthält.

10. Verfahren zur Bekämpfung unerwünschten Pflanzenwuchses oder zur Beeinflussung des Pflanzenwuchses, dadurch gekennzeichnet, dass man eine herbizid wirksame oder eine pflanzenwuchsregulatorisch wirksame Menge eines Mittels gemäss Anspruch 9 auf die zu bekämpfende Pflanze oder deren Lebensraum einwirken lässt.,

## Claims

### Claims for the following Contracting States : CH, DE, FR, GB, IT, LI

1. A urea of formula I

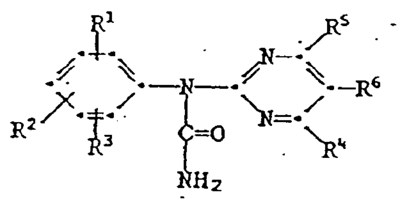

(I)

in which

$R^1$, $R^2$ and $R^3$ are each, independently of the others, hydrogen; nitro; cyano; halogen; $C_1$-$C_4$alkyl; $C_1$-$C_4$alkyl-S(O)$_n$; $C_1$-$C_4$alkoxy; $C_1$-$C_4$haloalkyl; $C_1$-$C_4$haloalkoxy; $C_1$-$C_4$haloalkyl-S(O)$_n$; $C_1$-$C_4$alkoxycarbonyl; $C_1$-$C_4$alkylcarbonyl; aminocarbonyl; mono-$C_1$-$C_4$alkylaminocarbonyl; or di-$C_1$-$C_4$alkylaminocarbonyl;

$R^4$ and $R^5$ are each, independently of the other, hydrogen; $C_1$-$C_4$alkyl; $C_1$-$C_4$alkoxy; $C_1$-$C_4$alkyl-S(O)$_n$; $C_1$-$C_4$haloalkyl; $C_1$-$C_4$haloalkoxy; $C_1$-$C_4$haloalkyl-S(O)$_n$; phenyl that is unsubstituted or is substituted by up to three identical or different substituents from $C_1$-$C_4$alkyl, halogen, $C_1$-$C_4$alkoxy, $C_1$-$C_4$haloalkyl, nitro and cyano; furanyl; thiophenyl; $C_3$-$C_6$cycloalkyl; $C_1$-$C_4$alkoxycarbonyl; $C_1$-$C_4$alkoxy-$C_1$-$C_4$alkyl; $C_1$-$C_4$alkoxycarbonyl-$C_1$-$C_4$alkyl; $C_1$-$C_4$alkylcarbonyl-$C_1$-$C_4$alkyl; $C_3$-$C_4$alkenyloxycarbonyl-$C_1$-$C_4$alkyl; $C_3$-$C_4$alkynyloxycarbonyl-$C_1$-$C_4$-alkyl; halogen; or cyano;

$R^6$ is $C_1$-$C_4$alkyl; halogen; cyano; $C_3$-$C_6$cycloalkyl; $C_1$-$C_4$haloalkyl; $C_1$-$C_4$alkoxy; nitro; $C_1$-$C_4$alkyl-S(O)$_n$; $C_1$-$C_4$haloalkoxy; $C_1$-$C_4$haloalkyl-S(O)$_n$; or phenyl that is unsubstituted or is substituted by up to three identical or different substituents from halogen, $C_1$-$C_4$alkyl, $C_1$-$C_4$haloalkyl, $C_1$-$C_4$alkyl-S(O)$_n$, $C_1$-$C_4$alkoxy, $C_1$-$C_4$haloalkoxy, nitro and cyano; or $C_1$-$C_4$alkylthio-$C_1$-$C_4$alkyl;

n is 0, 1 or 2; or

$R^5$ and $R^6$, together with the two carbon atoms to which they are bonded, are a fused, partially unsaturated, 4-to 8-membered ring which may be substituted by up to three identical or different substituents from $C_1$-$C_4$-alkyl, halogen and $C_1$-$C_4$alkoxycarbonyl and/or is interrupted by O, S or N-($C_1$-$C_4$)alkyl and/or may contain a double bond and/or a carbonyl group;

or a salt thereof with an acid, base or complex former.

2. A urea according to claim 1 of formula Ih

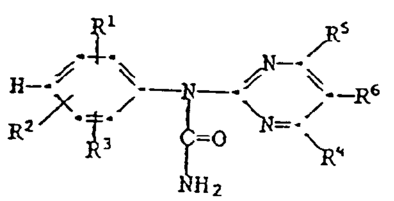

(Ih),

in which

$R^1$ is hydrogen; nitro; cyano; halogen; $C_1$-$C_4$alkyl; $C_1$-$C_4$-alkyl-S(O)$_n$; $C_1$-$C_4$alkoxy; $C_1$-$C_4$haloalkyl; $C_1$-$C_4$haloalkoxy; $C_1$-$C_4$haloalkyl-S(O)$_n$; $C_1$-$C_4$alkoxycarbonyl; $C_1$-$C_4$-alkylcarbonyl; aminocarbonyl; mono-$C_1$-$C_4$alkylaminocarbonyl; or di-$C_1$-$C_4$alkylaminocarbonyl;
$R^2$ is hydrogen; nitro; halogen; $C_1$-$C_4$alkyl; or $C_1$-$C_4$haloalkyl; and
$R^3$ is hydrogen; halogen; or $C_1$-$C_4$alkyl and
$R^4$ to $R^6$ and n are as defined in claim 1.

3. A urea according to claim 1 of formula Ih

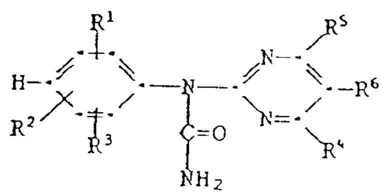

(Ih),

in which
$R^1$ is hydrogen; nitro; cyano; halogen; $C_1$-$C_3$alkyl; $C_1$-$C_2$-alkyl-S(O)$_n$; $C_1$-$C_2$haloalkyl-S(O)$_n$; $C_1$-$C_3$alkoxy; $C_1$-$C_3$-haloalkyl; $C_1$-$C_2$haloalkoxy; $C_1$-$C_3$alkoxycarbonyl; or dimethylaminocarbonyl;
$R^2$ is hydrogen; nitro; fluorine; chlorine; bromine; or $C_1$-$C_3$alkyl;
$R^3$ is hydrogen; chlorine; or $C_1$-$C_3$alkyl;
$R^4$ is hydrogen; $C_1$-$C_4$alkyl; $C_1$-$C_3$alkoxy; $C_1$-$C_4$alkyl-S(O)$_n$-; $C_1$-$C_3$haloalkyl; $C_1$-$C_2$haloalkoxy; phenyl; chlorophenyl; furanyl; $C_3$-$C_6$cycloalkyl; $C_1$-$C_4$alkoxycarbonyl-$C_1$-$C_2$alkyl; $C_1$-$C_2$alkoxy-$C_1$-$C_2$alkyl; $C_1$-$C_2$haloalkylthio; chlorine; cyano; or acetonyl;
$R^5$ is hydrogen; $C_1$-$C_3$alkyl; $C_1$-$C_3$alkoxy; halogen; $C_1$-$C_3$-haloalkyl; or $C_1$-$C_3$haloalkoxy;
$R^6$ is $C_1$-$C_3$alkyl; $C_1$-$C_3$alkoxy; $C_1$-$C_3$haloalkyl; $C_1$-$C_3$haloalkoxy; chlorine; bromine; $C_1$-$C_3$alkylthio; cyano; nitro; phenyl; chlorophenyl; $C_1$-$C_2$alkylthio-$C_1$-$C_2$alkyl; or $C_3$-$C_6$cycloalkyl;
n is 0, 1 or 2;
$R^5$ and $R^6$ together are a $-CH_2CH_2-$, $-CH_2-CH_2-CH_2-$, $-CH_2-CH_2-CH_2-CH_2-$, $-CH_2-CH_2-CO-CH_2-$, $-CH=CH-CH_2-$, $-CH=CH-CH_2-CH_2-$, $-O-CH_2-CH_2-CH_2-$, $-CH_2-CH_2-S-$, $-CH_2-CH_2-S-CH_2-$, $-CH_2-CH_2-O-CH_2-$ or $-CH_2-CH_2-N(C_1-C_3\text{-alkyl})-CH_2-$ bridge, each of which is unsubstituted or is substituted by up to three $C_1$-$C_3$alkyl radicals or by one $C_1$-$C_3$alkoxycarbonyl radical or one chlorine atom.

4. A urea according to claim 1 of formula Ih

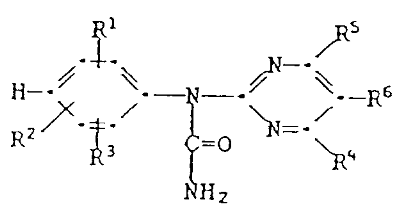

(Ih),

in which
$R^1$ is nitro; cyano; halogen; methyl; ethyl; $C_1$-$C_3$haloalkyl; $C_1$-$C_2$haloalkyl-S(O)$_n$; methoxy; ethoxy; $C_1$-$C_3$haloalkyl; halomethoxy; $C_1$-$C_3$alkoxycarbonyl; or dimethylaminocarbonyl;
n is 0, 1 or 2;
$R^2$ is hydrogen; fluorine; chlorine; bromine; nitro; or methyl;
$R^3$ is hydrogen; chlorine; or methyl;
$R^4$ is $C_1$-$C_4$alkyl; $C_1$-$C_3$alkoxy; $C_1$-$C_4$alkylthio; methylsulfinyl; ethylsulfinyl; methylsulfonyl; ethylsulfonyl; $C_3$-$C_6$cycloalkyl; chlorine; cyano; $C_1$-$C_3$haloalkyl; methoxymethyl; methoxycarbonylmethyl; acetonyl; phenyl; 4-chlorophenyl; or 2-furanyl;
$R^5$ is $C_1$-$C_2$alkyl; chlorine; $C_1$-$C_3$haloalkyl; or halomethoxy;
$R^6$ is $C_1$-$C_3$alkyl; methoxy; ethoxy; $C_1$-$C_2$haloalkyl; chlorine; bromine; $C_1$-$C_3$alkylthio; cyano; nitro; phenyl; 4-chlorophenyl; 2-methylthioethyl; or $C_3$-$C_6$cycloalkyl; or
$R_5$ and $R_6$ together are $-(CH_2)_3-$, $-CHCH_3-CH_2-CH_2-$, $-C(CH_3)_2-CH_2-CH_2-$, $-CH_2-CH(CH_3)-CH_2-$, $-CH_2-CH_2-CH(CH_3)-$, $-CH_2-C(CH_3)_2-CH_2-$, $-CH_2-CH_2-$, $-(CH_2)_4-$, $-CH(CH_3)-(CH_2)_3-$, $-CH_2-CH(CH_3)-(CH_2)_2-$, $-(CH_2)_3-$

CH(CH$_3$)-, -(CH$_2$)$_2$-CH(CH$_3$)-CH$_2$-, -(CH$_2$)$_5$-, -(CH$_2$)$_6$- or -(CH$_2$)$_3$-O-.

5. A urea according to any one of claims 1 to 4 of formula Ia or Ib

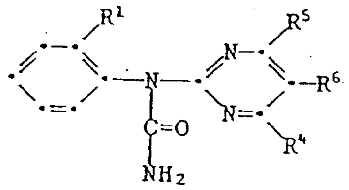

(Ia)

or

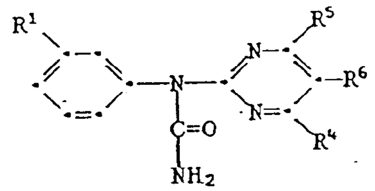

(Ib).

6. A urea according to any one of claims 1 to 4 of formula Ic, Id, Ie or If

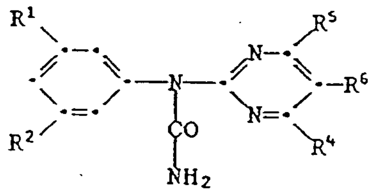

(Ic)

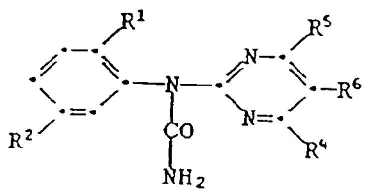

(Id)

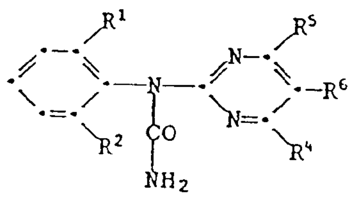

(Ie)

or

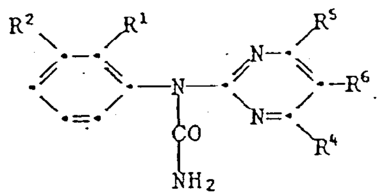

(If).

**7.** A urea according to any one of claims 1 to 4 of formula Ig

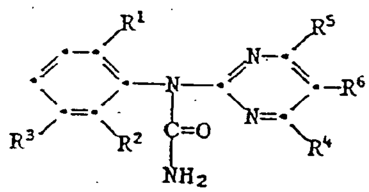

(Ig).

**8.** A process for the preparation of a urea of formula I according to any one of claims 1 to 7 which comprises
a) reacting an aniline of formula II with phosgene to form a carbamoyl chloride of formula III and reacting this with $NH_3$ in a second step

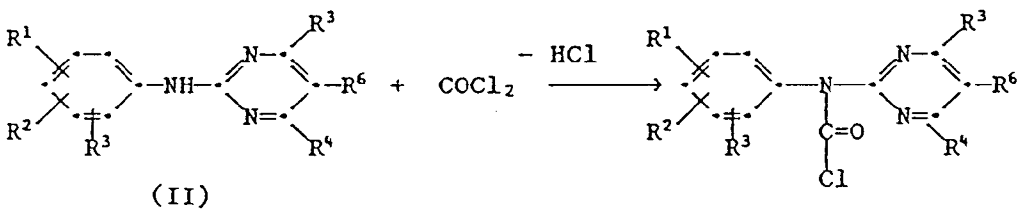


(II) (III)

$$III + NH_3 \xrightarrow{-HCl} I$$

to form a urea of formula I, or
b) reacting an aniline of formula II with a halosulfonyl isocyanate XVIII to form a halosulfonylurea of formula IV

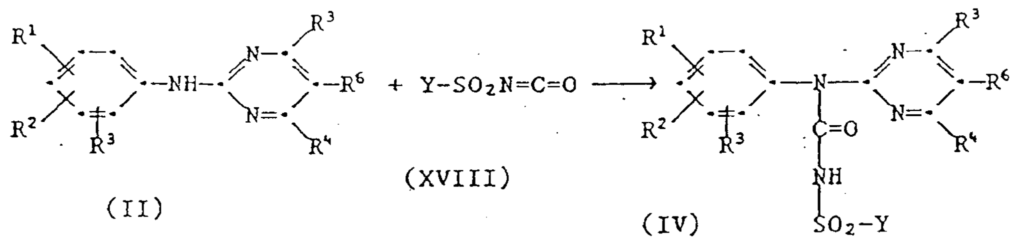


(II) (XVIII) (IV)

$$IV + 2H_2O \xrightarrow[-SO_4H_2]{-HY} I$$

and hydrolysing this in a second step, or directly, to a compound of formula I, Y being a group that can be removed under the reaction conditions, such as halogen, preferably chlorine.

**9.** A process for the preparation of a urea of formula I'

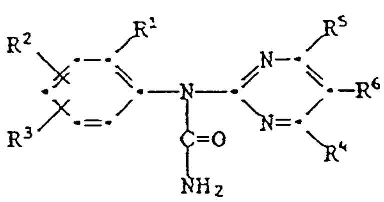

(I')

in which $R^1$ is bonded in the ortho-position of the phenyl ring and represents nitro, and the radicals $R^2$ to

$R^6$ are as defined in any one of claims 1 to 7, which comprises rearranging a sulfonylurea of formula V, under the action of an aqueous base, to form a urea of formula I'

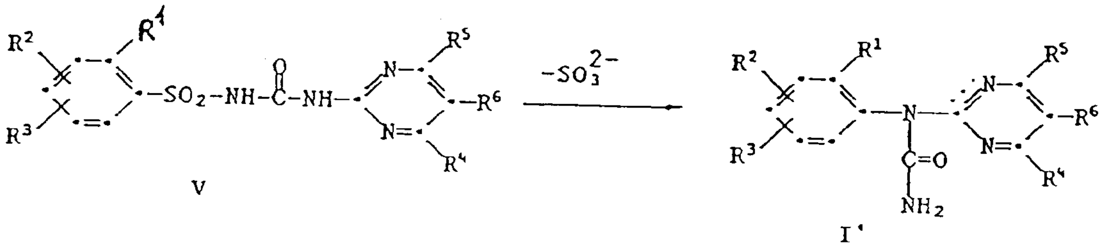


V

I'

**10.** A carbamoyl chloride of formula III

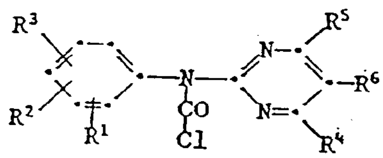

(III),

in which the radicals $R^1$ to $R^6$ are as defined in any one of claims 1 to 7.

**11.** A process for the preparation of a carbamoyl chloride of formula III according to claim 10, which comprises reacting an aniline of formula II with phosgene to form III

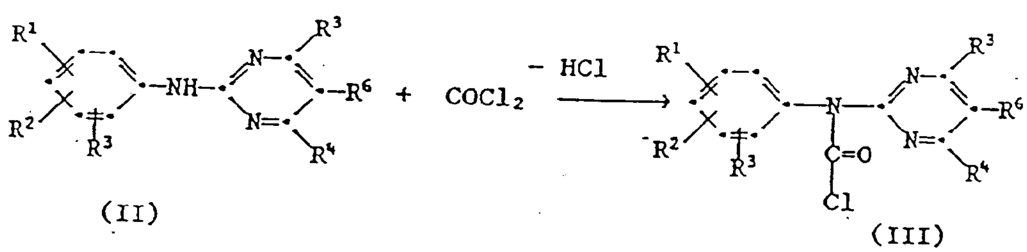


(II) (III)

**12.** A urea of formula IV

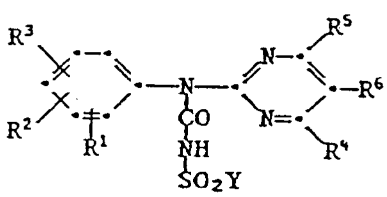

(IV)

in which the radicals $R^1$ to $R^6$ are as defined in any one of claims 1 to 7 and Y is halogen.

**13.** A process for the preparation of a urea according to claim 12, which comprises reacting an aniline of formula II, in which $R^1$ to $R^6$ are as defined in claim 12, with a halosulfonyl isocyanate XVIII, to form a halosulfonylurea of formula IV

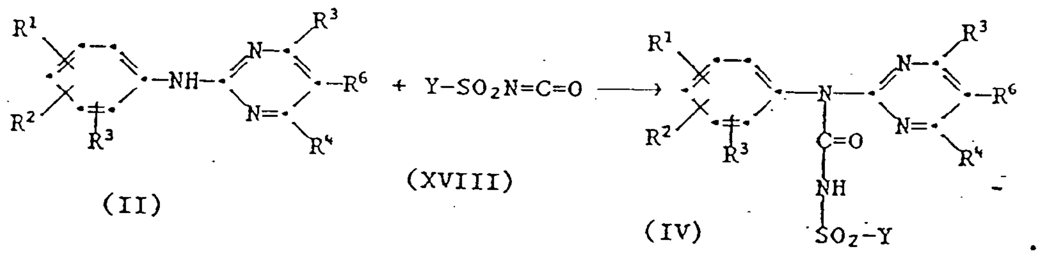

(II) (XVIII) (IV)

14. An isocyanate of formula XIII

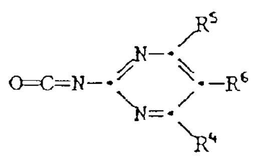

XIII

in which the radicals $R^4$ to $R^6$ are as defined in claim 12.

15. A herbicidal or plant growth-regulating composition comprising as active ingredient a compound of formula I according to any one of claims 1 to 7 together with further adjuvants and/or carriers.

16. A method of controlling undesired plant growth which comprises allowing to act upon the plant to be controlled or the locus thereof a herbicidally effective amount of a compound according to any one of claims 1 to 7 or a composition according to claim 15.

17. A method according to claim 16 for the pre- or post-emergence control of undesired plant growth in crops of useful plants.

18. A method of influencing plant growth which comprises allowing to act upon the plant or the locus thereof an amount that is effective in regulating plant growth of a compound according to any one of claims 1 to 7, or of a composition according to claim 15.

**Claims for the following Contracting State : ES**

1. A process for the preparation of a urea of formula I

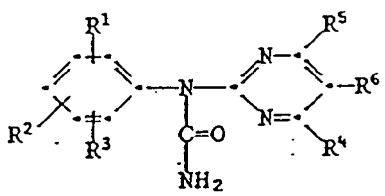

(I)

in which
$R^1$, $R^2$ and $R^3$ are each, independently of the others, hydrogen; nitro; cyano; halogen; $C_1$-$C_4$alkyl; $C_1$-$C_4$alkyl-S(O)$_n$; $C_1$-$C_4$alkoxy; $C_1$-$C_4$haloalkyl; $C_1$-$C_4$haloalkoxy; $C_1$-$C_4$haloalkyl-S(O)$_n$; $C_1$-$C_4$alkoxycarbonyl; $C_1$-$C_4$alkylcarbonyl; aminocarbonyl; mono-$C_1$-$C_4$alkylaminocarbonyl; or di-$C_1$-$C_4$alkylaminocarbonyl;
$R^4$ and $R^5$ are each, independently of the other, hydrogen; $C_1$-$C_4$alkyl; $C_1$-$C_4$alkoxy; $C_1$-$C_4$alkyl-S(O)$_n$; $C_1$-$C_4$haloalkyl; $C_1$-$C_4$haloalkoxy; $C_1$-$C_4$haloalkyl-S(O)$_n$; phenyl that is unsubstituted or is substituted by up to three identical or different substituents from $C_1$-$C_4$alkyl, halogen, $C_1$-$C_4$alkoxy, $C_1$-$C_4$haloalkyl, nitro and cyano; furanyl; thiophenyl; $C_3$-$C_6$cycloalkyl; $C_1$-$C_4$alkoxycarbonyl; $C_1$-$C_4$alkoxy-$C_1$-$C_4$alkyl; $C_1$-$C_4$alkoxycarbonyl-$C_1$-$C_4$alkyl; $C_1$-$C_4$alkylcarbonyl-$C_1$-$C_4$alkyl; $C_3$-$C_4$alkenyloxycarbonyl-$C_1$-$C_4$alkyl; $C_3$-$C_4$alkynyloxycarbonyl-$C_1$-$C_4$-alkyl; halogen; or cyano;
$R^6$ is $C_1$-$C_4$alkyl; halogen; cyano; $C_3$-$C_6$cycloalkyl; $C_1$-$C_4$haloalkyl; $C_1$-$C_4$alkoxy; nitro; $C_1$-$C_4$alkyl-S(O)$_n$;

$C_1$-$C_4$haloalkoxy; $C_1$-$C_4$haloalkyl-$S(O)_n$; or phenyl that is unsubstituted or is substituted by up to three identical or different substituents from halogen, $C_1$-$C_4$alkyl, $C_1$-$C_4$haloalkyl, $C_1$-$C_4$alkyl-$S(O)_n$, $C_1$-$C_4$alkoxy, $C_1$-$C_4$haloalkoxy, nitro and cyano; or $C_1$-$C_4$alkylthio-$C_1$-$C_4$alkyl;

n is 0, 1 or 2; or

$R^5$ and $R^6$, together with the two carbon atoms to which they are bonded; are a fused, partially unsaturated, 4- to 8-membered ring which may be substituted by up to three identical or different substituents from $C_1$-$C_4$alkyl, halogen and $C_1$-$C_4$alkoxycarbonyl and/or is interrupted by O, S or N-($C_1$-$C_4$)alkyl and/or may contain a double bond and/or a carbonyl group;

which comprises

a) reacting an aniline of formula II with phosgene to form a carbamoyl chloride of formula III and reacting this with $NH_3$ in a second step

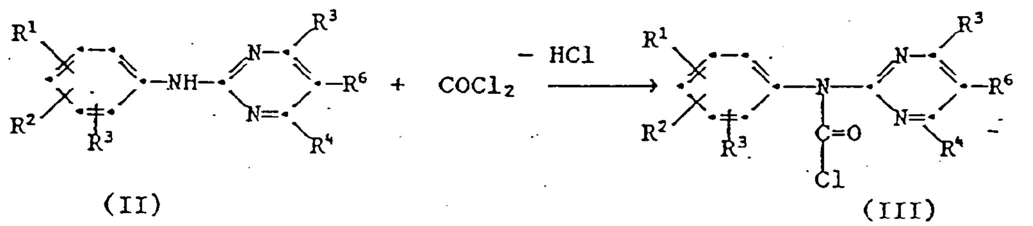

(II) (III)

$$III + NH_3 \xrightarrow{-\ HCl} I$$

to form a urea of formula I, or

b) reacting an aniline of formula II with a halosulfonyl isocyanate XVIII to form a halosulfonylurea of formula IV

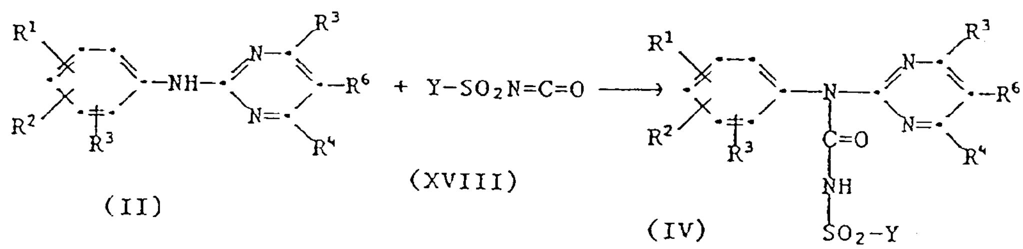

(II) (XVIII) (IV)

$$IV + 2H_2O \xrightarrow[-\ SO_4H_2]{-\ HY} I$$

and hydrolysing this in a second step, or directly, to a compound of formula I, Y being a group that can be removed under the reaction conditions, such as halogen, preferably chlorine.

2. A process according to claim 1 for the preparation of a urea of formula (Ih)

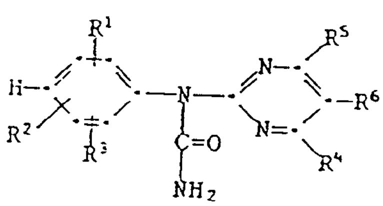

(Ih)

in which
$R^1$ is hydrogen; nitro; cyano; halogen; $C_1$-$C_3$alkyl; $C_1$-$C_2$alkyl-$S(O)_n$; $C_1$-$C_2$haloalkyl-$S(O)_n$; $C_1$-$C_3$alkoxy; $C_1$-$C_3$haloalkyl; $C_1$-$C_2$haloalkoxy; $C_1$-$C_3$alkoxycarbonyl; or dimethylaminocarbonyl;
$R^2$ is hydrogen; nitro; fluorine; chlorine; bromine; or $C_1$-$C_3$alkyl;
$R^3$ is hydrogen; chlorine; or $C_1$-$C_3$alkyl;
$R^4$ is hydrogen; $C_1$-$C_4$alkyl; $C_1$-$C_3$alkoxy; $C_1$-$C_4$alkyl-$S(O)_n$-; $C_1$-$C_3$haloalkyl; $C_1$-$C_2$haloalkoxy; phenyl; chlorophenyl; furanyl; $C_3$-$C_6$cycloalkyl; $C_1$-$C_4$alkoxycarbonyl-$C_1$-$C_2$alkyl; $C_1$-$C_2$alkoxy-$C_1$-$C_2$alkyl; $C_1$-$C_2$haloalkylthio; chlorine; cyano; or acetonyl;
$R^5$ is hydrogen; $C_1$-$C_3$alkyl; $C_1$-$C_3$alkoxy; halogen; $C_1$-$C_3$haloalkyl; or $C_1$-$C_3$haloalkoxy;
$R^6$ is $C_1$-$C_3$alkyl; $C_1$-$C_3$alkoxy; $C_1$-$C_3$haloalkyl; $C_1$-$C_3$haloalkoxy; chlorine; bromine; $C_1$-$C_3$alkylthio; cyano; nitro; phenyl; chlorophenyl; $C_1$-$C_2$alkylthio-$C_1$-$C_2$alkyl; or $C_3$-$C_6$cycloalkyl;
n is 0, 1 or 2;
$R^5$ and $R^6$ together are a $-CH_2CH_2-$, $-CH_2-CH_2-CH_2-$, $-CH_2-CH_2-CH_2-CH_2-$, $-CH_2-CH_2-CO-CH_2-$, $-CH=CH-CH_2-$, $-CH=CH-CH_2-CH_2-$, $-O-CH_2-CH_2-CH_2-$, $-CH_2-CH_2-S-$, $-CH_2-CH_2-S-CH_2-$, $-CH_2-CH_2-O-CH_2-$ or $-CH_2-CH_2-N(C_1$-$C_3alkyl)-CH_2-$ bridge, each of which is unsubstituted or is substituted by up to three $C_1$-$C_3$alkyl radicals or by one $C_1$-$C_3$alkoxycarbonyl radical or one chlorine atom.

3. A process for the preparation of a urea of formula I'

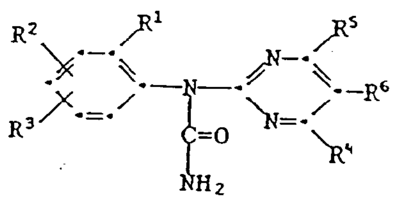

(I')

in which $R^1$ is bonded in the ortho-position of the phenyl ring and represents nitro, and the radicals $R^2$ to $R^6$ are as defined in claim 1 or 2, which comprises rearranging a sulfonylurea of formula V, under the action of an aqueous base, to form a urea of formula I'

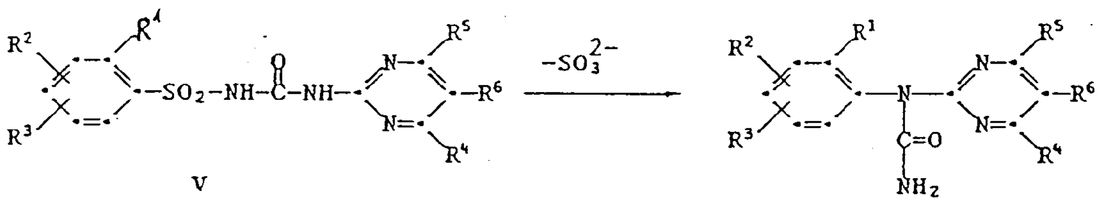


V

I'

4. A process for the preparation of a carbamoyl chloride of formula III

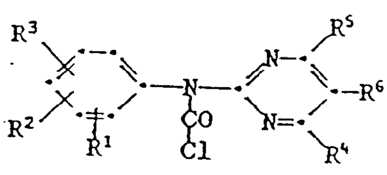

(III),

in which the radicals $R^1$ to $R^6$ are as defined in claim 1 or 2, which comprises reacting an aniline of formula II with phosgene to form III

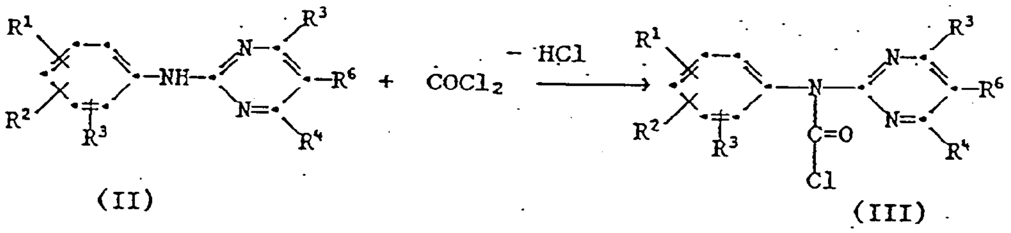


(II) (III)

5. A process for the preparation of a urea of formula IV

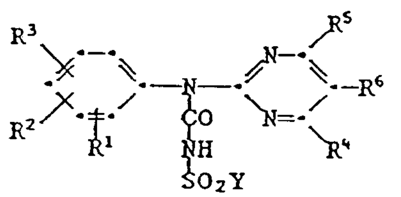

(IV)

in which the radicals $R^1$ to $R^6$ are as defined in claim 1 or 2 and Y is halogen, which comprises reacting an aniline of formula II, in which $R^1$ to $R^6$ are as defined hereinbefore, with a halosulfonyl isocyanate XVIII, to form a halosulfonylurea of formula IV

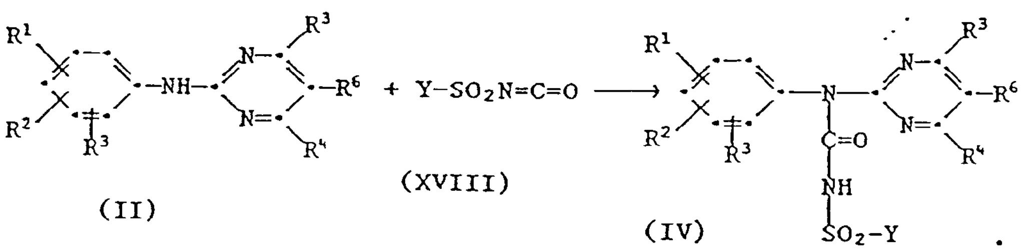

(II) (XVIII) (IV)

6. A method of controlling undesired plant growth or influencing plant growth which comprises allowing to act upon the plant or the locus thereof, in a herbicidally effective amount or in an amount that is effective in plant growth regulation, a urea of formula I

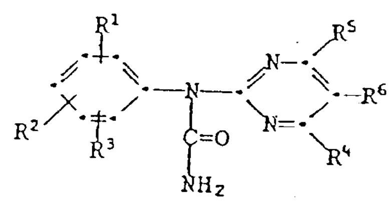

(I)

in which

$R^1$, $R^2$ and $R^3$ are each, independently of the others, hydrogen; nitro; cyano; halogen; $C_1$-$C_4$alkyl; $C_1$-$C_4$alkyl-$S(O)_n$; $C_1$-$C_4$alkoxy; $C_1$-$C_4$haloalkyl; $C_1$-$C_4$haloalkoxy; $C_1$-$C_4$haloalkyl-$S(O)_n$; $C_1$-$C_4$alkoxycarbonyl; $C_1$-$C_4$alkylcarbonyl; aminocarbonyl; mono-$C_1$-$C_4$alkylaminocarbonyl; or di-$C_1$-$C_4$alkylaminocarbonyl;

$R^4$ and $R^5$ are each, independently of the other, hydrogen; $C_1$-$C_4$alkyl; $C_1$-$C_4$alkoxy; $C_1$-$C_4$alkyl-$S(O)_n$; $C_1$-$C_4$haloalkyl; $C_1$-$C_4$haloalkoxy; $C_1$-$C_4$haloalkyl-$S(O)_n$; phenyl that is unsubstituted or is substituted by up to three identical or different substituents from $C_1$-$C_4$alkyl, halogen, $C_1$-$C_4$alkoxy, $C_1$-$C_4$haloalkyl, nitro and cyano; furanyl; thiophenyl; $C_3$-$C_6$cycloalkyl; $C_1$-$C_4$alkoxycarbonyl; $C_1$-$C_4$alkoxy-$C_1$-$C_4$alkyl; $C_1$-$C_4$alkoxycarbonyl-$C_1$-$C_4$alkyl; $C_1$-$C_4$alkylcarbonyl-$C_1$-$C_4$alkyl; $C_3$-$C_4$alkenyloxycarbonyl-$C_1$-$C_4$alkyl; $C_3$-$C_4$alkynyloxycarbonyl-$C_1$-$C_4$alkyl; halogen; or cyano;

$R^6$ is $C_1$-$C_4$alkyl; halogen; cyano; $C_3$-$C_6$cycloalkyl; $C_1$-$C_4$haloalkyl; $C_1$-$C_4$alkoxy; nitro; $C_1$-$C_4$alkyl-$S(O)_n$; $C_1$-$C_4$haloalkoxy; $C_1$-$C_4$haloalkyl-$S(O)_n$; or phenyl that is unsubstituted or is substituted by up to three identical or different substituents from halogen, $C_1$-$C_4$alkyl, $C_1$-$C_4$haloalkyl, $C_1$-$C_4$alkyl-S(O)$_n$, $C_1$-$C_4$alkoxy, $C_1$-$C_4$haloalkoxy, nitro and cyano; or $C_1$-$C_4$alkylthio-$C_1$-$C_4$alkyl;
n is 0, 1 or 2; or
$R^5$ and $R^6$, together with the two carbon atoms to which they are bonded, are a fused, partially unsaturated, 4- to 8-membered ring which may be substituted by up to three identical or different substituents from $C_1$-$C_4$alkyl, halogen and $C_1$-$C_4$alkoxycarbonyl and/or is interrupted by O, S or N-($C_1$-$C_4$)alkyl and/or may contain a double bond and/or a carbonyl group.

7. A method according to claim 6 of controlling undesired plant growth in crops of useful plants.

8. A method according to claim 6 of influencing the growth of cultivated plants.

9. A herbicidal composition comprising a compound of formula I

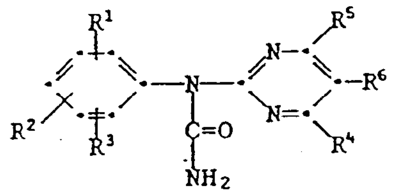

(I)

in which
$R^1$, $R^2$ and $R^3$ are each, independently of the others, hydrogen; nitro; cyano; halogen; $C_1$-$C_4$alkyl; $C_1$-$C_4$alkyl-S(O)$_n$; $C_1$-$C_4$alkoxy; $C_1$-$C_4$haloalkyl; $C_1$-$C_4$haloalkoxy; $C_1$-$C_4$haloalkyl-S(O)$_n$; $C_1$-$C_4$alkoxycarbonyl; $C_1$-$C_4$alkylcarbonyl; aminocarbonyl; mono-$C_1$-$C_4$alkylaminocarbonyl; or di-$C_1$-$C_4$alkylaminocarbonyl;
$R^4$ and $R^5$ are each, independently of the other, hydrogen; $C_1$-$C_4$alkyl; $C_1$-$C_4$alkoxy; $C_1$-$C_4$alkyl-S(O)$_n$; $C_1$-$C_4$haloalkyl; $C_1$-$C_4$haloalkoxy; $C_1$-$C_4$haloalkyl-S(O)$_n$; phenyl that is,unsubstituted or is substituted by up to three identical or different substituents from $C_1$-$C_4$alkyl, halogen, $C_1$-$C_4$alkoxy, $C_1$-$C_4$haloalkyl, nitro and cyano; furanyl; thiophenyl; $C_3$-$C_6$cycloalkyl; $C_1$-$C_4$alkoxycarbonyl; $C_1$-$C_4$alkoxy-$C_1$-$C_4$alkyl; $C_1$-$C_4$alkoxycarbonyl-$C_1$-$C_4$alkyl; $C_1$-$C_4$alkylcarbonyl-$C_1$-$C_4$alkyl; $C_3$-$C_4$alkenyloxycarbonyl-$C_1$-$C_4$alkyl; $C_3$-$C_4$alkynyloxycarbonyl-$C_1$-$C_4$alkyl; halogen; or cyano;
$R^6$ is $C_1$-$C_4$alkyl; halogen; cyano; $C_3$-$C_6$cycloalkyl; $C_1$-$C_4$haloalkyl; $C_1$-$C_4$alkoxy; nitro; $C_1$-$C_4$alkyl-S(O)$_n$; $C_1$-$C_4$haloalkoxy; $C_1$-$C_4$haloalkyl-S(O)$_n$; or phenyl that is unsubstituted or is substituted by up to three identical or different substituents from halogen, $C_1$-$C_4$alkyl, $C_1$-$C_4$haloalkyl, $C_1$-$C_4$alkyl-S(O)$_n$, $C_1$-$C_4$alkoxy, $C_1$-$C_4$haloalkoxy, nitro and cyano; or $C_1$-$C_4$alkylthio-$C_1$-$C_4$alkyl;
n is 0, 1 or 2; or
$R^5$ and $R^6$, together with the two carbon atoms to which they are bonded, are a fused, partially unsaturated, 4- to 8-membered ring which may be substituted by up to three identical or different substituents from $C_1$-$C_4$alkyl, halogen and $C_1$-$C_4$alkoxycarbonyl and/or is interrupted by O, S or N-($C_1$-$C_4$)alkyl and/or may contain a double bond and/or a carbonyl group;
which comprises from 0.1 to 80 % of a compound of formula I, from 1 to 99.9 % of a solid or liquid adjuvant and from 0 to 25 % of a surfactant.

10. A method of controlling undesired plant growth or of influencing plant growth which comprises allowing to act upon the plant to be controlled or upon the locus thereof an amount that is herbicidally effective or is effective in regulating plant growth of a composition according to claim 9.

## Revendications

### Revendications pour les Etats contractants suivants : CH, DE, FR, GB, IT, LI

1. Urées de formule I

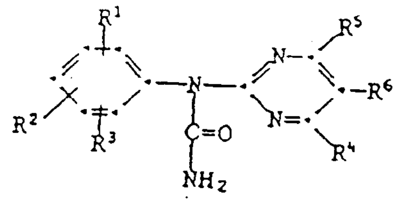

(I),

dans laquelle

$R^1$, $R^2$ et $R^3$ représentent indépendamment l'un de l'autre un hydrogène ; nitro ; cyano ; halogène ; alcoyle en $C_{1-4}$ ; alcoyle en $C_{1-4}S(O)_n$ ; alcoxy en $C_{1-4}$, halogènalcoyle en $C_{1-4}$ ; halogènalcoxy en $C_{1-4}$ ; halogènalcoyle en $C_{1-4}$-$S(O)_n$ ; alcoxycarbonyle en $C_{1-4}$ ; alcoylcarbonyle en $C_{1-4}$ ; aminocarbonyle ; mono-alcoyle en $C_{1-4}$-amino-carbonyle ; ou dialcoyle en $C_{1-4}$-aminocarbonyle ;

$R^4$ et $R^5$ représentent indépendamment l'un de l'autre un hydrogène ; alcoyle en $C_{1-4}$ ; alcoxy en $C_{1-4}$ ; alcoyle en $C_{1-4}$-$S(O)_n$ ; halogènalcoyle en $C_{1-4}$ ; halogènalcoxy en $C_{1-4}$ ; halogènalcoyle en $C_{1-4}$-$S(O)_n$ ; un phényle non-substitué ou jusqu'à trois fois substitué, de façon identique ou différente, par alcoyle en $C_{1-4}$, halogène, alcoxy en $C_{1-4}$, halogènalcoyle en $C_{1-4}$, nitro ou cyano ; furanyle ; thiophényle ; cycloalcoyle en $C_{3-6}$ ; alcoxy en $C_{1-4}$-carbonyle ; alcoxy en $C_{1-4}$-alcoyle en $C_{1-4}$ ; alcoxy en $C_{1-4}$-carbonyle-alcoyle en $C_{1-4}$ ; alcoyle en $C_{1-4}$-carbonyle-alcoyle en $C_{1-4}$ ; alcényloxy en $C_{3-4}$-carbonyle-alcoyle en $C_{1-4}$ ;-alcynyloxy en $C_{3-4}$-carbonyle-alcoyle en $C_{1-4}$ ; halogène ; ou cyano ;

$R^6$ représente un alcoyle en $C_{1-4}$ ; halogène, cyano ; cycloalcoyle en $C_{3-6}$ ; halogènalcoyle en $C_{1-4}$ ; alcoxy en $C_{1-4}$ ; nitro ; alcoyle en $C_{1-4}$-$S(O)_n$ ; halogènalcoxy en $C_{1-4}$ ; halogènalcoyle en $C_{1-4}$-$S(O)_n$ ; ou un phényle substitué jusqu'à trois fois de manière identique ou différente par un halogène, alcoyle en $C_{1-4}$, halogènalcoyle en $C_{1-4}$, alcoyle en $C_{1-4}$-$S(O)_n$, alcoxy en $C_{1-4}$, halogènalcoxy en $C_{1-4}$, nitro ou cyano ; ou un alcoyle en $C_{1-4}$-thio-alcoyle en $C_{1-4}$ ;

n vaut 0, 1 ou 2 ; ou

$R^5$ et $R^6$ représentent ensemble avec les deux atomes de carbone auxquels ils sont liés un noyau condensé, partiellement insaturé, à 4 à 8 chaînons, qui peut être substitué jusqu'à trois fois de manière identique ou différente par un alcoyle en $C_{1-4}$, un halogène, un alcoxy en $C_{1-4}$-carbonyle et/ou être interrompu par O, S ou N-alcoyle en $C_{1-4}$, et/ou contenir une double liaison, et/ou un groupe carbonyle ;

y compris leurs sels avec des acides, des bases et des formateurs de complexes.

2. Urées selon la revendication 1, de formule Ih :

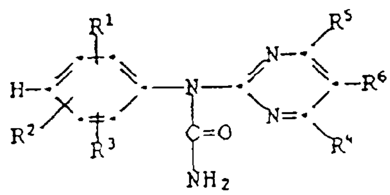

(Ih),

où

$R^1$ représente un hydrogène ; nitro ; cyano ; halogène ; alcoyle en $C_{1-4}$, alcoyle en $C_{1-4}$-$S(O)_n$ ; alcoxy en $C_{1-4}$ ; halogènalcoyle en $C_{1-4}$ ; halogènalcoxy en $C_{1-4}$ ; halogènalcoyle en $C_{1-4}$-$S(O)_n$ ; alcoxy en $C_{1-4}$-carbonyle ; alcoyle en $C_{1-4}$-carbonyle ; aminocarbonyle ; mono-alcoyle en $C_{1-4}$-aminocarbonyle ; ou di-alcoyle en $C_{1-4}$-aminocarbonyle;

$R^2$ représente un hydrogène ; nitro, halogène ; alcoyle en $C_{1-4}$ ; ou halogèneakoyle en $C_{1-4}$ ; et

$R^3$ représente un hydrogène ; halogène ; ou alcoyle en $C_{1-4}$;

et

$R^4$ à $R^6$ et n sont tels que définis dans la revendication 1.

3. Urées selon la revendication 1, de formule Ih :

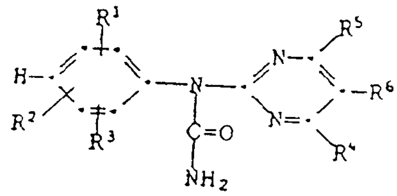

(Ih),

où

$R^1$ représente un hydrogène ; nitro ; cyano ; halogène ; alcoyle en $C_{1-3}$ ; alcoyle en $C_{1-2}$-$S(O)_n$ ; halogènalcoyle en $C_{1-2}$-$S(O)_n$ ; alcoxy en $C_{1-3}$ ; halogènalcoyle en $C_{1-3}$; halogènalcoxy en $C_{1-2}$ ; alcoxycarbonyle en $C_{1-3}$ ; ou diméthylaminocarbonyle ;

$R^2$ représente un hydrogène ; nitro ; fluor ; brome ; ou alcoyle en $C_{1-3}$ ;

$R^3$ représente un hydrogène ; chloro ; ou alcoyle en $C_{1-3}$ ;

$R^4$ représente un hydrogène ; alcoyle en $C_{1-4}$ ; alcoxy en $C_{1-3}$ ; alcoyle en $C_{1-4}$-$S(O)_n$ ; halogènalcoyle en $C_{1-3}$ ; halogènalcoxy en $C_{1-2}$ ; phényle ; chlorophényle ; furanyle ; cycloalcoyle en $C_{3-6}$, alcoxy en en $C_{1-4}$-carbonyle-alcoyle en $C_{1-2}$ ; alcoxy en $C_{1-2}$-alcoyle en $C_{1-2}$ ; halogènalcoylthio en $C_{1-2}$ ; chlore ; cyano ; ou acétonyle ;

$R^5$ représente un hydrogène ; alcoyle en $C_{1-3}$ ; alcoxy en $C_{1-3}$ ; halogène ; halogènalcoyle en $C_{1-3}$ ; ou halogènalcoxy en $C_{1-3}$ ;

$R^6$ représeente un alcoyle en $C_{1-3}$ ; alcoxy en $C_{1-3}$ ; halogènalcoyle en $C_{1-3}$ ; halogènalcoxy en $C_{1-3}$ ; chlore, brome ; alcoylthio en $C_{1-3}$ ; cyano ; nitro ; phényle ; chlorophényle ; alcoylthio en $C_{1-2}$-alcoyle en $C_{1-2}$ ; ou cycloalcoyle en $C_{3-6}$ ;

n vaut 0, 1 ou 2 ;

$R^5$ et $R^6$ représentent ensemble un pont $-CH_2CH_2-$, $-CH_2-CH_2-CH_2-$, $-CH_2-CH_2-CH_2-CH_2-$, $-CH_2-CH_2-CO-CH_2-$, $-CH=CH-CH_2-$, $-CH=CH-CH_2-CH_2-$, $-O-CH_2-CH_2-CH_2-$, $-CH_2-CH_2-S-$, $-CH_2-CH_2-S-CH_2-$, $-CH_2-CH_2-O-CH_2-$ ou $-CH_2-CH_2-N$(alcoyle en $C_{1-3}$)$-CH_2-$ jusqu'à trois fois substitué par un alcoyle en $C_{1-3}$ ou le cas échéant monosubstitué par un alcoxy en $C_{1-3}$-carbonyle ou un chlore.

4. Urées selon la revendication 1, de formule Ih :

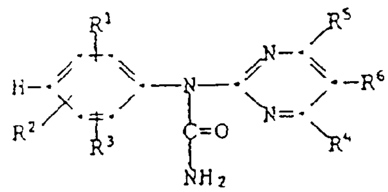

(Ih),

dans laquelle

$R^1$ représente un nitro ; cyano ; halogène ; méthyle ; éthyle ; halogènalcoyle en $C_{1-3}$ ; halogènalcoyle en $C_{1-2}$-$S(O)_n$ ; méthoxy ; éthoxy ; halogènalcoyle en $C_{1-3}$ ; halogèneméthoxy ; alcoxy en $C_{1-3}$-carbonyle ; ou diméthylaminocarbonyle ;

n vaut 0, 1 ou 2 ;

$R^2$ représente un hydrogène ; fluor ; chlore ; brome ; nitro ; ou méthyle ;

$R^3$ représente un hydrogène ; chlore ; ou méthyle ;

$R^4$ représente un alcoyle en $C_{1-4}$, alcoxy en $C_{1-3}$ ; alcoylthio en $C_{1-4}$ ; méthylsulfinyle ; éthylsulfinyle ; méthylsulfonyle ; éthylsulfonyle ; cycloalcoyle en $C_{3-6}$ ; chlore ; cyano ; halogènalcoyle en $C_{1-3}$ ; méthoxyméthyle ; méthoxycarbonylméthyle ; acétonyle ; phényle ; 4-chlorophényle ; ou 2-furanyle ;

$R^5$ représente un alcoyle en $C_{1-2}$ ; chlore ; haloalcoyle en $C_{1-3}$ ; ou halogèneméthoxy ;

$R^6$ représente un alcoyle en $C_{1-3}$ ; méthoxy ; éthoxy ; haloalcoyle en $C_{1-2}$ ; chlore ; brome ; alcoylthio en $C_{1-3}$ ; cyano ; nitro ; phényle ; 4-chlorophényle ; 2-méthylthioéthyle ; ou cycloalcoyle en $C_{3-6}$ ; ou

$R^5$ et $R^6$ représentent ensemble $-(CH_2)_3-$, $-CHCH_3-CH_2-CH_2-$, $-C(CH_3)_2-CH_2-CH_2-$, $-CH_2-CH(CH_3)-CH_2-$, $-CH_2-CH_2-CH(CH_3)-$, $-CH_2-C(CH_3)_2-CH_2-$, $-CH_2-CH_2-$, $-(CH_2)_4-$, $-CH(CH_3)-(CH_2)_3-$, $-CH_2-CH(CH_3)-(CH_2)_2-$, $-(CH_2)_3-CH(CH_3)-$, $-(CH_2)_2-CH(CH_3)-CH_2-$, $-(CH_2)_5-$, $-(CH_2)_6-$ ou $-(CH_2)_3-O-$.

5. Urées selon l'une des revendications 1 à 4 de formules Ia ou Ib :

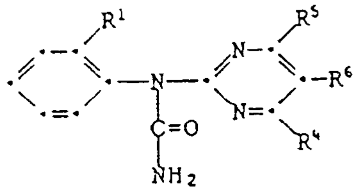

(Ia)

ou

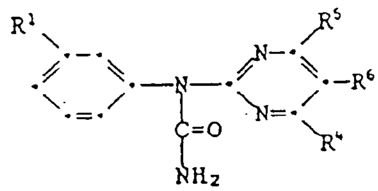

(Ib).

6. Urées selon l'une des revendications 1 à 4, de formules Ic, Id, Ie ou If :

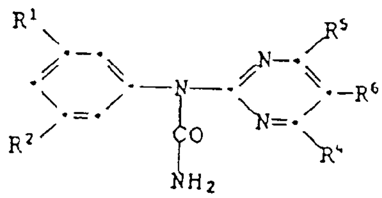

(Ic)

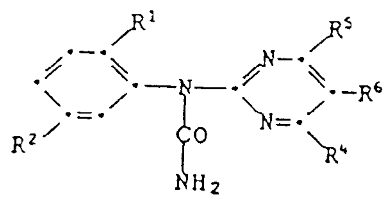

(Id)

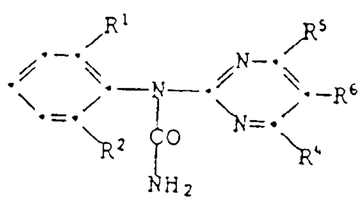

(Ie)

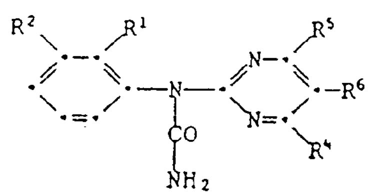

(If).

7. Urées selon l'une des revendications 1 à 4 de formule Ig

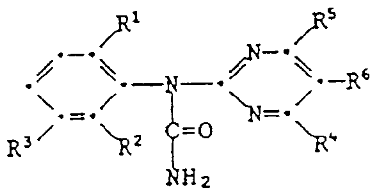

(Ig).

8. Procédé de préparation d'urées de formule I selon l'une des revendications 1 à 7, caractérisé en ce qu'on procède

a) en faisant réagir une aniline de formule II avec du phosgène pour donner un chlorure de carbamine de formule III et en faisant réagir celui-ci dans une seconde étape avec $NH_3$ pour donner

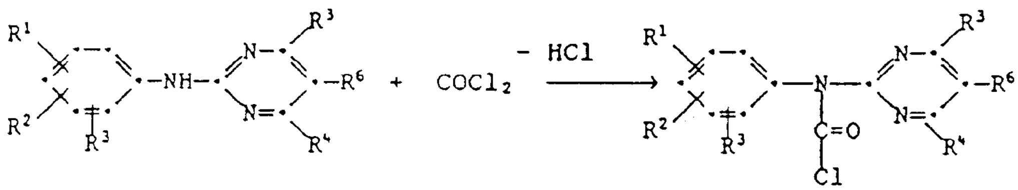


(II) (III)

$$III + NH_3 \xrightarrow{-HCl} I$$

ou urée de formule I ou

b) en faisant réagir une aniline de formule II avec un isocyanate d'halogènesulfonyle XVIII pour donner une halogènesulfonylurée de formule IV

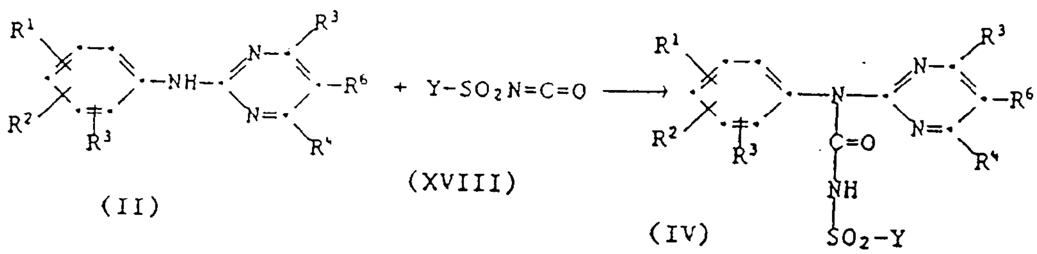


(II) (XVIII) (IV)

$$IV + 2H_2O \xrightarrow[-SO_4H_2]{-HY} I$$

et dans une seconde étape en hydrolysant directement celle-ci pour donner un composé de formule I, où Y représente un groupe séparable dans les conditions de la réaction, comme halogène, de préférence chlore.

9. Procédé de préparation d'urées de formule I'

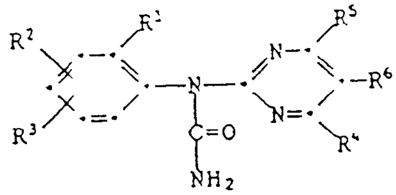

(I').

où $R^1$ est lié en position ortho du noyau phényle et représente un nitro, et les radicaux $R^2$ à $R^6$ sont tels que définis dans les revendications 1 à 7, caractérisé en ce qu'on transpose une sulfonylurée de formule V en faisant agir une base aqueuse pour donner une urée de formule I' :

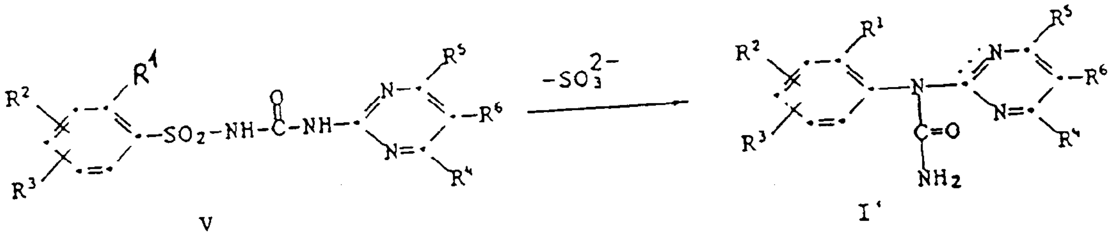


V

I'

**10.** Chlorures de carbamoyle de formule III

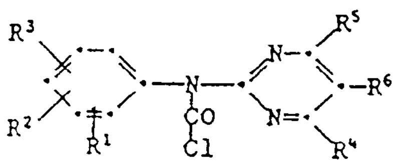

(III),

où les radicaux $R^1$ à $R^6$ sont tels que définis dans l'une des revendications 1 à 7.

**11.** Procédé de préparation de chlorures de carbamoyle de formule III selon la revendication 10, caractérisé en ce qu'on fait réagir une aniline de formule II avec du phosgène pour donner III

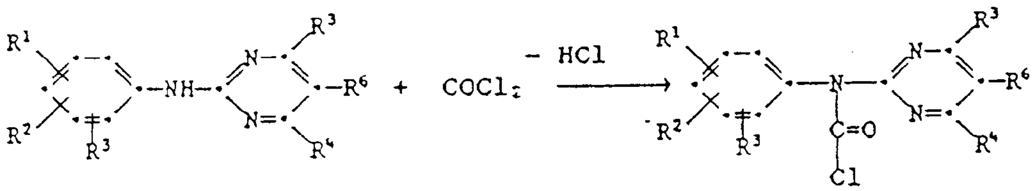


(II)

(III)

**12.** Urées de formule IV

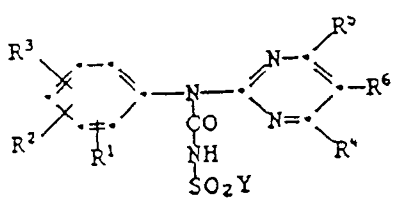

(IV),

où les radicaux $R^1$ à $R^6$ sont tels que définis dans l'une des revendications 1 à 7 et Y représente un halogène.

**13.** Procédé de préparation d'urées selon la revendication 12, caractérisé en ce qu'on fait réagir une aniline de formule II où $R^1$ à $R^6$ sont tels que définis dans la revendication 12, avec un isocyanate d'halogènesulfonyle XVIII, pour donner une halogènesulfonylurée de formule IV

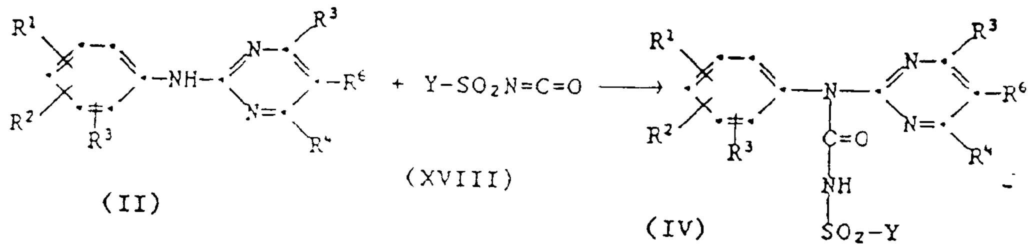

(II) (XVIII) (IV)

**14.** Isocyanates de formule XIII

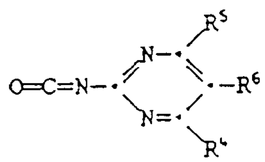

XIII,

où les radicaux $R^4$ à $R^6$ sont tels que définis dans la revendication 12.

**15.** Agents herbicides de régulation de la croissance des plantes contenant comme matière active un composé de formule I selon l'une des revendications 1 à 7, outre d'autres additifs et/ou supports.

**16.** Procédé pour combattre la croissance végétale indésirable, caractérisé en ce qu'on fait agir une quantité herbicide d'un composé selon l'une des revendications 1 à 7, ou d'un agent selon la revendication 15 sur les plantes à traiter ou leur habitat.

**17.** Procédé selon la revendication 16 de lutte en pré- ou en post-levée contre la croissance indésirable dans les cultures de plantes utiles.

**18.** Procédé pour influer sur la croissance végétale, caractérisé en ce qu'on fait agir une quantité à action de régulation de la croissance des plantes d'un composé selon l'une des revendications 1 à 7 ou d'un agent selon la revendication 15, sur les plantes ou leur habitat.

**Revendications pour l'Etat contractant suivant : ES**

**1.** Procédé de préparation d'urées de formule I

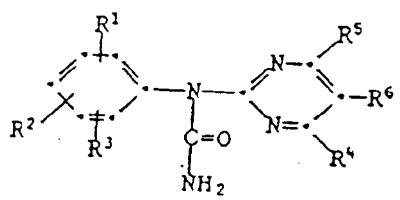

(I),

dans laquelle

$R^1$, $R^2$ et $R^3$ représentent indépendamment l'un de l'autre un hydrogène ; nitro ; cyano ; halogène ; alcoyle en $C_{1-4}$ ; alcoyle en $C_{1-4}$-S(O)$_n$ ; alcoxy en $C_{1-4}$, halogènalcoyle en $C_{1-4}$ ; halogènalcoxy en $C_{1-4}$ ; halogènalcoyle en $C_{1-4}$-S(O)$_n$ ; alcoxycarbonyle en $C_{1-4}$ ; alcoylcarbonyle en $C_{1-4}$ ; aminocarbonyle ; mono-alcoyle en $C_{1-4}$-amino-carbonyle ; ou dialcoyle en $C_{1-4}$-aminocarbonyle ;

$R^4$ et $R^5$ représentent indépendamment l'un de l'autre un hydrogène ; alcoyle en $C_{1-4}$ ; alcoxy en $C_{1-4}$ ; alcoyle en $C_{1-4}$-$S(O)_n$ ; halogènalcoyle en $C_{1-4}$ ; halogènalcoxy en $C_{1-4}$ ; halogènalcoyle en $C_{1-4}$-$S(O)_n$ ; un phényle non-substitué ou jusqu'à trois fois substitué, de façon identique ou différente, par alcoyle en $C_{1-4}$, halogène, alcoxy en $C_{1-4}$, halogènalcoyle en $C_{1-4}$, nitro ou cyano ; furanyle ; thiophényle ; cycloalcoyle en $C_{3-6}$ ; alcoxy en $C_{1-4}$-carbonyle ; alcoxy en $C_{1-4}$-alcoyle en $C_{1-4}$ ; alcoxy en $C_{1-4}$-carbonyle-alcoyle en $C_{1-4}$ ; alcoyle en $C_{1-4}$-carbonyle-alcoyle en $C_{1-4}$ ; alcényloxy en $C_{3-4}$-carbonyle-alcoyle en $C_{1-4}$ ; alcynyloxy en $C_{3-4}$-carbonyle-alcoyle en $C_{1-4}$ ; halogène ; ou cyano ;

$R^6$ représente un alcoyle en $C_{1-4}$ ; halogène, cyano ; cycloalcoyle en $C_{3-6}$ ; halogènalcoyle en $C_{1-4}$ ; alcoxy en $C_{1-4}$ ; nitro ; alcoyle en $C_{1-4}$-$S(O)_n$ ; halogènalcoxy en $C_{1-4}$ ; halogènalcoyle en $C_{1-4}$-$S(O)_n$ ; ou un phényle substitué jusqu'à trois fois de manière identique ou différente par un halogène, alcoyle en $C_{1-4}$, halogènalcoyle en $C_{1-4}$, alcoyle en $C_{1-4}$-$S(O)_n$, alcoxy en $C_{1-4}$, halogènalcoxy en $C_{1-4}$, nitro ou cyano ; ou un alcoyle en $C_{1-4}$-thio-alcoyle en $C_{1-4}$ ;

n vaut 0, 1 ou 2 ; ou

$R^5$ et $R^6$ représentent ensemble avec les deux atomes de carbone auxquels ils sont liés un noyau condensé, partiellement insaturé, à 4 à 8 chaînons, qui peut être substitué jusqu'à trois fois de manière identique ou différente par un alcoyle en $C_{1-4}$, un halogène, un alcoxy en $C_{1-4}$-carbonyle et/ou être interrompu par O, S ou N-alcoyle en $C_{1-4}$, et/ou contenir une double liaison, et/ou un groupe carbonyle ;

y compris leurs sels avec des acides, des bases et des formateurs de complexes,

caractérisé en ce qu'on procède

a) en faisant réagir une aniline de formule II avec du phosgène pour donner un chlorure de carbamine de formule III et en faisant réagir celui-ci dans une seconde étape avec $NH_3$ pour donner

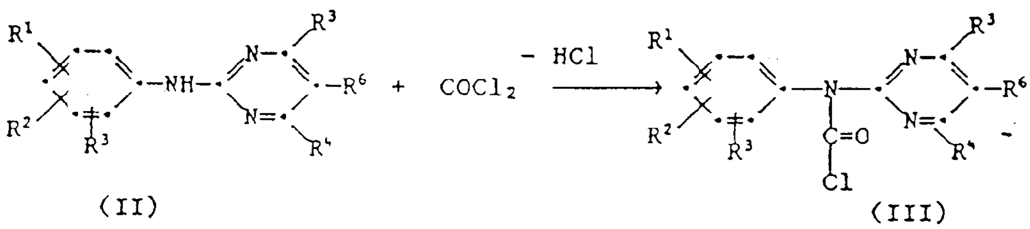


(II) (III)

$$III + NH_3 \xrightarrow{-HCl} I$$

ou urée de formule I ou

b) en faisant réagir une aniline de formule II avec un isocyanate d'halogènesulfonyle XVIII pour donner une halogènesulfonylurée de formule IV et

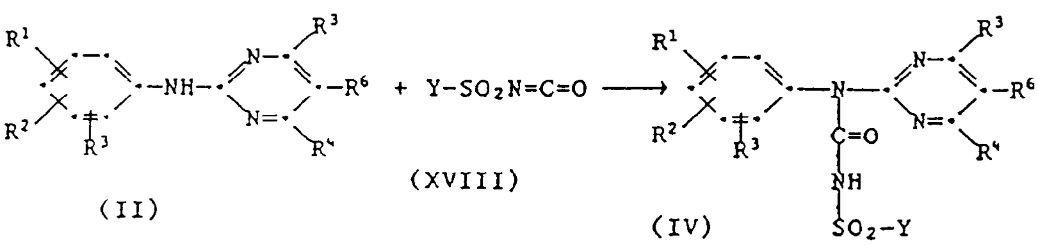


(II) (XVIII) (IV)

$$IV + 2H_2O \xrightarrow[-SO_4H_2]{-HY} I$$

et dans une seconde étape en hydrolysant directement celle-ci pour donner un composé de formule I, où Y représente un groupe séparable dans les conditions de la réaction, comme halogène, de préférence chlore.

2. Procédé selon la revendication 1, de préparation d'urées de formule Ih

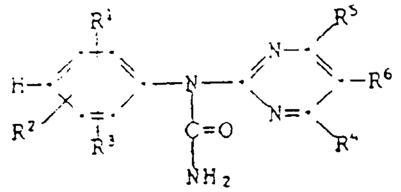

(Ih)

,

où

$R^1$ représente un hydrogène ; nitro ; cyano ; halogène ; alcoyle en $C_{1-3}$ ; alcoyle en $C_{1-2}$-$S(O)_n$ ; halogènalcoyle en $C_{1-2}$-$S(O)_n$ ; alcoxy en $C_{1-3}$ ; halogènalcoyle en $C_{1-3}$ ; halogènalcoxy en $C_{1-2}$ ; alcoxycarbonyle en $C_{1-3}$ ; ou diméthylaminocarbonyle ;

$R^2$ représente un hydrogène ; nitro ; fluor ; brome ; ou alcoyle en $C_{1-3}$ ;

$R^3$ représente un hydrogène ; chloro ; ou alcoyle en $C_{1-3}$ ;

$R^4$ représente un hydrogène ; alcoyle en $C_{1-4}$ ; alcoxy en $C_{1-3}$ ; alcoyle en $C_{1-4}$-$S(O)_n$ ; halogènalcoyle en $C_{1-3}$ ; halogènalcoxy en $C_{1-2}$ ; phényle ; chlorophényle ; furanyle ; cycloalcoyle en $C_{3-6}$, alcoxy en en $C_{1-4}$-carbonyle-alcoyle en $C_{1-2}$ ; alcoxy en $C_{1-2}$-alcoyle en $C_{1-2}$ ; halogènalcoylthio en $C_{1-2}$ ; chlore ; cyano ; ou acétonyle ;

$R^5$ représente un hydrogène ; alcoyle en $C_{1-3}$ ; alcoxy en $C_{1-3}$ ; halogène ; halogènalcoyle en $C_{1-3}$ ; ou halogènalcoxy en $C_{1-3}$ ;

$R^6$ représeente un alcoyle en $C_{1-3}$ ; alcoxy en $C_{1-3}$ ; halogènalcoyle en $C_{1-3}$ ; halogènalcoxy en $C_{1-3}$ ; chlore, brome ; alcoylthio en $C_{1-3}$ ; cyano ; nitro ; phényle ; chlorophényle ; alcoylthio en $C_{1-2}$-alcoyle en $C_{1-2}$ ; ou cycloalcoyle en $C_{3-6}$ ;

n vaut 0, 1 ou 2 ;

$R^5$ et $R^6$ représentent ensemble un pont $-CH_2CH_2-$, $-CH_2-CH_2-CH_2-$, $-CH_2-CH_2-CH_2-CH_2-$, $-CH_2-CH_2-CO-CH_2-$, $-CH=CH-CH_2-$, $-CH=CH-CH_2-CH_2-$, $-O-CH_2-CH_2-CH_2-$, $-CH_2-CH_2-S-$, $-CH_2-CH_2-S-CH_2-$, $-CH_2-CH_2-O-CH_2-$ ou $-CH_2-CH_2-N$(alcoyle en $C_{1-3}$)$-CH_2-$ jusqu'à trois fois substitué par un alcoyle en $C_{1-3}$ ou le cas échéant monosubstitué par un alcoxy en $C_{1-3}$-carbonyle ou un chlore.

3. Procédé de préparation d'urées de formule I'

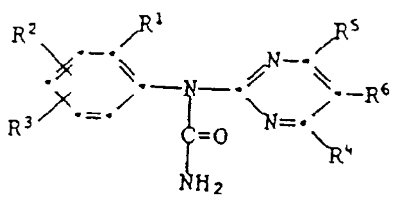

(I').

où $R^1$ est lié en position ortho du noyau phényle et représente un nitro, et les radicaux $R^2$ à $R^6$ sont tels que définis dans les revendications 1 à 7, caractérisé en ce qu'on transpose une sulfonylurée de formule V en faisant agir une base aqueuse pour donner une urée de formule I' :

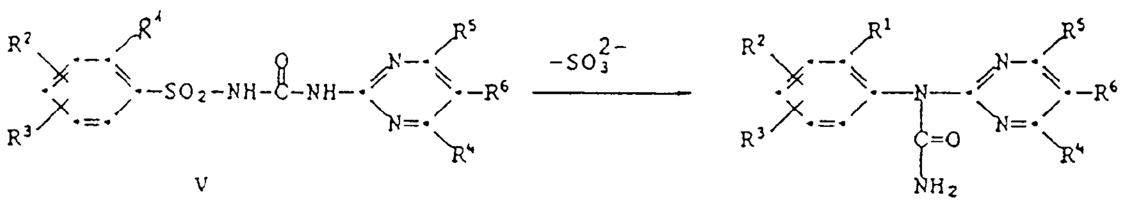


V

I'

4. Procédé de préparation de chlorure de carbamoyle de formule III

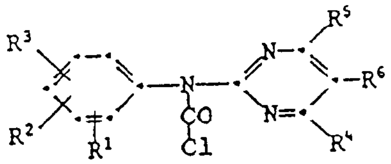

(III),

où les radicaux $R^1$ à $R^6$ sont tels que définis dans la revendication 1 ou 2, caractérisé en ce qu'on fait réagir une aniline de formule II avec du phosgène pour donner III

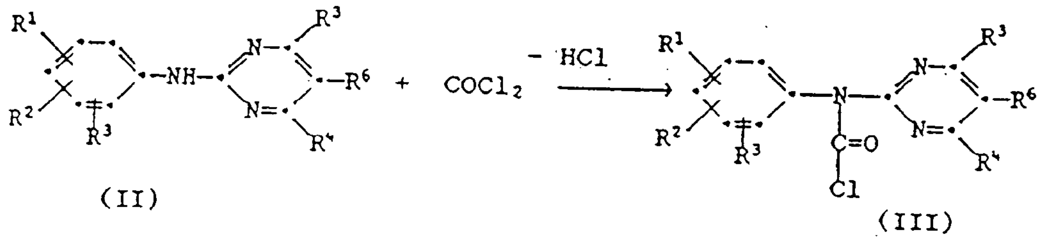


(II)

(III)

5. Procédé de préparation d'urées de formule IV

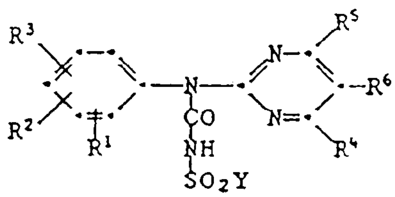

(IV),

où les radicaux $R^1$ à $R^6$ sont tels que définis dans la revendication 1 ou 2 et Y représente un halogène, caractérisé en ce qu'on fait réagir une aniline de formule II où $R^1$ à $R^6$ sont tels que définis ci-dessus, avec un isocyanate d'halogènesulfonyle XVIII, pour donner une halogènesulfonylurée de formule IV

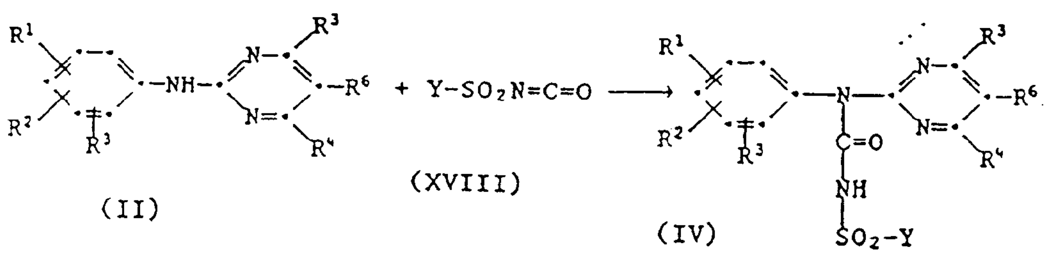


(II)

(XVIII)

(IV)

6. Procédé pour combattre la croissance végétale indésirable ou pour influer sur la croissance végétale, caractérisé en ce qu'on fait agir sur les plantes ou leur habitat une quantité à action de régulation de la croissance des plantes d'une urée de formule I

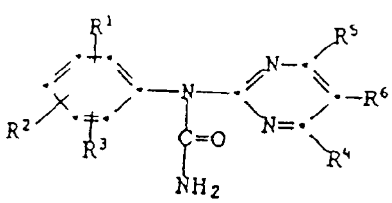

(I),

dans laquelle

$R^1$, $R^2$ et $R^3$ représentent indépendamment l'un de l'autre un hydrogène ; nitro ; cyano ; halogène ; alcoyle en $C_{1-4}$ ; alcoyle en $C_{1-4}$-$S(O)_n$ ; alcoxy en $C_{1-4}$, halogènalcoyle en $C_{1-4}$ ; ha-

logènalcoxy en $C_{1-4}$ ; halogènalcoyle en $C_{1-4}$-$S(O)_n$ ; alcoxycarbonyle en $C_{1-4}$ ; alcoylcarbonyle en $C_{1-4}$ ; aminocarbonyle ; mono-alcoyle en $C_{1-4}$-amino-carbonyle ; ou dialcoyle en $C_{1-4}$-aminocarbonyle ;

$R^4$ et $R^5$ représentent indépendamment l'un de l'autre un hydrogène ; alcoyle en $C_{1-4}$ ; alcoxy en $C_{1-4}$ ; alcoyle en $C_{1-4}$-$S(O)_n$ ; halogènalcoyle en $C_{1-4}$ ; halogènalcoxy en $C_{1-4}$ ; halogènalcoyle en $C_{1-4}$-$S(O)_n$ ; un phényle non-substitué ou jusqu'à trois fois substitué, de façon identique ou différente, par alcoyle en $C_{1-4}$, halogène, alcoxy en $C_{1-4}$, halogènalcoyle en $C_{1-4}$, nitro ou cyano ; furanyle ; thiophényle ; cycloalcoyle en $C_{3-6}$ ; alcoxy en $C_{1-4}$-carbonyle ; alcoxy en $C_{1-4}$-alcoyle en $C_{1-4}$ ; alcoxy en $C_{1-4}$-carbonyle-alcoyle en $C_{1-4}$ ; alcoyle en $C_{1-4}$-carbonyle-alcoyle en $C_{1-4}$ ; alcényloxy en $C_{3-4}$-carbonyle-alcoyle en $C_{1-4}$ ; alcynyloxy en $C_{3-4}$ carbonyle-alcoyle en $C_{1-4}$ ; halogène ; ou cyano ;

$R^6$ représente un alcoyle en $C_{1-4}$ ; halogène, cyano ; cycloalcoyle en $C_{3-6}$ ; halogènalcoyle en $C_{1-4}$ ; alcoxy en $C_{1-4}$ ; nitro ; alcoyle en $C_{1-4}$-$S(O)_n$ ; halogènalcoxy en $C_{1-4}$ ; halogènalcoyle en $C_{1-4}$-$S(O)_n$ ; ou un phényle substitué jusqu'à trois fois de manière identique ou différente par un halogène, alcoyle en $C_{1-4}$, halogènalcoyle en $C_{1-4}$, alcoyle en $C_{1-4}$-$S(O)_n$, alcoxy en $C_{1-4}$, halogènalcoxy en $C_{1-4}$, nitro ou cyano ; ou un alcoyle en $C_{1-4}$-thio-alcoyle en $C_{1-4}$ ;

n vaut 0, 1 ou 2 ; ou

$R^5$ et $R^6$ représentent ensemble avec les deux atomes de carbone auxquels ils sont liés un noyau condensé, partiellement insaturé, à 4 à 8 chaînons, qui peut être substitué jusqu'à trois fois de manière identique ou différente par un alcoyle en $C_{1-4}$, un halogène, un alcoxy en $C_{1-4}$-carbonyle et/ou être interrompu par O, S ou N-alcoyle en $C_{1-4}$, et/ou contenir une double liaison, et/ou un groupe carbonyle ;

y compris leurs sels avec des acides, des bases et des formateurs de complexes.

7. Procédé selon la revendication 6 pour combattre la croissance végétale indésirable dans les cultures de plantes utiles.

8. Procédé selon la revendication 6, pour influer sur la croissance des plantes cultivées.

9. Agent herbicide contenant un composé de formule I

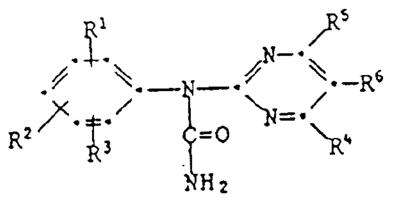

(I),

dans laquelle

$R^1$, $R^2$ et $R^3$ représentent indépendamment l'un de l'autre un hydrogène ; nitro ; cyano ; halogène ; alcoyle en $C_{1-4}$ ; alcoyle en $C_{1-4}$-$S(O)_n$ ; alcoxy en $C_{1-4}$, halogènalcoyle en $C_{1-4}$ ; halogènalcoxy en $C_{1-4}$ ; halogènalcoyle en $C_{1-4}$-$S(O)_n$ ; alcoxycarbonyle en $C_{1-4}$ ; alcoylcarbonyle en $C_{1-4}$ ; aminocarbonyle ; mono-alcoyle en $C_{1-4}$-amino-carbonyle ; ou dialcoyle en $C_{1-4}$-aminocarbonyle ;

$R^4$ et $R^5$ représentent indépendamment l'un de l'autre un hydrogène ; alcoyle en $C_{1-4}$ ; alcoxy en $C_{1-4}$ ; alcoyle en $C_{1-4}$-$S(O)_n$ ; halogènalcoyle en $C_{1-4}$ ; halogènalcoxy en $C_{1-4}$ ; halogènalcoyle en $C_{1-4}$-$S(O)_n$ ; un phényle non-substitué ou jusqu'à trois fois substitué, de façon identique ou différente, par alcoyle en $C_{1-4}$, halogène, alcoxy en $C_{1-4}$, halogènalcoyle en $C_{1-4}$, nitro ou cyano ; furanyle ; thiophényle ; cycloalcoyle en $C_{3-6}$ ; alcoxy en $C_{1-4}$-carbonyle ; alcoxy en $C_{1-4}$-alcoyle en $C_{1-4}$ ; alcoxy en $C_{1-4}$-carbonyle-alcoyle en $C_{1-4}$ ; alcoyle en $C_{1-4}$-carbonyle-alcoyle en $C_{1-4}$ ; alcényloxy en $C_{3-4}$-carbonyle-alcoyle en $C_{1-4}$ ; alcynyloxy en $C_{3-4}$-carbonyle-alcoyle en $C_{1-4}$ ; halogène ; ou cyano ;

$R^6$ représente un alcoyle en $C_{1-4}$ ; halogène, cyano ; cycloalcoyle en $C_{3-6}$ ; halogènalcoyle en $C_{1-4}$ ; alcoxy en $C_{1-4}$ ; nitro ; alcoyle en $C_{1-4}$-$S(O)_n$ ; halogènalcoxy en $C_{1-4}$ ; halogènalcoyle en $C_{1-4}$-$S(O)_n$ ; ou un phényle substitué jusqu'à trois fois de manière identique ou différente par un halogène, alcoyle en $C_{1-4}$, halogènalcoyle en $C_{1-4}$, alcoyle en $C_{1-4}$-$S(O)_n$, alcoxy en $C_{1-4}$, halogènalcoxy en $C_{1-4}$, nitro ou cyano ; ou un alcoyle en $C_{1-4}$-thio-alcoyle en $C_{1-4}$ ;

n vaut 0, 1 ou 2 ; ou
$R^5$ et $R^6$ représentent ensemble avec les deux atomes de carbone auxquels ils sont liés un noyau condensé, partiellement insaturé, à 4 à 8 chaînons, qui peut être substitué jusqu'à trois fois de manière identique ou différente par un alcoyle en $C_{1-4}$, un halogène, un alcoxy en $C_{1-4}$-carbonyle et/ou être interrompu par O, S ou N-alcoyle en $C_{1-4}$, et/ou contenir une double liaison, et/ou un groupe carbonyle ;

caractérisé en ce qu'il contient entre 0,1 et 80% de matière active de formule I, de 1 à 99,9% d'additif solide ou liquide et de 0 à 25% d'un agent tensio-actif.

**10.** Procédé pour combattre la croissance végétale indésirable ou pour influer sur la croissance végétale, caractérisé en ce qu'on fait agir sur les plantes à combattre ou leur habitat une quantité à action herbicide ou de régulation de la croissance des plantes d'un agent selon la revendication 9.